(19)

**Europäisches Patentamt**
**European Patent Office**
**Office européen des brevets**

(11) **EP 4 700 787 A2**

(12) # EUROPEAN PATENT APPLICATION

(43) Date of publication:
**25.02.2026 Bulletin 2026/09**

(21) Application number: **25209246.5**

(22) Date of filing: **26.11.2020**

(51) International Patent Classification (IPC):
**G16H 40/63** *(2018.01)*

(52) Cooperative Patent Classification (CPC):
(C-Sets available)
**A61M 16/0605; A61M 16/024; A61M 16/0683;**
**A61M 16/16; G06T 7/74; G06V 10/82;**
**G09B 19/003; G16H 20/40; G16H 40/63;**
A61M 16/0006; A61M 16/0051; A61M 16/0066;
A61M 16/0075; A61M 16/0622; A61M 16/0633;

(Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **27.11.2019 SG 10201911248T**

(62) Document number(s) of the earlier application(s) in
accordance with Art. 76 EPC:
**20892817.6 / 4 066 212**

(71) Applicant: **ResMed Asia Pte. Ltd.**
**Singapore 639443 (SG)**

(72) Inventors:
• **TAN, Ben Hai**
  **Singapore 509016 (SG)**
• **BIN ABDUL HALIM, Muhammad Adil**
  **Singapore 509016 (SG)**
• **LIM, Yong Keat**
  **Singapore 509016 (SG)**
• **CHAN, Yun Hoi**
  **Singapore 509016 (SG)**

• **JADHAV, Amit Arunchandra**
  **Singapore 509016 (SG)**
• **TAY, Barry Eng Keong**
  **Singapore 509016 (SG)**
• **LEE, Cheoung Hong**
  **Singapore 509016 (SG)**
• **CHUAH, Teong Hong**
  **Singapore 509016 (SG)**
• **CHEN, Jing**
  **Singapore 509016 (SG)**

(74) Representative: **Vossius & Partner**
**Patentanwälte Rechtsanwälte mbB**
**Siebertstraße 3**
**81675 München (DE)**

Remarks:
•This application was filed on 16.10.2025 as a
divisional application to the application mentioned
under INID code 62.
•Claims filed after the date of receipt of the divisional
application (Rule 68(4) EPC).

(54) **APPLICATION TO GUIDE MASK FITTING**

(57) The present invention relates to a device comprising a display, a camera, a memory, and a processing system. The processing system includes at least one hardware processor coupled to the display, the camera, and the memory. The processing system is configured to capture one or more images including a patient using the camera, determine characteristics of facial features from the captured images. The processing system is further configured to transmit the determined characteristics of the facial features to a remote processing system for analysis. Based on analysis performed by the remote processing system, the proceeding system receives, from the remote processing system, data for one or more settings of a patient interface configured to engage with at least one airway of the patient and supply breathable gas received from a continuous positive air pressure (CPAP) device to the patient; and displays, on the display, instructions for adjusting the one or more settings of the patient interface based on the received data for the one or more settings of the patient interface.

EP 4 700 787 A2

(52) Cooperative Patent Classification (CPC): (Cont.)
A61M 16/0666; A61M 16/0672; A61M 16/0688;
A61M 16/0825; A61M 16/0858; A61M 16/0891;
A61M 16/1055; A61M 16/107; A61M 16/1085;
A61M 16/109; A61M 16/1095; A61M 16/12;
A61M 16/161; A61M 16/20; A61M 2016/0021;
A61M 2016/0024; A61M 2016/0027;
A61M 2016/0039; A61M 2016/0661;
A61M 2202/0208; A61M 2202/0225;
A61M 2205/0216; A61M 2205/0294;
A61M 2205/13; A61M 2205/14; A61M 2205/15;
A61M 2205/18; A61M 2205/21; A61M 2205/3306;
A61M 2205/3317; A61M 2205/332;
A61M 2205/3331; A61M 2205/3334;
A61M 2205/3365; A61M 2205/3368;
A61M 2205/3379; A61M 2205/3553;
A61M 2205/3561; A61M 2205/3584;
A61M 2205/3592; A61M 2205/3653;
A61M 2205/42; A61M 2205/52; A61M 2205/581;
A61M 2205/582; A61M 2205/583;
A61M 2205/6054; A61M 2205/6063;
A61M 2205/6072; A61M 2205/609;
A61M 2205/7518; A61M 2205/80;
A61M 2205/8206; A61M 2206/14; A61M 2207/00;
A61M 2209/01; A61M 2209/086; A61M 2210/0618;
A61M 2230/205; A61M 2230/40; A61M 2230/42;
A61M 2230/50; G06F 9/453; G06T 2200/24;
G06T 2207/10016; G06T 2207/10048;
G06T 2207/30196; Y02A 90/10

C-Sets
A61M 2202/0208, A61M 2202/0007;
A61M 2202/0225, A61M 2202/0085;
A61M 2230/205, A61M 2230/005;
A61M 2230/40, A61M 2230/005;
A61M 2230/42, A61M 2230/005;
A61M 2230/50, A61M 2230/005

**Description**

1 CROSS-REFERENCE TO RELATED APPLICATIONS

This application claims priority to Singapore Patent Application No. 10201911248T, filed November 27, 2019, which is incorporated herein by reference in the entirety.

2 BACKGROUND OF THE TECHNOLOGY

2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use, and more particularly to methods and systems for detecting improper patient interface positioning associated with the medical devices or apparatuses and/or guiding the patient to position and/or adjust to correct fitting position of the patient interface.

2.2 DESCRIPTION OF THE RELATED ART

**2.2.1 Human Respiratory System and its Disorders**

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"Respiratory Physiology",* by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient $CO_2$ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired $CO_2$ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 2.2.2.3 Supplementary oxygen

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

### 2.2.3 Respiratory therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 $cmH_2O$ relative to ambient pressure. For other forms of therapy, such as the delivery of oxygen, the patient interface may not include a seal sufficient to facilitate delivery to the airways of a supply of gas at a positive pressure of about 10 $cmH_2O$. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

*2.2.3.1.1 Seal-forming structure*

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.)*,* assigned to Puritan-Bennett Corporation.

ResMed Limited has manufactured the following products that incorporate nasal pillows: SWIFT™ nasal pillows mask, SWIFT™ II nasal pillows mask, SWIFT™ LT nasal pillows mask, SWIFT™ FX nasal pillows mask and MIRAGE LIBERTY™ full-face mask. The following patent applications, assigned to ResMed Limited, describe examples of nasal pillows masks: International Patent Application WO2004/073,778 (describing amongst other things aspects of the ResMed Limited SWIFT™ nasal pillows), US Patent Application 2009/0044808 (describing amongst other things aspects of the ResMed Limited SWIFT™ LT nasal pillows); International Patent Applications WO 2005/063,328 and WO 2006/130,903 (describing amongst other things aspects of the ResMed Limited MIRAGE LIBERTY™ full-face mask); International Patent Application WO 2009/052,560 (describing amongst other things aspects of the ResMed Limited SWIFT™ FX nasal pillows).

### *2.2.3.1.2 Positioning and stabilising*

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH$_2$O).

| RPT Device name | A-weighted sound pressure level dB(A) | Year (approx.) |
| --- | --- | --- |
| C-Series Tango™ | 31.9 | 2007 |
| C-Series Tango™ with Humidifier | 33.1 | 2007 |
| S8 Escape™ II | 30.5 | 2005 |
| S8 Escape™ II with H4i™ Humidifier | 31.1 | 2005 |
| S9 AutoSet™ | 26.5 | 2010 |
| S9 AutoSet™ with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar™ Series of Adult and Paediatric Ventilators may provide support for invasive and non-invasive non-dependent ventilation for a range of patients for treating a number of conditions such as but not limited to NMD, OHS and COPD.

The ResMed Elisée™ 150 ventilator and ResMed VS III™ ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air. Humidifiers therefore often have the capacity to heat the flow of air was well as humidifying it.

A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

### 2.2.3.5 Oxygen source

Experts in this field have recognized that exercise for respiratory failure patients provides long term benefits that slow the progression of the disease, improve quality of life and extend patient longevity. Most stationary forms of exercise like tread mills and stationary bicycles, however, are too strenuous for these patients. As a result, the need for mobility has long been recognized. Until recently, this mobility has been facilitated by the use of small compressed oxygen tanks or cylinders mounted on a cart with dolly wheels. The disadvantage of these tanks is that they contain a finite amount of oxygen and are heavy, weighing about 50 pounds when mounted.

Oxygen concentrators have been in use for about 50 years to supply oxygen for respiratory therapy. Traditional oxygen concentrators have been bulky and heavy making ordinary ambulatory activities with them difficult and impractical. Recently, companies that manufacture large stationary oxygen concentrators began developing portable oxygen concentrators (POCs). The advantage of POCs is that they can produce a theoretically endless supply of oxygen. In order to make these devices small for mobility, the various systems necessary for the production of oxygen enriched gas are condensed. POCs seek to utilize their produced oxygen as efficiently as possible, in order to minimise weight, size, and power consumption. This may be achieved by delivering the oxygen as series of pulses or "boli", each bolus timed to coincide with the start of inspiration. This therapy mode is known as pulsed or demand (oxygen) delivery (POD), in contrast with traditional continuous flow delivery more suited to stationary oxygen concentrators.

### 2.2.3.6 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.7 Mandibular repositioning

A mandibular repositioning device (MRD) or mandibular advancement device (MAD) is one of the treatment options for sleep apnea and snoring. It is an adjustable oral appliance available from a dentist or other supplier that holds the lower jaw (mandible) in a forward position during sleep. The MRD is a removable device that a patient inserts into their mouth prior to going to sleep and removes following sleep. Thus, the MRD is not designed to be worn all of the time. The MRD may be custom made or produced in a standard form and includes a bite impression portion designed to allow fitting to a patient's teeth. This mechanical protrusion of the lower jaw expands the space behind the tongue, puts tension on the pharyngeal walls to reduce collapse of the airway and diminishes palate vibration.

In certain examples a mandibular advancement device may comprise an upper splint that is intended to engage with or fit over teeth on the upper jaw or maxilla and a lower splint that is intended to engage with or fit over teeth on the upper jaw or mandible. The upper and lower splints are connected together laterally via a pair of connecting rods. The pair of connecting rods are fixed symmetrically on the upper splint and on the lower splint.

In such a design the length of the connecting rods is selected such that when the MRD is placed in a patient's mouth the mandible is held in an advanced position. The length of the connecting rods may be adjusted to change the level of protrusion of the mandible. A dentist may determine a level of protrusion for the mandible that will determine the length of the connecting rods.

Some MRDs are structured to push the mandible forward relative to the maxilla while other MADs, such as the ResMed Narval CC™ MRD are designed to retain the mandible in a forward position. This device also reduces or minimises dental and temporo-mandibular joint (TMJ) side effects. Thus, it is configured to minimises or prevent any movement of one or more of the teeth.

### 2.2.3.8 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

Table of noise of prior masks (ISO 17510-2:2007, 10 cmH$_2$O pressure at 1m)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage™ (*) | nasal | 29.5 | 21.5 | 1998 |
| ResMed UltraMirage™ | nasal | 36(3) | 28 (3) | 2000 |
| ResMed Mirage Activa™ | nasal | 32 (3) | 24 (3) | 2002 |
| ResMed Mirage Micro™ | nasal | 30 (3) | 22 (3) | 2008 |
| ResMed Mirage™ SoftGel | nasal | 29 (3) | 22 (3) | 2008 |
| ResMed Mirage™ FX | nasal | 26 (3) | 18 (3) | 2010 |
| ResMed Mirage Swift™ (*) | nasal pillows | 37 | 29 | 2004 |
| ResMed Mirage Swift™ II | nasal pillows | 28 (3) | 20 (3) | 2005 |
| ResMed Mirage Swift™ LT | nasal pillows | 25 (3) | 17 (3) | 2008 |
| ResMed AirFit P10 | nasal pillows | 21 (3) | 13 (3) | 2014 |
| (* one specimen only, measured using test method specified in ISO 3744 in CPAP mode at 10 cmH$_2$O) | | | | |

Sound pressure values of a variety of objects are listed below

| Object | A-weighted sound pressure dB(A) | Notes |
|---|---|---|
| Vacuum cleaner: Nilfisk Walter Broadly Litter Hog: B+ Grade | 68 | ISO 3744 at 1m distance |
| Conversational speech | 60 | 1m distance |
| Average home | 50 | |
| Quiet library | 40 | |
| Quiet bedroom at night | 30 | |
| Background in TV studio | 20 | |

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

### 3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

One form of the present technology comprises a device comprising: a display; a camera; memory; and a processing system including at least one hardware processor coupled to the display, the camera, and the memory, the processing system configured to: receive one or more images including a patient with a patient interface configured to engage with at least one airway of a patient and supply breathable gas received from a continuous positive air pressure (CPAP) device to the patient; analyse the received one or more images; and based on the analysis, display, on the display, feedback for improving the fit and/or operation of the patient interface.

Another aspect of the present technology relates to a respiratory pressure therapy system for providing continuous positive air pressure to a patient via a patient interface configured to engage with at least one airway of the patient. The system includes: a flow generator configured to generate supply of breathable gas for delivery to the patient via the patient interface; at least one sensor; a display; and a computing device. The computing device is configured to: receive sensor data that is based on measured physical property of the supply of breathable gas; control, based on the received sensor data, the flow generator to adjust a property of the supply of breathable gas; receive an input indicating assistance is needed with using the patient interface; receive one or more images of the patient with the patient interface; analyse the received one or more images; and based on the analysis, display, on the display, instructions for positioning the patient interface.

Another aspect of the present technology relates to a respiratory pressure therapy system for providing continuous positive air pressure (CPAP) to a patient via a patient interface configured to engage with at least one airway of the patient, the system comprising: a flow generator configured to generate supply of breathable gas for delivery to the patient via the patient interface, wherein the breathable gas is output from the flow generator at a pressure level that is above atmospheric pressure; at least one sensor that is configured to measure a physical quantity while the breathable gas is supplied to the patient; a display; and a computing device including memory and at least one hardware processor. The computing device is configured to: receive, from the at least one sensor, sensor data that is based on measured physical property of the supply of breathable gas; control, based on the received sensor data, the flow generator to adjust a property of the supply of breathable gas that is delivered to the patient; receive, an input indicating assistance is needed with using the patient interface; receive one or more images including the patient with the patient interface; analyse the received one or more images; and based on the analysis, display, on the display, instructions for positioning the patient interface.

In examples, (a) the memory includes one or more reference images including the patient with the patient interface, and the displayed instructions are generated by the computing device based on comparison between the one or more reference images and the received one or more images; (b) computing device is coupled to a camera configured to capture the one or more reference images; (c) the computing device is configured to compare the one or more reference images and the received one or more images to determine improper patient interface fitting position; (d) the computing device is configured to superimpose a correct position of the patient interface on the received one or more images, and the displayed instructions include the one or more superimposed images; (e) system includes a remote computing system and the remote computing system is configured to determine the instructions for positioning the patient interface, and transmit the instructions to the computing device; (f) the remote computing system is configured to: receive, from the computing device, the one or more images; train a machine learning model for instructing correct positioning of a patient interface; and the instructions for positioning the patient interface are determined based on the trained machine learning model; (g) the computing device is further configured to receive an input indicating a type of the patient interface, and display, on the display, instructions for using the type of patient interface indicated by the input; (h) the computing device is configured to transmit the received one or more images to a remote processing system configured to perform machine learning using the one or more images; and/or (i) the instructions include text, images, and/or graphics.

Another aspect of the present technology relates to a non-transitory computer readable storage medium storing instructions for use with a computing device that is configured to control a continuous positive air pressure (CPAP) device configured to generate the supply of breathable gas that is delivered to a patient via a patient interface configured to engage with at least one airway of the patient, wherein the breathable gas is output from a flow generator at a pressure level that is above atmospheric pressure, the CPAP device associated with at least one sensor that is configured to measure a physical quantity while the breathable gas is supplied to the patient, the computing device including at least one hardware processor, the stored instructions comprising instructions that are configured to cause the computing device to: receive, from the at least one sensor, sensor data that is based on measured physical property of the supply of breathable gas; control, based on the received sensor data, the flow generator to adjust a property of the supply of breathable gas that is delivered to the patient; receive, an input indicating assistance is needed with using the patient interface; receive one or more images including the patient with the patient interface; analyse the received one or more images; and based on the analysis, display, on the display, instructions for positioning the patient interface.

Another aspect of the present technology relates to a device comprising: a display; a camera; a memory; and a processing system including at least one hardware processor coupled to the display, the camera, and the memory. The processing system configured to: receive an indication identifying a type of a patient interface, configured to engage with at least one airway of a patient and supply breathable gas received from a continuous positive air pressure (CPAP) device to the patient; receive, from the camera, one or more images including a patient with the patient interface; analyse the received one or more images to determine fit of the patient interface on the patient; and based on the analysis, display, on the display, feedback for improving the fit of the patient interface on the patient.

In examples, (a) the received indication identifying the type of the patient interface is a user input; (b) the received indication identifying the type of the patient interface is determined, by the processing system, based on an image including the patient interface; (c) the analysis includes comparing the received one or more images to one or more reference images; (d) the one or more reference images include the patient with the patient interface; (e) the one or more reference images include a plurality of reference points, and the analysis includes, detecting reference points in the received one or more images and comparing the detected reference points to the plurality of reference points in the one or more defence images; (f) the analysis includes extracting, from the received one or more images, one or more indicators included in patient interface; (g) the received one or more images are captured from different positions and orientations of the camera; (h) a plurality of images are captured at different times and the analysis includes comparing the plurality of images to determine changes in positioning of the patient interface between images captured at the different times; (i) displaying the feedback includes displaying at least one of the received images, and the processing system is configured to include, in the displayed received images, one or more visual indicators indicating locations on the patient interface where the fit of the patient interface can be improved; (j) the processing system is configured to display instructions for using the identified type of patient interface; (k) the analysis includes comparing the received one or more images to models generated based on information received from other patients; (1) the analysis includes extracting features from the received one or more images, and comparing positions and/or orientations of the features to a three-dimensional model of the patient interface; (m) the analysis includes comparing the received one or more images to a machine trained model that is updated based on data received from other patients; (n) the device is a mobile phone, tablet or remote control; (o) the processing system is further configured to receive sensor data from one or more sensors disposed on a surface of or in the patient interface; (p) the processing system is further configured to perform the analysis and display the feedback based on data received from the sensors; and/or (q) the sensor data is compared to pre-set sensor values stored in the memory.

Another aspect of the present technology relates to a non-transitory computer readable storage medium storing instructions for use with a computing device, the stored instructions comprising instructions that are configured to cause the computing device to: receive an indication identifying a type of a patient interface, configured to engage with at least one airway of a patient and supply breathable gas received from a continuous positive air pressure (CPAP) device to the patient; receive, from a camera, one or more images including a patient with the patient interface; analyse the received one or more images to determine fit of the patient interface on the patient; and based on the analysis, display, on the display, feedback for improving the fit of the patient interface on the patient.

Another aspect of the present technology relates to a respiratory pressure therapy system for providing continuous positive air pressure (CPAP) to a patient via a patient interface configured to engage with at least one airway of the patient, the system comprising: a flow generator configured to generate supply of breathable gas for delivery to the patient via the patient interface, wherein the breathable gas is output from the flow generator at a pressure level that is above atmospheric pressure; a display; and a computing device including memory and at least one hardware processor. The computing device is configured to: control the flow generator to adjust a property of the supply of breathable gas that is delivered to the patient; receive sensor data from the one or more patient interface sensors; compare the received sensor data to pre-set values in the memory; and based on the comparison, display, on the display, instructions for positioning the patient interface.

In examples, (a) the memory includes one or more images including a patient with the patient interface, and the displayed instructions include displaying the one or more images based on the comparison; (b) the computing device is coupled to a camera configured to capture one or more images of the patient with the patient interface; (c) the computing device is configured to compare one or more reference images stored in memory and the captured one or more images to determine improper patient interface fitting position; (d) the computing device is configured to superimpose a correct position of the patient interface on the captured one or more images, and the displayed instructions include the one or more superimposed images; (e) further comprises a remote computing system and the remote computing system is configured to determine the instructions for positioning the patient interface, and transmit the instructions to the computing device; (f) the computing device is further configured to receive an input indicating a type of the patient interface, and display, on the display, instructions for using the type of patient interface indicated by the input; (g) at least one of the one or more patient interface sensors is disposed on a surface of the patient interface; (h) at least one of the one or more patient interface sensors is a pressure sensor configured to measure pressure inside of a mask of the patient interface; (i) at least one of the one or more patient interface sensors is a sensor configured to measure a physical property of the supply of breathable gas in an oral or nasal cushion of the patient interface; and/or (j) at least one of the one or more patient interface sensors is disposed on a surface or inside of a strap of the patient interface.

Another aspect of the present technology relates to a program for troubleshooting, and includes: instructing a user to take an image or video, comparing the image or video to a baseline video to create a result, and instructing the user to take action based on the result.

Another aspect of the present technology relates to a program for troubleshooting, and includes: instructing a user to take an image or video, comparing the image or video to a baseline image or video to create a result, and instructing the user to take action based on the result.

Another aspect of the present technology relates to a non-transitory computer readable storage medium storing instructions for use with a computing device, the stored instructions comprising instructions that, when executed, cause the computing device to: display instructions to take an image or video, compare a captured image or video to a baseline image or video to create a result, and display instructions for an action based on the results.

In examples, (a) the stored instructions further include instructions that, when executed, cause the computing device to: in response to an input capture an image or video; (b) the captured image or video includes a user and a patient interface; and/or (c) the stored instructions further include instructions that, when executed, cause the computing device to: receive an input indicating a type of patient interface, and display, in response to the input, instructions for using the patient interface.

Another aspect of the present technology relates to a device comprising: a display; a camera; a memory; and a processing system including at least one hardware processor coupled to the display, the camera, and the memory. The processing system configured to: capture one or more images including a patient using the camera; determine characteristics of facial features from the captured images; transmit the determined characteristics of the facial features to a remote processing system for analysis; receive, from the remote processing system, data for one or more settings of a patient interface configured to engage with at least one airway of the patient and supply breathable gas received from a continuous positive air pressure (CPAP) device to the patient; and display, on the display, instructions for adjusting the one or more settings of the patient interface based on the received data for the one or more settings of the patient interface.

In examples, (a) the processing system is further configured to: receive a user input identifying a type of the patient interface; and transmit the type of the patient interface to the remote processing system for analysis; (b) the processing system is further configured to: receive, from the camera, one or more second images including the patient with the patient interface; analyse the received one or more second images to determine fit of the patient interface on the patient; and based on the analysis and the received data for the one or more settings of the patient interface, display, on the display, feedback for improving the fit of the patient interface on the patient; (c) the analysis includes comparing the received one or more second images to one or more reference images; (d) the one or more reference images include the patient wearing the patient interface; (e) the one or more reference images include a plurality of reference points, and the analysis includes, detecting reference points in the received one or more second images and comparing the detected reference points to the plurality of reference points in the one or more defence images; (f) the analysis includes extracting, from the received one or more second images, one or more indicators included on the patient interface; (g) the one or more indicators include a plurality of indicators provided on one or more straps of the patient interface; (h) the one or more indicators include at least one indicator provided on a mask of the patient interface; (i) the one or more indicators include an indicator provided on a connector configured to connect at least one of the straps to a mask of the patient interface; (j) the processing system is further configured to: determine a force applied by one or more straps based on the characteristics of the one or more indicators in the one or more second images; (k) the processing system is further configured to compare forces applied by different straps of the patient interface based on characteristics of the one or more indicators included in the different straps; (l) the processing system is further configured to indicate that one or more straps are over tightened based on characteristics of the one or more indicators in the one or more straps; (m) the analysis performed by the remote processing system includes comparing the received one or more images to models generated based on information received from a plurality of other patients; and/or (o) the device is the respiratory pressure therapy device for providing continuous positive air pressure (CPAP) to the patient, the respiratory pressure therapy device comprising: a flow generator configured to generate supply of breathable gas for delivery to the patient via the patient interface, wherein the breathable gas is output from the flow generator at a pressure level that is above atmospheric pressure; and at least one sensor that is configured to measure a physical quantity while the breathable gas is supplied to the patient.

Another aspect of the present technology relates to a non-transitory computer readable storage medium storing instructions for use with a computing device, the stored instructions comprising instructions that are configured to cause the computing device to: receive one or more images including a patient using captured by a camera; determine characteristics of facial features from the captured images; transmit the determined characteristics of the facial features to a remote processing system for analysis; receive, from the remote processing system, data for one or more settings of a patient interface configured to engage with at least one airway of the patient and supply breathable gas received from a continuous positive air pressure (CPAP) device to the patient; and output instructions for adjusting the one or more settings of the patient interface based on the received data for the one or more settings of the patient interface.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

## 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

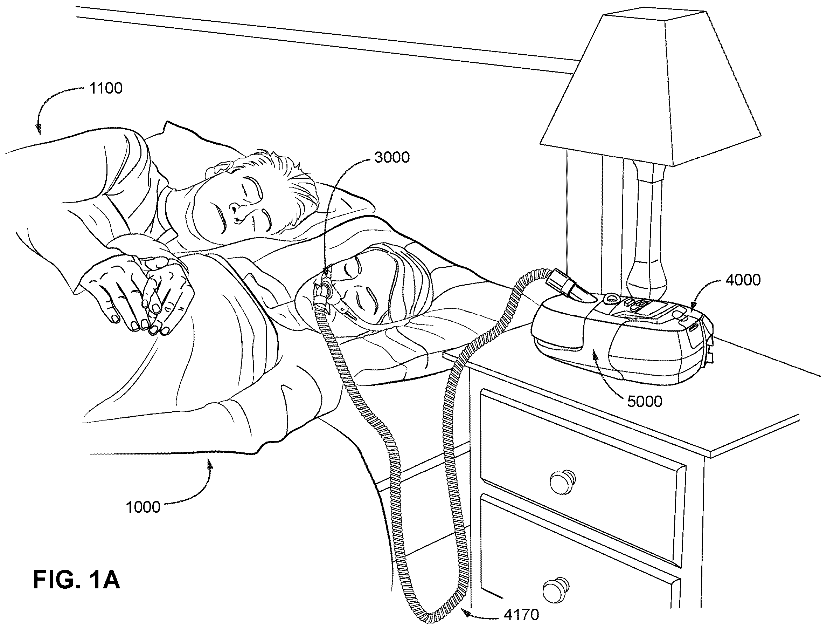

Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.

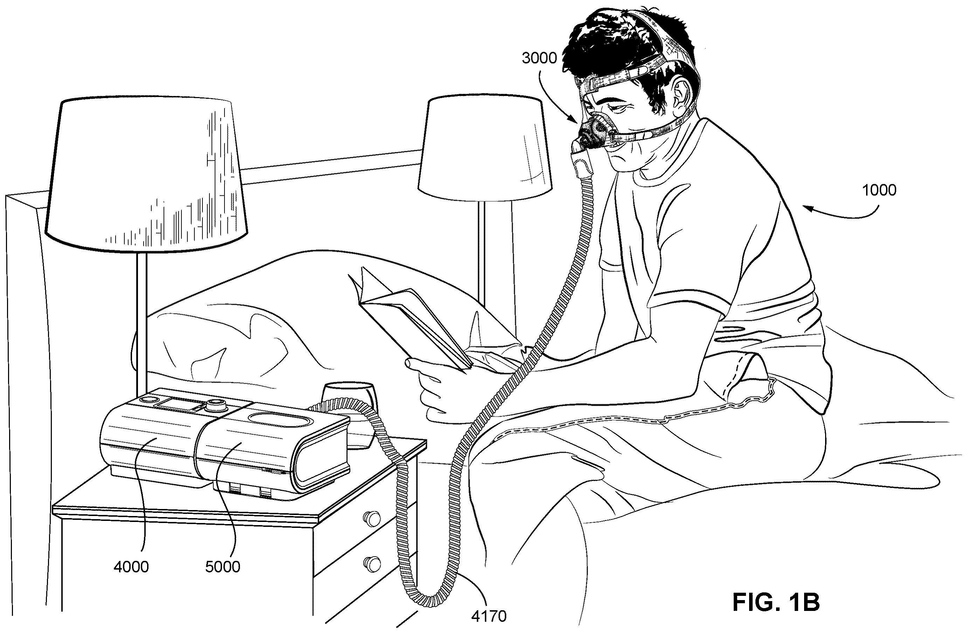

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

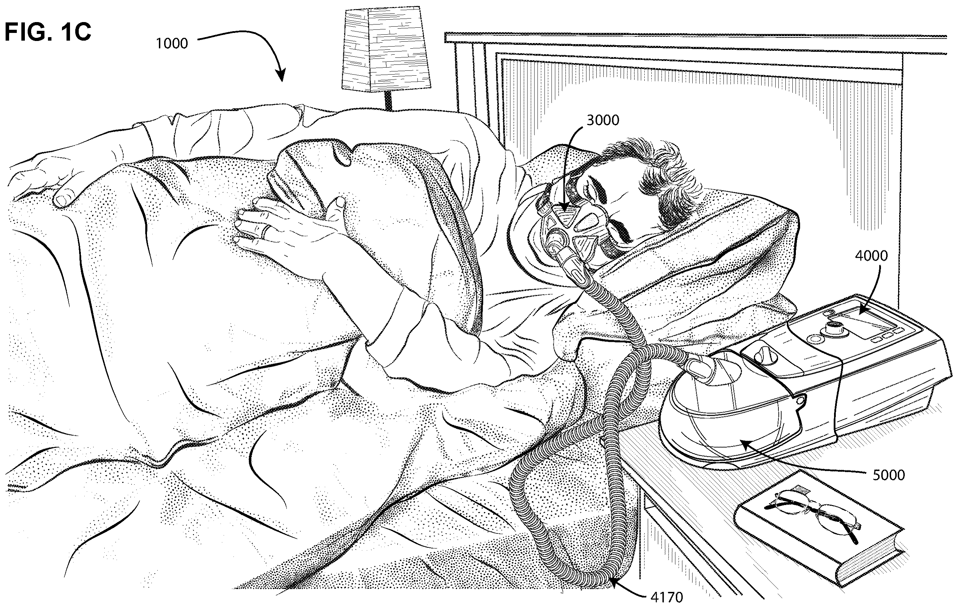

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

Fig. 2A shows an overview of a human respiratory system including the nasal and oral cavities, the larynx, vocal folds, oesophagus, trachea, bronchus, lung, alveolar sacs, heart and diaphragm.

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

Fig. 2E is a further side view of a head. The approximate locations of the Frankfort horizontal and nasolabial angle are indicated. The coronal plane is also indicated.

Fig. 2F shows a base view of a nose with several features identified including naso-labial sulcus, lip inferior, upper Vermilion, naris, subnasale, columella, pronasale, the major axis of a naris and the midsagittal plane.

Fig. 2G shows a side view of the superficial features of a nose.

Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.

Fig. 2I shows a medial dissection of a nose, approximately several millimeters from the midsagittal plane, amongst other things showing the septum cartilage and medial crus of greater alar cartilage.

Fig. 2J shows a front view of the bones of a skull including the frontal, nasal and zygomatic bones. Nasal concha are indicated, as are the maxilla, and mandible.

Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

Fig. 2L shows an anterolateral view of a nose.

4.3 PATIENT INTERFACE

Fig. 3A shows a patient interface in the form of a nasal mask in accordance with one form of the present technology.

Fig. 3B shows a schematic of a cross-section through a structure at a point. An outward normal at the point is indicated. The curvature at the point has a positive sign, and a relatively large magnitude when compared to the magnitude of the curvature shown in Fig. 3C.

Fig. 3C shows a schematic of a cross-section through a structure at a point. An outward normal at the point is indicated. The curvature at the point has a positive sign, and a relatively small magnitude when compared to the magnitude of the curvature shown in Fig. 3B.

Fig. 3D shows a schematic of a cross-section through a structure at a point. An outward normal at the point is indicated. The curvature at the point has a value of zero.

Fig. 3E shows a schematic of a cross-section through a structure at a point. An outward normal at the point is indicated. The curvature at the point has a negative sign, and a relatively small magnitude when compared to the magnitude of the curvature shown in Fig. 3F.

Fig. 3F shows a schematic of a cross-section through a structure at a point. An outward normal at the point is indicated. The curvature at the point has a negative sign, and a relatively large magnitude when compared to the magnitude of the curvature shown in Fig. 3E.

Fig. 3G shows a cushion for a mask that includes two pillows. An exterior surface of the cushion is indicated. An edge of the surface is indicated. Dome and saddle regions are indicated.

Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.

Fig. 3I shows the surface of a structure, with a one dimensional hole in the surface. The illustrated plane curve forms the boundary of a one dimensional hole.

Fig. 3J shows a cross-section through the structure of Fig.3I. The illustrated surface bounds a two dimensional hole in the structure of Fig. 3I.

Fig. 3K shows a perspective view of the structure of Fig. 3I, including the two dimensional hole and the one dimensional hole. Also shown is the surface that bounds a two dimensional hole in the structure of Fig. 3I.

Fig. 3L shows a mask having an inflatable bladder as a cushion.

Fig. 3M shows a cross-section through the mask of Fig. 3L, and shows the interior surface of the bladder. The interior surface bounds the two dimensional hole in the mask.

Fig. 3N shows a further cross-section through the mask of Fig. 3L. The interior surface is also indicated.

Fig. 3O illustrates a left-hand rule.

Fig. 3P illustrates a right-hand rule.

Fig. 3Q shows a left ear, including the left ear helix.

Fig. 3R shows a right ear, including the right ear helix.

Fig. 3S shows a right-hand helix.

Fig. 3T shows a view of a mask, including the sign of the torsion of the space curve defined by the edge of the sealing membrane in different regions of the mask.

Fig. 3U shows a view of a plenum chamber 3200 showing a sagittal plane and a mid-contact plane.

Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

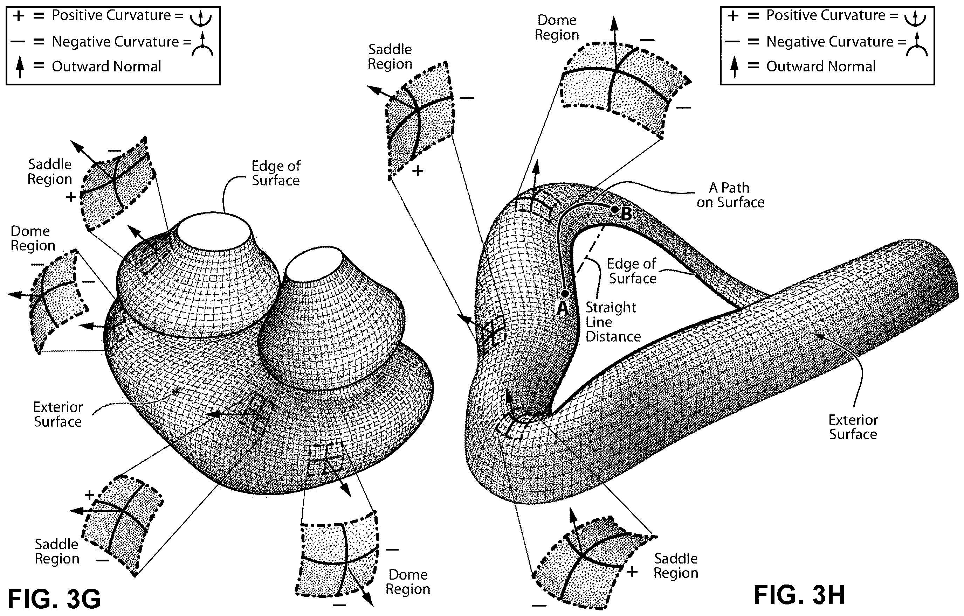

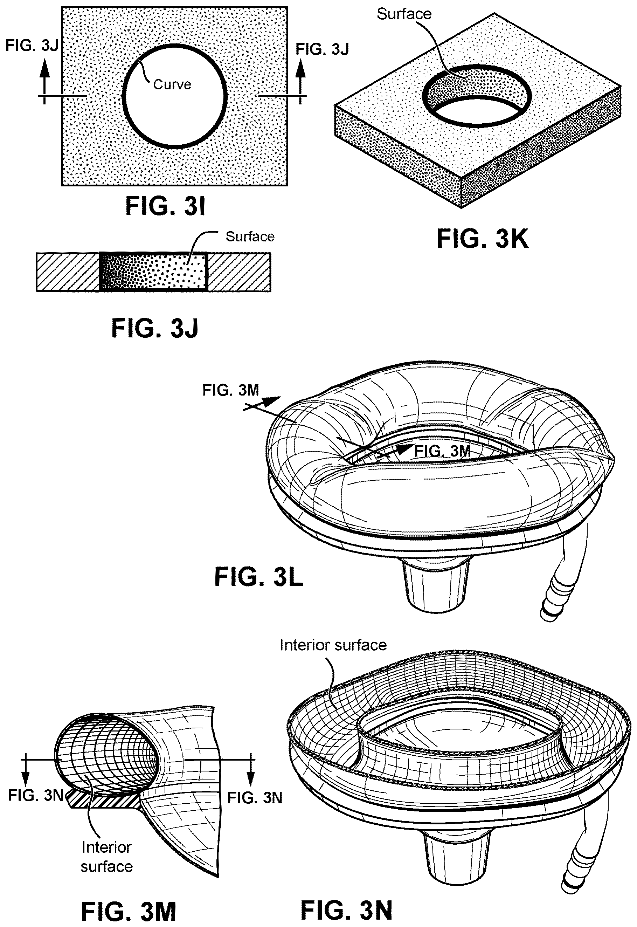

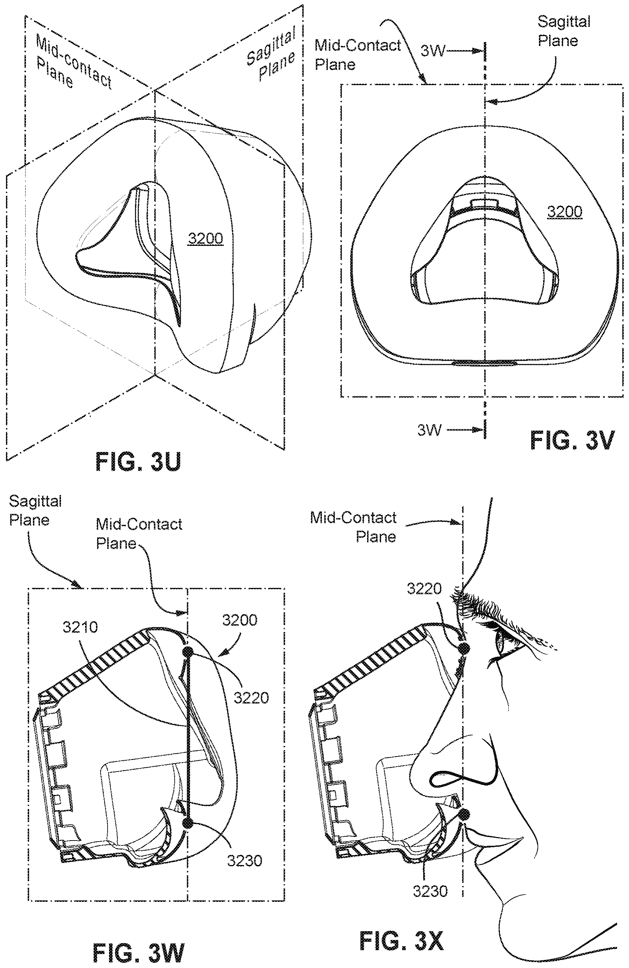

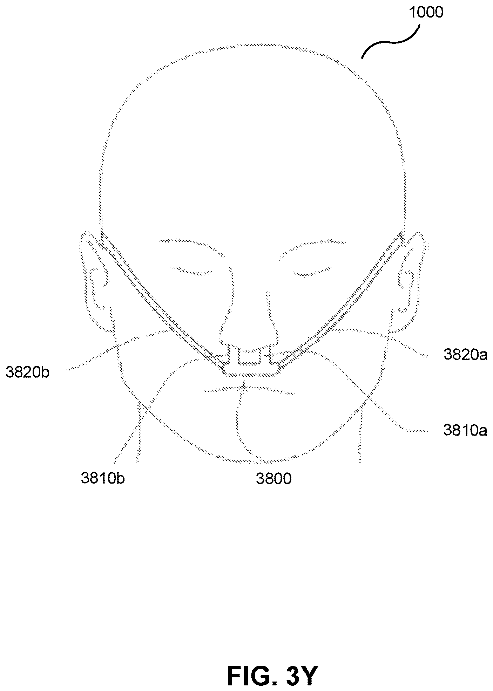

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.

Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.

Fig. 3Y shows a patient interface in the form of a nasal cannula in accordance with one form of the present technology.

4.4 RPT DEVICE

Fig. 4A shows an RPT device in accordance with one form of the present technology.

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

Fig. 4C is a schematic diagram of the electrical components of an RPT device in accordance with one form of the present technology.

Fig. 4D is a schematic diagram of the algorithms implemented in an RPT device in accordance with one form of the present technology.

Fig. 4E is a flow chart illustrating a method carried out by the therapy engine module of Fig. 4D in accordance with one form of the present technology.

4.5 HUMIDIFIER

Fig. 5A shows an isometric view of a humidifier in accordance with one form of the present technology.

Fig. 5B shows an isometric view of a humidifier in accordance with one form of the present technology, showing a humidifier reservoir 5110 removed from the humidifier reservoir dock 5130.

Fig. 5C shows a schematic of a humidifier in accordance with one form of the present technology.

### 4.6 GUIDED PATIENT INTERFACE FITTING

Fig. 6A illustrates an example patient interface 3000 fitting based on a baseline according to one form of the present technology.

Fig. 6B shows a diagram of a communication system between an RPT device 4000, a local external computing device 4288 and a remote external device 4286 according to one form of the present technology.

Fig. 6C illustrates an example of guided patient interface fitting based on a baseline associated with a patient interface 3000 during setup according to one form of the present technology.

Fig. 7A shows a patient interface 3000 including a plurality of measurable indicators 3350 and reference points according to one form of the present technology.

Fig. 7B shows measurable indicators 3350 and/or reference points 3360 according that may be extracted from images captured of the patient and the patient interface according to one form of the present technology.

Fig. 7C shows a side view of an adjustable strap, according to one form of the present technology.

Fig. 7D shows a top view of the strap shown in Fig. 7C according to one form of the present technology.

Fig. 7E shows a top view and a side view of an adjustable strap with no stretch according to one form of the present technology.

Fig. 7F shows a top and side views of an adjustable strap stretched according to one form of the present technology.

Fig. 8A shows a series of display screens that assist a user in setting up a mask (a patient interface) and capturing a baseline of the setup mask.

Fig. 8B shows a series of display screens that assist a user in correcting positioning and setup of a mask (a patient interface).

Fig. 8C shows a series of display screens that assist a user in setting up a mask (a patient interface).

Fig. 9 shows a block diagram of an example computing device 600.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.
The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

## 5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

## 5.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

An unsealed patient interface 3800, in the form of a nasal cannula, includes nasal prongs 3810a, 3810b which can deliver air to respective nares of the patient 1000 via respective orifices in their tips. Such nasal prongs do not generally form a seal with the inner or outer skin surface of the nares. The air to the nasal prongs may be delivered by one or more air supply lumens 3820a, 3820b that are coupled with the nasal cannula 3800. The lumens 3820a, 3820b lead from the nasal cannula 3800 to a respiratory therapy device via an air circuit. The unsealed patient interface 3800 is particularly suitable for delivery of flow therapies, in which the RPT device generates the flow of air at controlled flow rates rather than controlled pressures. The "vent" at the unsealed patient interface 3800, through which excess airflow escapes to ambient, is the passage between the end of the prongs 3810a and 3810b of the cannula 3800 via the patient's nares to atmosphere.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH$_2$O with respect to ambient.

The patient interface 3000 in accordance with one form of the present technology is constructed and arranged to be able to provide a supply of air at a positive pressure of at least 10 cmH$_2$O with respect to ambient.

The patient interface 3000 in accordance with one form of the present technology is constructed and arranged to be able to provide a supply of air at a positive pressure of at least 20 cmH$_2$O with respect to ambient.

### 5.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 5.3.1.1 Sealing mechanisms

In one form, the seal-forming structure includes a sealing flange utilizing a pressure assisted sealing mechanism.

In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 5.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 5.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 5.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 5.3.1.5 Forehead region

In one form, the seal-forming structure that forms a seal in use on a forehead region of the patient's face. In such a form, the plenum chamber may cover the eyes in use.

### 5.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

**5.3.2 Plenum chamber**

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

**5.3.3 Positioning and stabilising structure**

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another suitable for a small sized head, but not a large sized head.

### 5.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled $CO_2$ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

### 5.3.5 Decoupling structure(s)

In one form the patient interface 3000 includes at least one decoupling structure, for example, a swivel or a ball and socket.

### 5.3.6 Connection port

Connection port 3600 allows for connection to the air circuit 4170.

### 5.3.7 Forehead support

In one form, the patient interface 3000 includes a forehead support 3700.

### 5.3.8 Anti-asphyxia valve

In one form, the patient interface 3000 includes an anti-asphyxia valve.

### 5.3.9 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 5.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to + 150 L/min while maintaining a positive pressure of at least 6 $cmH_2O$, or at least $10cmH_2O$, or at least 20 $cmH_2O$.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000 or 3800.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000 or 3800.

### 5.4.1.3 Pressure generator

In one form of the present technology, a pressure generator 4140 for producing a flow, or a supply, of air at positive pressure is a controllable blower 4142. For example the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 $cmH_2O$ to about 20 $cmH_2O$, or in other forms up to about 30 $cmH_2O$ when delivering respiratory pressure therapy. The blower may be as described in any one of the following patents or patent applications the contents of which are incorporated herein by reference in their entirety: U.S. Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller 4240.

In other forms, a pressure generator 4140 may be a piston-driven pump, a pressure regulator connected to a high pressure source (e.g. compressed air reservoir), or a bellows.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000 or 3800.

In one form, a signal from a transducer 4270 may be filtered, such as by low-pass, high-pass or band-pass filtering.

#### 5.4.1.4.1 Flow rate sensor

A flow rate sensor 4274 in accordance with the present technology may be based on a differential pressure transducer, for example, an SDP600 Series differential pressure transducer from SENSIRION.

In one form, a signal generated by the flow rate sensor 4274 and representing a flow rate is received by the central controller 4230.

#### 5.4.1.4.2 Pressure sensor

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal generated by the pressure sensor 4272 is received by the central controller 4230.

#### 5.4.1.4.3 Motor speed transducer

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

### 5.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

In one form, the input device 4220 may include an imaging sensor configured to capture an image and/or series of images (e.g., a video).

### 5.4.2.3 Central controller

In one form of the present technology, the central controller 4230 is one or a plurality of processors suitable to control an RPT device 4000.

Suitable processors may include an x86 INTEL processor, a processor based on ARM® Cortex®-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit.

In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms 4300 expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

### 5.4.2.4 Clock

The RPT device 4000 may include a clock 4232 that is connected to the central controller 4230.

### 5.4.2.5 Therapy device controller

In one form of the present technology, therapy device controller 4240 is a therapy control module 4330 that forms part of the algorithms 4300 executed by the central controller 4230.

In one form of the present technology, therapy device controller 4240 is a dedicated motor control integrated circuit. For example, in one form a MC33035 brushless DC motor controller, manufactured by ONSEMI is used.

### 5.4.2.6 Protection circuits

The one or more protection circuits 4250 in accordance with the present technology may comprise an electrical protection circuit, a temperature and/or pressure safety circuit.

### 5.4.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

### 5.4.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (e.g. via Ethernet, or optical fiber) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

### 5.4.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

#### 5.4.2.9.1 Display driver

A display driver 4292 receives as an input the characters, symbols, or images intended for display on the display 4294, and converts them to commands that cause the display 4294 to display those characters, symbols, or images.

#### 5.4.2.9.2 Display

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 5.4.3 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

### 5.4.3.1 Pre-processing module

A pre-processing module 4310 in accordance with one form of the present technology receives as an input a signal from a transducer 4270, for example a flow rate sensor 4274 or pressure sensor 4272, and performs one or more process steps to calculate one or more output values that will be used as an input to another module, for example a therapy engine module 4320.

In one form of the present technology, the output values include the interface pressure *Pm,* the respiratory flow rate *Qr,* and the leak flow rate *Ql.*

In various forms of the present technology, the pre-processing module 4310 comprises one or more of the following algorithms: interface pressure estimation 4312, vent flow rate estimation 4314, leak flow rate estimation 4316, and respiratory flow rate estimation 4318.

### 5.4.3.1.1 Interface pressure estimation

In one form of the present technology, an interface pressure estimation algorithm 4312 receives as inputs a signal from the pressure sensor 4272 indicative of the pressure in the pneumatic path proximal to an outlet of the pneumatic block (the device pressure *Pd*) and a signal from the flow rate sensor 4274 representative of the flow rate of the airflow leaving the RPT device 4000 (the device flow rate *Qd).* The device flow rate *Qd,* absent any supplementary gas 4180, may be used as the total flow rate *Qt.* The interface pressure algorithm 4312 estimates the pressure drop $\Delta P$ through the air circuit 4170. The dependence of the pressure drop $\Delta P$ on the total flow rate *Qt* may be modelled for the particular air circuit 4170 by a pressure drop characteristic $\Delta P(Q)$. The interface pressure estimation algorithm, 4312 then provides as an output an estimated pressure, *Pm,* in the patient interface 3000 or 3800. The pressure, *Pm,* in the patient interface 3000 or 3800 may be estimated as the device pressure *Pd* minus the air circuit pressure drop $\Delta P.$

### 5.4.3.1.2 Vent flow rate estimation

In one form of the present technology, a vent flow rate estimation algorithm 4314 receives as an input an estimated pressure, *Pm,* in the patient interface 3000 or 3800 from the interface pressure estimation algorithm 4312 and estimates a vent flow rate of air, *Qv,* from a vent 3400 in a patient interface 3000 or 3800. The dependence of the vent flow rate *Qv* on the interface pressure *Pm* for the particular vent 3400 in use may be modelled by a vent characteristic *Qv(Pm).*

### 5.4.3.1.3 Leak flow rate estimation

In one form of the present technology, a leak flow rate estimation algorithm 4316 receives as an input a total flow rate, *Qt,* and a vent flow rate *Qv,* and provides as an output an estimate of the leak flow rate *Ql.* In one form, the leak flow rate estimation algorithm estimates the leak flow rate *Ql* by calculating an average of the difference between total flow rate *Qt* and vent flow rate Qv over a period sufficiently long to include several breathing cycles, e.g. about 10 seconds.

In one form, the leak flow rate estimation algorithm 4316 receives as an input a total flow rate *Qt,* a vent flow rate *Qv,* and an estimated pressure, *Pm,* in the patient interface 3000 or 3800, and provides as an output a leak flow rate *Ql,* by calculating a leak conductance, and determining a leak flow rate *Ql* to be a function of leak conductance and pressure, *Pm.* Leak conductance is calculated as the quotient of low pass filtered non-vent flow rate equal to the difference between total flow rate *Qt* and vent flow rate *Qv,* and low pass filtered square root of pressure *Pm,* where the low pass filter time constant has a value sufficiently long to include several breathing cycles, e.g. about 10 seconds. The leak flow rate *Ql* may be estimated as the product of leak conductance and a function of pressure, *Pm.*

### 5.4.3.1.4 Respiratory flow rate estimation

In one form of the present technology, a respiratory flow rate estimation algorithm 4318 receives as an input a total flow rate, *Qt,* a vent flow rate, *Qv,* and a leak flow rate, *Ql,* and estimates a respiratory flow rate of *air, Qr,* to the patient, by subtracting the vent flow rate *Qv* and the leak flow rate *Ql* from the total flow rate *Qt.*

### 5.4.3.2 Therapy Engine Module

In one form of the present technology, a therapy engine module 4320 receives as inputs one or more of a pressure, *Pm,* in a patient interface 3000 or 3800, and a respiratory flow rate of air to a patient, *Qr,* and provides as an output one or more therapy parameters.

In one form of the present technology, a therapy parameter is a treatment pressure *Pt.*

In one form of the present technology, therapy parameters are one or more of an amplitude of a pressure variation, a base pressure, and a target ventilation.

In various forms, the therapy engine module 4320 comprises one or more of the following algorithms: phase determination 4321, waveform determination 4322, ventilation determination 4323, inspiratory flow limitation determination 4324, apnea / hypopnea determination 4325, snore determination 4326, airway patency determination 4327, target ventilation determination 4328, and therapy parameter determination 4329.

### 5.4.3.2.1 Phase determination

In one form of the present technology, the RPT device 4000 does not determine phase.

In one form of the present technology, a phase determination algorithm 4321 receives as an input a signal indicative of respiratory flow rate, $Qr$, and provides as an output a phase $\Phi$ of a current breathing cycle of a patient 1000.

In some forms, known as discrete phase determination, the phase output $\Phi$ is a discrete variable. One implementation of discrete phase determination provides a bi-valued phase output $\Phi$ with values of either inhalation or exhalation, for example represented as values of 0 and 0.5 revolutions respectively, upon detecting the start of spontaneous inhalation and exhalation respectively. RPT devices 4000 that "trigger" and "cycle" effectively perform discrete phase determination, since the trigger and cycle points are the instants at which the phase changes from exhalation to inhalation and from inhalation to exhalation, respectively. In one implementation of bi-valued phase determination, the phase output $\Phi$ is determined to have a discrete value of 0 (thereby "triggering" the RPT device 4000) when the respiratory flow rate $Qr$ has a value that exceeds a positive threshold, and a discrete value of 0.5 revolutions (thereby "cycling" the RPT device 4000) when a respiratory flow rate $Qr$ has a value that is more negative than a negative threshold. The inhalation time $Ti$ and the exhalation time $Te$ may be estimated as typical values over many respiratory cycles of the time spent with phase $\Phi$ equal to 0 (indicating inspiration) and 0.5 (indicating expiration) respectively.

Another implementation of discrete phase determination provides a tri-valued phase output $\Phi$ with a value of one of inhalation, mid-inspiratory pause, and exhalation.

In other forms, known as continuous phase determination, the phase output $\Phi$ is a continuous variable, for example varying from 0 to 1 revolutions, or 0 to $2\pi$ radians. RPT devices 4000 that perform continuous phase determination may trigger and cycle when the continuous phase reaches 0 and 0.5 revolutions, respectively. In one implementation of continuous phase determination, a continuous value of phase $\Phi$ is determined using a fuzzy logic analysis of the respiratory flow rate $Qr$. A continuous value of phase determined in this implementation is often referred to as "fuzzy phase". In one implementation of a fuzzy phase determination algorithm 4321, the following rules are applied to the respiratory flow rate $Qr$:

1. If the respiratory flow rate is zero and increasing fast then the phase is 0 revolutions.

2. If the respiratory flow rate is large positive and steady then the phase is 0.25 revolutions.

3. If the respiratory flow rate is zero and falling fast, then the phase is 0.5 revolutions.

4. If the respiratory flow rate is large negative and steady then the phase is 0.75 revolutions.

5. If the respiratory flow rate is zero and steady and the 5-second low-pass filtered absolute value of the respiratory flow rate is large then the phase is 0.9 revolutions.

6. If the respiratory flow rate is positive and the phase is expiratory, then the phase is 0 revolutions.

7. If the respiratory flow rate is negative and the phase is inspiratory, then the phase is 0.5 revolutions.

8. If the 5-second low-pass filtered absolute value of the respiratory flow rate is large, the phase is increasing at a steady rate equal to the patient's breathing rate, low-pass filtered with a time constant of 20 seconds.

The output of each rule may be represented as a vector whose phase is the result of the rule and whose magnitude is the fuzzy extent to which the rule is true. The fuzzy extent to which the respiratory flow rate is "large", "steady", etc. is determined with suitable membership functions. The results of the rules, represented as vectors, are then combined by some function such as taking the centroid. In such a combination, the rules may be equally weighted, or differently weighted.

In another implementation of continuous phase determination, the phase $\Phi$ is first discretely estimated from the respiratory flow rate $Qr$ as described above, as are the inhalation time $Ti$ and the exhalation time $Te$. The continuous phase $\Phi$ at any instant may be determined as the half the proportion of the inhalation time $Ti$ that has elapsed since the previous trigger instant, or 0.5 revolutions plus half the proportion of the exhalation time $Te$ that has elapsed since the previous cycle instant (whichever instant was more recent).

### 5.4.3.2.2 Waveform determination

In one form of the present technology, the therapy parameter determination algorithm 4329 provides an approximately constant treatment pressure throughout a respiratory cycle of a patient.

In other forms of the present technology, the therapy control module 4330 controls the pressure generator 4140 to provide a treatment pressure $Pt$ that varies as a function of phase $\Phi$ of a respiratory cycle of a patient according to a waveform template $\Pi(\Phi)$.

In one form of the present technology, a waveform determination algorithm 4322 provides a waveform template $\Pi(\Phi)$ with values in the range [0, 1] on the domain of phase values $\Phi$ provided by the phase determination algorithm 4321 to be used by the therapy parameter determination algorithm 4329.

In one form, suitable for either discrete or continuously-valued phase, the waveform template $\Pi(\Phi)$ is a square-wave template, having a value of 1 for values of phase up to and including 0.5 revolutions, and a value of 0 for values of phase above 0.5 revolutions. In one form, suitable for continuously-valued phase, the waveform template $\Pi(\Phi)$ comprises two smoothly curved portions, namely a smoothly curved (e.g. raised cosine) rise from 0 to 1 for values of phase up to 0.5 revolutions, and a smoothly curved (e.g. exponential) decay from 1 to 0 for values of phase above 0.5 revolutions. In one form, suitable for continuously-valued phase, the waveform template $\Pi(\Phi)$ is based on a square wave, but with a smooth rise from 0 to 1 for values of phase up to a "rise time" that is less than 0.5 revolutions, and a smooth fall from 1 to 0 for values of phase within a "fall time" after 0.5 revolutions, with a "fall time" that is less than 0.5 revolutions.

In some forms of the present technology, the waveform determination algorithm 4322 selects a waveform template $\Pi(\Phi)$ from a library of waveform templates, dependent on a setting of the RPT device. Each waveform template $\Pi(\Phi)$ in the library may be provided as a lookup table of values $\Pi$ against phase values $\Phi$. In other forms, the waveform determination algorithm 4322 computes a waveform template $\Pi(\Phi)$ "on the fly" using a predetermined functional form, possibly parametrised by one or more parameters (e.g. time constant of an exponentially curved portion). The parameters of the functional form may be predetermined or dependent on a current state of the patient 1000.

In some forms of the present technology, suitable for discrete bi-valued phase of either inhalation ($\Phi = 0$ revolutions) or exhalation ($\Phi = 0.5$ revolutions), the waveform determination algorithm 4322 computes a waveform template $\Pi$ "on the fly" as a function of both discrete phase $\Phi$ and time $t$ measured since the most recent trigger instant. In one such form, the waveform determination algorithm 4322 computes the waveform template $\Pi(\Phi, t)$ in two portions (inspiratory and expiratory) as follows:

$$\Pi(\Phi, t) = \begin{cases} \Pi_i(t), & \Phi = 0 \\ \Pi_e(t - T_i), & \Phi = 0.5 \end{cases}$$

where $\Pi_i(t)$ and $\Pi_e(t)$ are inspiratory and expiratory portions of the waveform template $\Pi(\Phi, t)$. In one such form, the inspiratory portion $\Pi_i(t)$ of the waveform template is a smooth rise from 0 to 1 parametrised by a rise time, and the expiratory portion $\Pi_e(t)$ of the waveform template is a smooth fall from 1 to 0 parametrised by a fall time.

### 5.4.3.2.3 Ventilation determination

In one form of the present technology, a ventilation determination algorithm 4323 receives an input a respiratory flow rate $Qr$, and determines a measure indicative of current patient ventilation, *Vent.*

In some implementations, the ventilation determination algorithm 4323 determines a measure of ventilation *Vent* that is an estimate of actual patient ventilation. One such implementation is to take half the absolute value of respiratory flow rate, $Qr$, optionally filtered by low-pass filter such as a second order Bessel low-pass filter with a corner frequency of 0.11 Hz.

In other implementations, the ventilation determination algorithm 4323 determines a measure of ventilation *Vent* that is broadly proportional to actual patient ventilation. One such implementation estimates peak respiratory flow rate *Qpeak* over the inspiratory portion of the cycle. This and many other procedures involving sampling the respiratory flow rate $Qr$ produce measures which are broadly proportional to ventilation, provided the flow rate waveform shape does not vary very much (here, the shape of two breaths is taken to be similar when the flow rate waveforms of the breaths normalised in time and amplitude are similar). Some simple examples include the median positive respiratory flow rate, the median of the absolute value of respiratory flow rate, and the standard deviation of flow rate. Arbitrary linear combinations of arbitrary order statistics of the absolute value of respiratory flow rate using positive coefficients, and even some using both positive and negative coefficients, are approximately proportional to ventilation. Another example is the mean of the respiratory flow rate in the middle K proportion (by time) of the inspiratory portion, where $0 < K < 1$. There is an arbitrarily large number of measures that are exactly proportional to ventilation if the flow rate shape is constant.

### 5.4.3.2.4 Determination of Inspiratory Flow limitation

In one form of the present technology, the central controller 4230 executes an inspiratory flow limitation determination algorithm 4324 for the determination of the extent of inspiratory flow limitation.

In one form, the inspiratory flow limitation determination algorithm 4324 receives as an input a respiratory flow rate signal $Qr$ and provides as an output a metric of the extent to which the inspiratory portion of the breath exhibits inspiratory flow limitation.

In one form of the present technology, the inspiratory portion of each breath is identified by a zero-crossing detector. A number of evenly spaced points (for example, sixty-five), representing points in time, are interpolated by an interpolator along the inspiratory flow rate-time curve for each breath. The curve described by the points is then scaled by a scalar to have unity length (duration/period) and unity area to remove the effects of changing breathing rate and depth. The scaled breaths are then compared in a comparator with a pre-stored template representing a normal unobstructed breath. Breaths deviating by more than a specified threshold (typically 1 scaled unit) at any time during the inspiration from this template, such as those due to coughs, sighs, swallows and hiccups, as determined by a test element, are rejected. For non-rejected data, a moving average of the first such scaled point is calculated by the central controller 4230 for the preceding several inspiratory events. This is repeated over the same inspiratory events for the second such point, and so on. Thus, for example, sixty five scaled data points are generated by the central controller 4230, and represent a moving average of the preceding several inspiratory events, e.g., three events. The moving average of continuously updated values of the (e.g., sixty five) points are hereinafter called the "scaled flow rate ", designated as $Qs(t)$. Alternatively, a single inspiratory event can be utilised rather than a moving average.

From the scaled flow rate, two shape factors relating to the determination of partial obstruction may be calculated.

Shape factor 1 is the ratio of the mean of the middle (e.g. thirty-two) scaled flow rate points to the mean overall (e.g. sixty-five) scaled flow rate points. Where this ratio is in excess of unity, the breath will be taken to be normal. Where the ratio is unity or less, the breath will be taken to be obstructed. A ratio of about 1.17 is taken as a threshold between partially obstructed and unobstructed breathing, and equates to a degree of obstruction that would permit maintenance of adequate oxygenation in a typical patient.

Shape factor 2 is calculated as the RMS deviation from unit scaled flow rate, taken over the middle (e.g. thirty two) points. An RMS deviation of about 0.2 units is taken to be normal. An RMS deviation of zero is taken to be a totally flow-limited breath. The closer the RMS deviation to zero, the breath will be taken to be more flow limited.

Shape factors 1 and 2 may be used as alternatives, or in combination. In other forms of the present technology, the number of sampled points, breaths and middle points may differ from those described above. Furthermore, the threshold values can be other than those described.

### 5.4.3.2.5 Determination of apneas and hypopneas

In one form of the present technology, the central controller 4230 executes an apnea / hypopnea determination algorithm 4325 for the determination of the presence of apneas and/or hypopneas.

In one form, the apnea / hypopnea determination algorithm 4325 receives as an input a respiratory flow rate signal $Qr$ and provides as an output a flag that indicates that an apnea or a hypopnea has been detected.

In one form, an apnea will be said to have been detected when a function of respiratory flow rate $Qr$ falls below a flow rate threshold for a predetermined period of time. The function may determine a peak flow rate, a relatively short-term mean flow rate, or a flow rate intermediate of relatively short-term mean and peak flow rate, for example an RMS flow rate. The flow rate threshold may be a relatively long-term measure of flow rate.

In one form, a hypopnea will be said to have been detected when a function of respiratory flow rate $Qr$ falls below a second flow rate threshold for a predetermined period of time. The function may determine a peak flow, a relatively short-term mean flow rate, or a flow rate intermediate of relatively short-term mean and peak flow rate, for example an RMS flow rate. The second flow rate threshold may be a relatively long-term measure of flow rate. The second flow rate threshold is greater than the flow rate threshold used to detect apneas.

### 5.4.3.2.6 Determination of snore

In one form of the present technology, the central controller 4230 executes one or more snore determination algorithms 4326 for the determination of the extent of snore.

In one form, the snore determination algorithm 4326 receives as an input a respiratory flow rate signal $Qr$ and provides as an output a metric of the extent to which snoring is present.

The snore determination algorithm 4326 may comprise the step of determining the intensity of the flow rate signal in the range of 30-300 Hz. Further, the snore determination algorithm 4326 may comprise a step of filtering the respiratory flow rate signal $Qr$ to reduce background noise, e.g., the sound of airflow in the system from the blower.

### 5.4.3.2.7 Determination of airway patency

In one form of the present technology, the central controller 4230 executes one or more airway patency determination algorithms 4327 for the determination of the extent of airway patency.

In one form, the airway patency determination algorithm 4327 receives as an input a respiratory flow rate signal *Qr,* and determines the power of the signal in the frequency range of about 0.75 Hz and about 3 Hz. The presence of a peak in this frequency range is taken to indicate an open airway. The absence of a peak is taken to be an indication of a closed airway.

In one form, the frequency range within which the peak is sought is the frequency of a small forced oscillation in the treatment pressure *Pt.* In one implementation, the forced oscillation is of frequency 2 Hz with amplitude about 1 cmH$_2$O.

In one form, airway patency determination algorithm 4327 receives as an input a respiratory flow rate signal *Qr,* and determines the presence or absence of a cardiogenic signal. The absence of a cardiogenic signal is taken to be an indication of a closed airway.

### 5.4.3.2.8 Determination of target ventilation

In one form of the present technology, the central controller 4230 takes as input the measure of current ventilation, *Vent,* and executes one or more target ventilation determination algorithms 4328 for the determination of a target value *Vtgt* for the measure of ventilation.

In some forms of the present technology, there is no target ventilation determination algorithm 4328, and the target value *Vtgt* is predetermined, for example by hard-coding during configuration of the RPT device 4000 or by manual entry through the input device 4220.

In other forms of the present technology, such as adaptive servo-ventilation (ASV), the target ventilation determination algorithm 4328 computes a target value *Vtgt* from a value *Vtyp* indicative of the typical recent ventilation of the patient.

In some forms of adaptive servo-ventilation, the target ventilation *Vtgt* is computed as a high proportion of, but less than, the typical recent ventilation *Vtyp.* The high proportion in such forms may be in the range (80%, 100%), or (85%, 95%), or (87%, 92%).

In other forms of adaptive servo-ventilation, the target ventilation *Vtgt* is computed as a slightly greater than unity multiple of the typical recent ventilation *Vtyp.*

The typical recent ventilation *Vtyp* is the value around which the distribution of the measure of current ventilation *Vent* over multiple time instants over some predetermined timescale tends to cluster, that is, a measure of the central tendency of the measure of current ventilation over recent history. In one implementation of the target ventilation determination algorithm 4328, the recent history is of the order of several minutes, but in any case should be longer than the timescale of Cheyne-Stokes waxing and waning cycles. The target ventilation determination algorithm 4328 may use any of the variety of well-known measures of central tendency to determine the typical recent ventilation *Vtyp* from the measure of current ventilation, *Vent.* One such measure is the output of a low-pass filter on the measure of current ventilation *Vent,* with time constant equal to one hundred seconds.

### 5.4.3.2.9 Determination of therapy parameters

In some forms of the present technology, the central controller 4230 executes one or more therapy parameter determination algorithms 4329 for the determination of one or more therapy parameters using the values returned by one or more of the other algorithms in the therapy engine module 4320.

In one form of the present technology, the therapy parameter is an instantaneous treatment pressure *Pt.* In one implementation of this form, the therapy parameter determination algorithm 4329 determines the treatment pressure *Pt* using the equation

$$Pt = A\Pi(\Phi, t) + P_0 \qquad\qquad (1)$$

where:

- *A* is the amplitude,
- $\Pi(\Phi, t)$ is the waveform template value (in the range 0 to 1) at the current value $\Phi$ of phase and *t* of time, and
- $P_0$ is a base pressure.

If the waveform determination algorithm 4322 provides the waveform template $\Pi(\Phi, t)$ as a lookup table of values

$\Pi$ indexed by phase $\Phi$, the therapy parameter determination algorithm 4329 applies equation (1) by locating the nearest lookup table entry to the current value $\Phi$ of phase returned by the phase determination algorithm 4321, or by interpolation between the two entries straddling the current value $\Phi$ of phase.

The values of the amplitude A and the base pressure $P_0$ may be set by the therapy parameter determination algorithm 4329 depending on the chosen respiratory pressure therapy mode in the manner described below.

### 5.4.3.3 Therapy Control module

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the present technology, the therapy parameter is a treatment pressure $Pt$, and the therapy control module 4330 controls the pressure generator 4140 to deliver a flow of air whose interface pressure $Pm$ at the patient interface 3000 or 3800 is equal to the treatment pressure $Pt$.

### 5.4.3.4 Detection of fault conditions

In one form of the present technology, the central controller 4230 executes one or more methods 4340 for the detection of fault conditions. The fault conditions detected by the one or more methods 4340 may include at least one of the following:

- Power failure (no power, or insufficient power)
- Transducer fault detection
- Failure to detect the presence of a component
- Operating parameters outside recommended ranges (e.g. pressure, flow rate, temperature, $PaO_2$)
- Failure of a test alarm to generate a detectable alarm signal.

Upon detection of the fault condition, the corresponding algorithm 4340 signals the presence of the fault by one or more of the following:

- Initiation of an audible, visual &/or kinetic (e.g. vibrating) alarm
- Sending a message to an external device
- Logging of the incident

## 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349, which is incorporated herewithin in its entirety by reference.

### 5.5.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3000 or 3800.

## 5.6 HUMIDIFIER

### 5.6.1 Humidifier overview

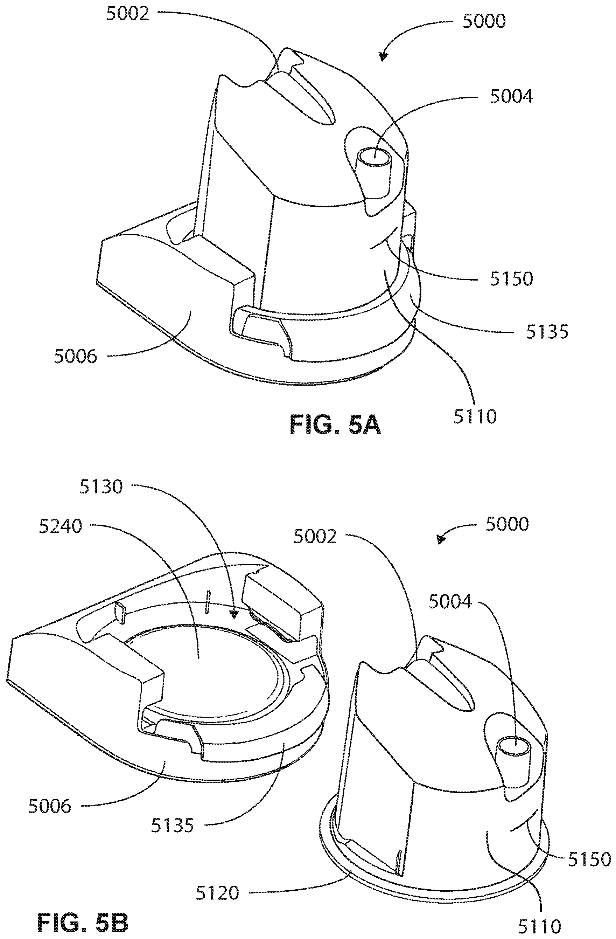

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 5.6.2 Humidifier components

### 5.6.2.1 Water reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 5A and Fig. 5B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

### 5.6.2.2 Conductive portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

### 5.6.2.3 Humidifier reservoir dock

In one form, the humidifier 5000 may comprise a humidifier reservoir dock 5130 (as shown in Fig. 5B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

### 5.6.2.4 Water level indicator

The humidifier reservoir 5110 may comprise a water level indicator 5150 as shown in Fig. 5A-5B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

### 5.6.2.5 Humidifier transducer(s)

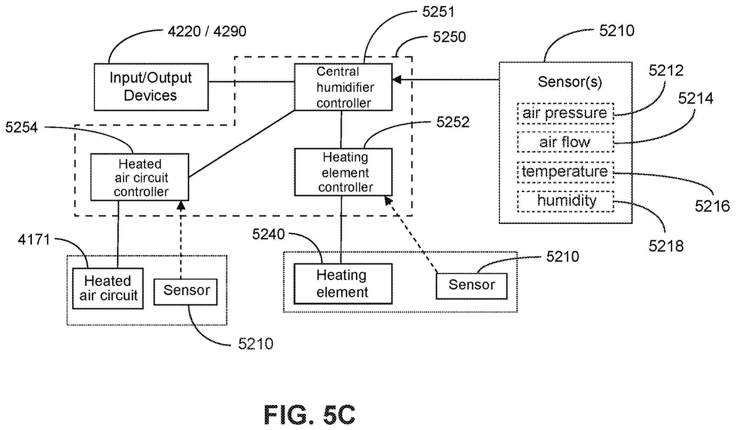

The humidifier 5000 may comprise one or more humidifier transducers (sensors) 5210 instead of, or in addition to, transducers 4270 described above. Humidifier transducers 5210 may include one or more of an air pressure sensor 5212, an *air* flow rate transducer 5214, a temperature sensor 5216, or a humidity sensor 5218 as shown in Fig. 5C. A humidifier transducer 5210 may produce one or more output signals which may be communicated to a controller such as the central controller 4230 and/or the humidifier controller 5250. In some forms, a humidifier transducer may be located externally to the humidifier 5000 (such as in the *air* circuit 4170) while communicating the output signal to the controller.

#### 5.6.2.5.1 Pressure transducer

One or more pressure transducers 5212 may be provided to the humidifier 5000 in addition to, or instead of, a pressure sensor 4272 provided in the RPT device 4000.

#### 5.6.2.5.2 Flow rate transducer

One or more flow rate transducers 5214 may be provided to the humidifier 5000 in addition to, or instead of, a flow rate sensor 4274 provided in the RPT device 4000.

#### 5.6.2.5.3 Temperature transducer

The humidifier 5000 may comprise one or more temperature transducers 5216. The one or more temperature transducers 5216 may be configured to measure one or more temperatures such as of the heating element 5240 and/or of the flow of *air* downstream of the humidifier outlet 5004. In some forms, the humidifier 5000 may further comprise a temperature sensor 5216 to detect the temperature of the ambient air.

#### 5.6.2.5.4 Humidity transducer

In one form, the humidifier 5000 may comprise one or more humidity sensors 5218 to detect a humidity of a gas, such as the ambient air. The humidity sensor 5218 may be placed towards the humidifier outlet 5004 in some forms to measure a humidity of the gas delivered from the humidifier 5000. The humidity sensor may be an absolute humidity sensor or a relative humidity sensor.

### 5.6.2.6 Heating element

A heating element 5240 may be provided to the humidifier 5000 in some cases to provide a heat input to one or more of the volume of water in the humidifier reservoir 5110 and/or to the flow of air. The heating element 5240 may comprise a heat generating component such as an electrically resistive heating track. One suitable example of a heating element 5240 is a layered heating element such as one described in the PCT Patent Application Publication No. WO 2012/171072, which is incorporated herewith by reference in its entirety.

In some forms, the heating element 5240 may be provided in the humidifier base 5006 where heat may be provided to the humidifier reservoir 5110 primarily by conduction as shown in Fig. 5B.

### 5.6.2.7 Humidifier controller

According to one arrangement of the present technology, a humidifier 5000 may comprise a humidifier controller 5250 as shown in Fig. 5C. In one form, the humidifier controller 5250 may be a part of the central controller 4230. In another form, the humidifier controller 5250 may be a separate controller, which may be in communication with the central controller 4230.

In one form, the humidifier controller 5250 may receive as inputs measures of properties (such as temperature, humidity, pressure and/or flow rate), for example of the flow of air, the water in the reservoir 5110 and/or the humidifier 5000. The humidifier controller 5250 may also be configured to execute or implement humidifier algorithms and/or deliver one or more output signals.

As shown in Fig. 5C, the humidifier controller 5250 may comprise one or more controllers, such as a central humidifier controller 5251, a heated air circuit controller 5254 configured to control the temperature of a heated air circuit 4171 and/or a heating element controller 5252 configured to control the temperature of a heating element 5240.

5.7 GUIDED PATIENT INTERFACE FITTING

### 5.7.1 Guided fitting overview

Patient interfaces 3000 associated with medical devices can require specialized knowledge (e.g., from a doctor or other medical professional) to set up and use. Furthermore, even if the patient interface 3000 is initially properly set up, over time adjustments may be needed that require specialised knowledge. However, medical professionals may not always be available to assist patients in setting up or adjusting the patient interface 3000. This may be the case where the patient interface 3000 associated with the medical device is being used in the home of the patient 1000. Without being able to properly set up or adjust the patient interface, the patient 1000 may experience discomfort and stop using the patient interface 3000 and/or receive less effective treatment.

In various forms of the present technology there is provided an application for guiding a patient 1000 on wearing a patient interface 3000 associated with a medical device (e.g., an RPT device 4000), detecting proper and/or improper patient interface positioning, and/or guiding the patient to adjust the patient interface 3000 to correct fitting position of the patient interface 3000.

Leveraging augmented reality, the patient may be connected with knowhow on headgear sizes and fitting based on various fitting range studies conducted on different patient ranges for various components of the patient interface 3000 and/or components coupled to the patient interface 3000. This provides a large database which can be tapped into through a virtual-to-physical interface to aid with patient interface 3000 fitting through efficient headgear sizing and tightening without regards to location of the patient.

As discussed in more detail below, the virtual solution provides a system (e.g., a camera system) to: scan a patient's facial profile, capture key points of facial structure as various data points, and send data to a data bank for analysis (e.g., using cloud technology).

The virtual solution provides the use of existing database (e.g., from the manufacturer's, distributor's, and/or clinician's mask and/or headgear selector tool) to determine key data points on patient's facial structure. The patient's facial structure may be cross referenced with one or more predetermined components of the patient interface 3000 (e.g., headgear strap type and/or length) to calculate for desired component settings (e.g., length and tightening of headgear adjustment). Data may be provided to a patient's device (e.g., a mobile device and/or the RPT devices 4000) to instruct the patient in making the determined component setting (e.g., show amount of tightening required on headgear to achieve good fit of the mask system).

Headgear fitting can be achieved through one or more options including: one or more physical markings on a headgear for manual positioning; physical markers on headgear to determine one or more stretch marks that change to show stretch forces; and/or use current facial measurements to determine length to stretch and fit the headgear, overlay estimated tightening unto patient's facial structure and/or provide visual and audible cues for when the patient has reached the estimated amount of headgear adjustment.

In some examples, to ensure that an adequate seal is achieved, the virtual solution may be integrated with the prescribed flow generator via communication technologies such as Bluetooth or WiFi to determine leakage during therapy.

Examples of the present technology overcome one or more limitations of conventional approaches to patient interface selection, fitting and/or adjustment. For example, conventional approaches provide a user guide in a printed form that may be hard for some patients to understand from pictures and words. Also, patients may need to travel down to a clinician's office for mask fitting or the clinician may need to travel to the location of the patient. This takes up more time and effort from the patient as they need to arrange an appointment (which may take time to organise e.g. sometimes days to weeks to arrange) and travel to the office to receive instructions.

In addition, even after an initial fitting session with the assistance of a clinician, the patient will be left on their own at home to replicate the fitting. If there is a change in the headgear fit or the patient forgets how to replicate the adjustments (e.g., after washing), the patient may either have to arrange another appointment or figure it out on their own, which might lead to improper fitting of the mask system and reduced efficacy of the therapy. Improving the ease of usage of the mask system/headgear will also help to improve compliance and retain patients in therapy.

In addition, if there is a change in the facial profile of the patient (e.g., result of losing weight or gaining weight) the fitting of the headgear will need to change accordingly. If patients are not able to adjust the fit on their own, they will have to obtained updated instructions on refitting the mask e.g. by revisiting the clinician's office.

Examples of the present technology provide for a video and virtual solution that patients can access in the ease of their home, streamlining the process of mask fitting and allowing the patient to start their therapy faster than conventional solutions.

### 5.7.1.1 Guided patient interface fitting based on established baseline

According to one form of the present technology, the patient interface fitting is performed based on a baseline. The baseline may be established by the manufacturer, clinician, during initial use of the patient interface 3000, and/or by the patient during use of the patient interface 3000. The baseline may define proper positioning of the patient interface 3000 on a patient's face. The baseline may be established based on one or more images and/or video of the patient interface on a person's face, which in some examples may be the patient's face. The images may be obtained from a database, captured during initial use, and/or subsequent use of the patient interface 3000.

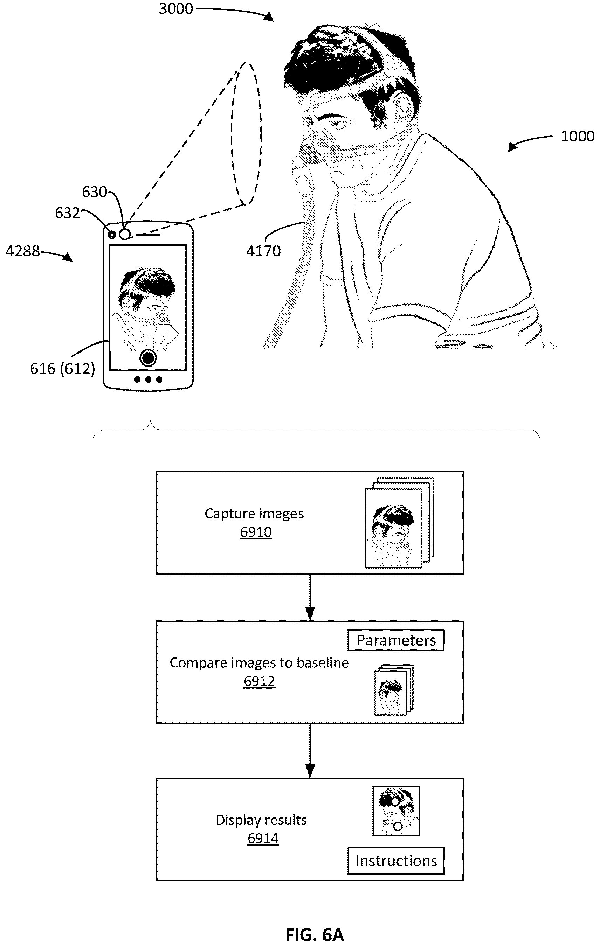

Fig. 6A illustrates an example patient interface 3000 fitting based on a baseline according to one form of the present technology. A patient 1000 wearing the patient interface 3000 may use a local external device 4288 (e.g., a personal computer, mobile phone, tablet and/or remote control) including a camera 630, a display device 616, and an input device 612 to properly position the patient interface 3000 on his face. While the examples are discussed with reference to a local external device 4288, in some forms of the present technology, one or more operations can be performed by an RPT devices 4000 including a camera 630, a display device 616, and/or input device 612.

As shown in Fig. 6A, a processing system of a local external device 4288 may be configured to capture one or more images of the patient 1000 and the patient interface 3000 (Step 6910), compare the images to a baseline (Step 6912), and display results (Step 6914).

The images may be captured after the patient has positioned the patient interface 3000 on the face and an input is received via the input device 612 from the patient to capture the images. A plurality of images may be captured. In some examples, video including a sequence of images may be captured. The local external device 4288 may guide the patient in capturing the images such that images are captures from different positions and/or orientations to capture a complete profile of the patients head and the patient interface 3000.

As shown in Fig. 6A a front facing camera 630 provided on a same side as the display device 616 may be controlled to capture the images while the display device 616 guides the user to position the camera for a next image. The image capturing application may be configured to automatically capture a series of the images as the camera is moved around the patient's head. In some examples, the camera 630 may include an infrared (IR) sensitive imaging sensor configured to capture IR images and/or include filter that enable the camera 630 to capture IR images in addition to and/or in place of regular images of visible light. In some examples, the local external device 4288 may include an IR emitter 632 configured to emit IR signals that can be detected by the camera 630.

The captured images may be compared to data defining a baseline to determine where positioning and/or setting of the patient interface 3000 are different from the baseline. The baseline may be defined by one or more images of the patient interface 3000 on the patient's head or another person's head, and/or other parameters. In some examples, the captured images may be used to generate a profile (e.g., a three-dimensional profile) of the patient and/or the patient interface and the generated profile can be compared to a baseline profile. The positioning and/or orientation of the camera, measured by sensors (e.g., acceleration sensors) in the device, at the time each image is captured can be used in generating the profile.

In some examples, data defining the baseline may include parameters that are associated with the patient interface 3000. The parameters may be assigned to a patient and/or a type (e.g., model and configuration) of patient interface 3000 used by the patient 1000 and the parameters may be used to determine if the patient interface 3000 in the captured images is being worn in the manner defined by the baseline. For example the parameters may define spacing and/or positioning of different components of the patient interface 3000 in relation to each other and/or relation of different component of the patient interface 3000 to facial features of the patient (e.g., eyes, nose, etc.). These parameters may be compared to corresponding data extracted from the captured images.

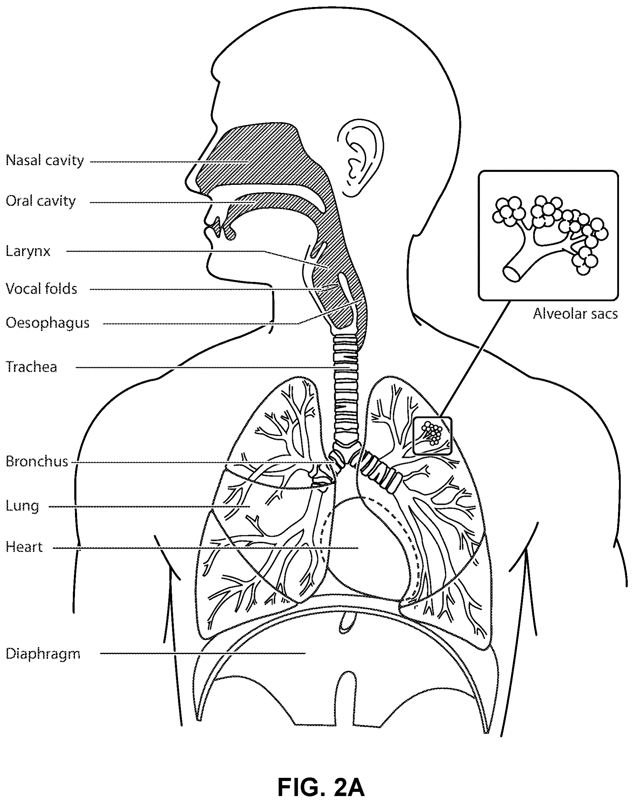

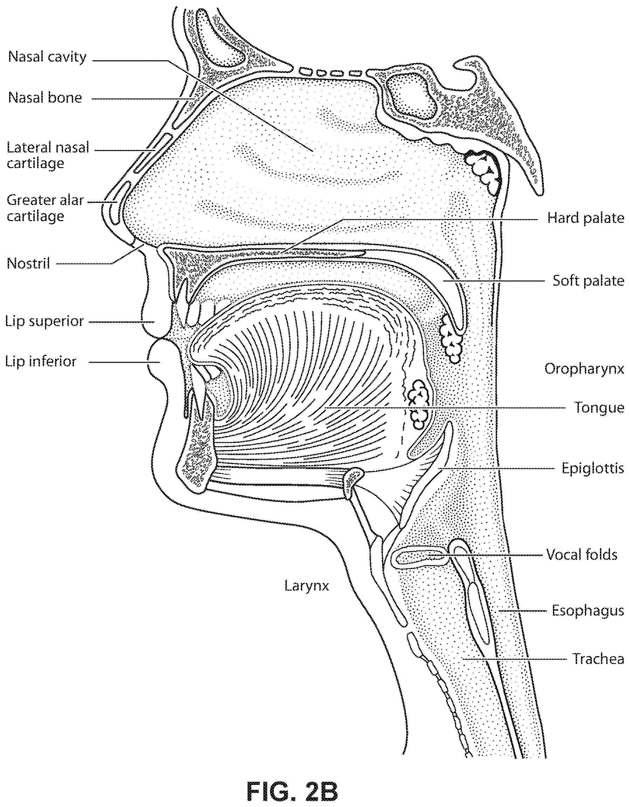

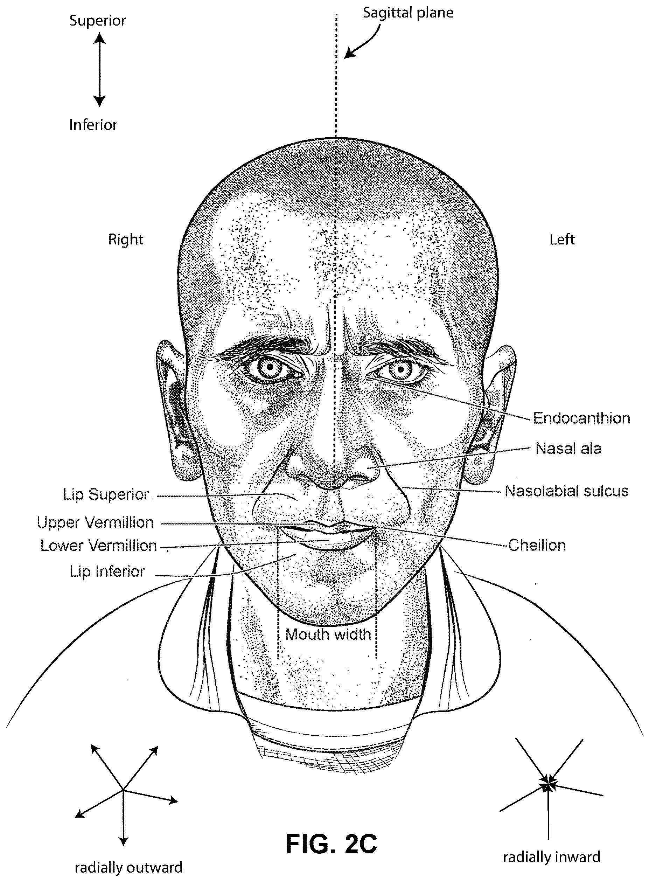

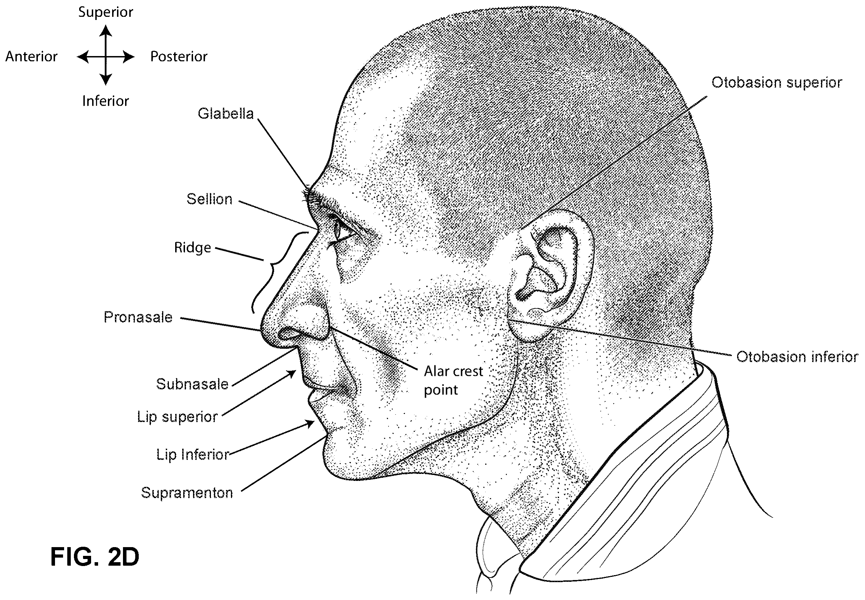

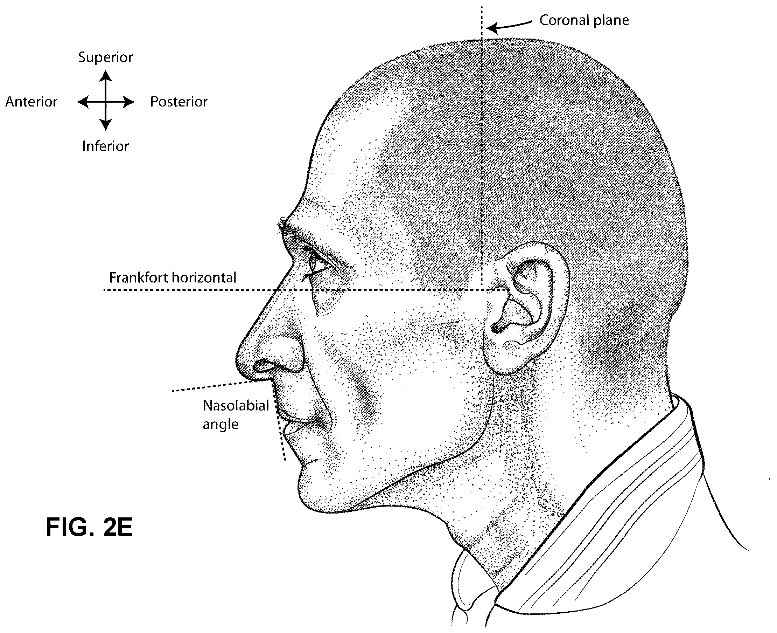

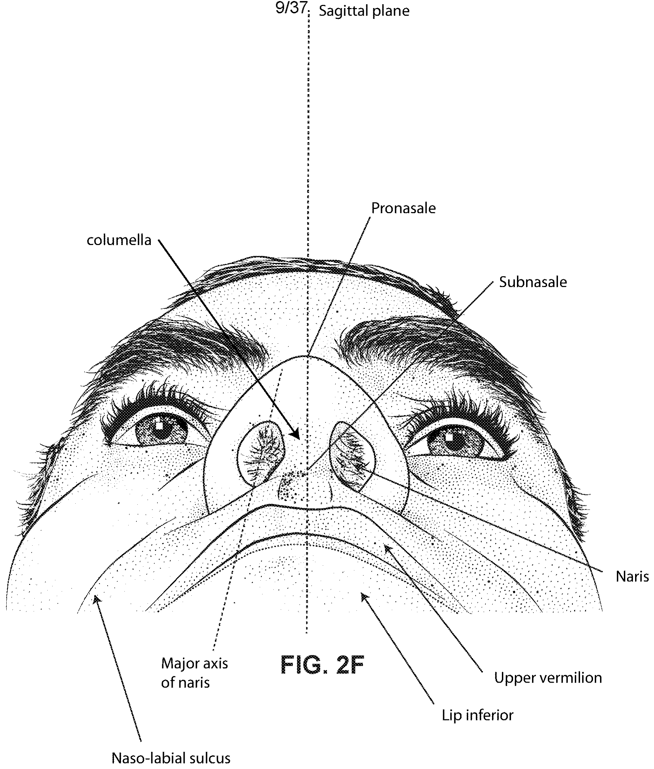

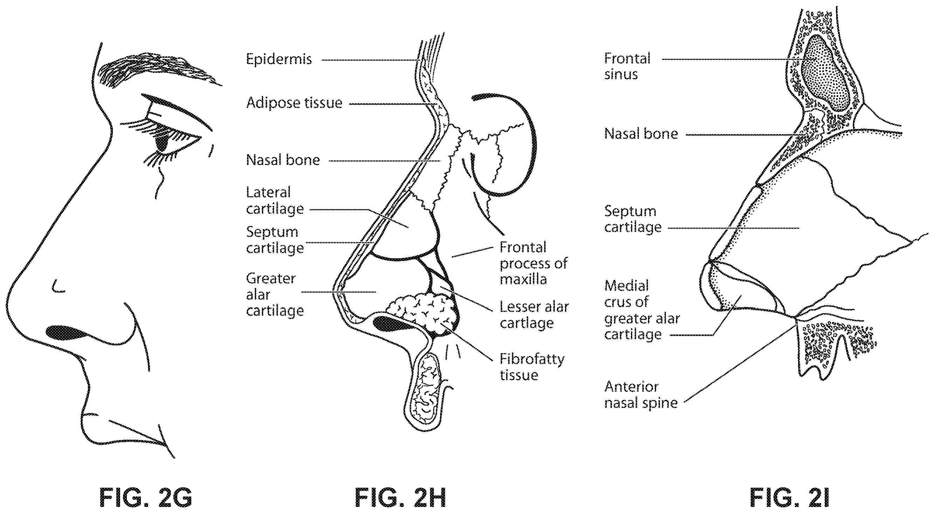

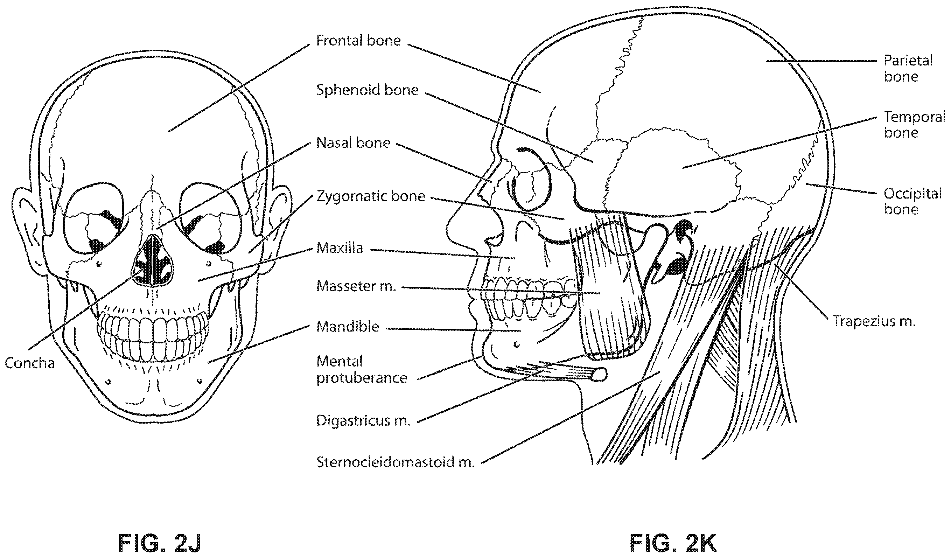

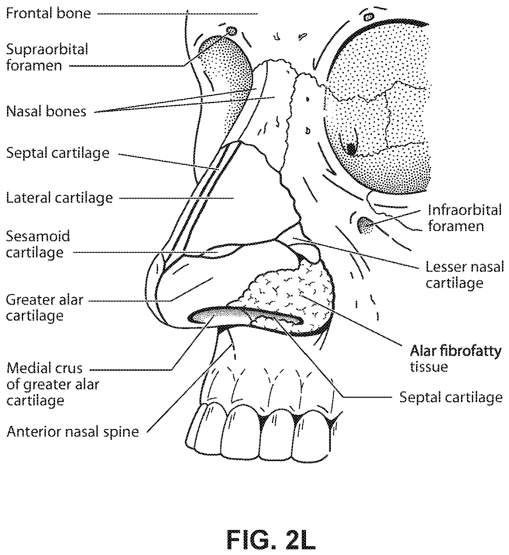

Comparing the images to a baseline may include performing image analysis to detect features in the images. The detected features may include detecting features and/or edges of the patient interface 3000, symbols and/or marks on the patient interface 3000, and/or facial features. The image analysis may include applying one or more filters (e.g., edge detection and features extraction) to the captured images. The detected features may be counted and/or compared to position of other features and/or marks. In some examples, the detected features may include one or more facial features shown in Figs. 2B-2F.

Based on the comparison, the local external device 4288 may display results of the comparison and/or provide instructions for correcting positioning and/or settings of the patient interface 3000. The settings of the patient interface 3000 may include length of tubes and/or straps of the positioning and stabilising structure 3300. The results may indicate that there are one or more issues, provide with options to correct the each of the issues. The results may be provided with text and/or images identifying the issues. The results may include instructions provided with text, images and/or videos explaining and/or showing how to correct the identified issues.

In some examples, the results may include superimposing a patient interface 3000 with the correct position and/or setting on the captured image(s). The results may include an animation including a series of images showing the superimposed patient interface 3000 moving from the position in the captured image to a correct position.

In some examples, the analysis may include detecting missing or broken components of the patient interface 3000, and the displays results showing how these components can be replaced. The displayed results may provide an option to order the missing or broken components. For example, the analysis may determine that a vent on the mask is missing and provide the patient an option to place an order for the missing vent.

### 5.7.2 Communication with remote computer system

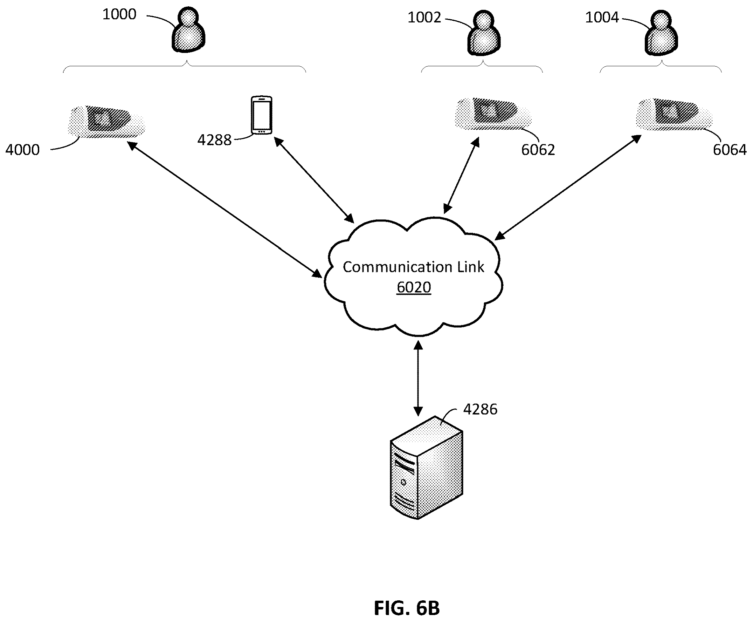

Fig. 6B shows a diagram of a communication system for transmitting data between an RPT device 4000, a local external computing device 4288 and a remote external device 4286 according to one form of the present technology. Fig. 6B includes an RPT devices 4000 associated with a patient 1000. The present technology is not limited to RPT device, but may be applied to other medical devices.

The RPT device 4000 may be configured to communicate via a data communication interface 4280 with a remote external device 4286 and/or local external devices 4288 (e.g., a personal computer, mobile phone, tablet and/or remote control). The local external devices 4288 may be configured to communicate directly with the RPT device 4000 when located in the vicinity of the RPT device 4000 or remotely via a local or external network when the local external device 4288 is not located near the RPT device 4000. The remote external device 4286 may be accessible to an appropriately authorised person such as a clinician, manufacturer, and/or supplier of the device. The remote external device 4286 may include a server and/or a cloud computing platform (e.g., Amazon Web Services™, Google™ cloud platform, Microsoft™ Azure). The remote external device 4286 may include application executable by the RPT device 4000 and/or the local external device 4288 for controlling operation of the RPT device 4000. The remote external device 4286 may include instructions for using the RPT device 4000 and/or the patient interface 3000, and application for troubleshooting operation and/or use of the RPT device 4000 and/or the patient interface 3000.

One or more other medical devices 6062 or 6064 (which may be RPT devices), associated with other patients 1002 and 1004, may be configured to communicate with the remote external device 4286, the server 6030 and/or the cloud computing platform 6040.

The devices illustrated in Fig. 6A may communicate via a communication link 6020 comprising a remote external communication network 4282 and/or a local external communication network 4284.

The RPT device 4000, medical devices 6062 and 6064, and local external computing device 4288 may be configured to receive data from the remote external device 4286 and transmit, via the communication link 6020, data including sensor data, feedback, images, and/or videos to the remote external device 4286. The remote external device 4286 may be configured to create a database to assist with guiding patients in fitting a patient interface 3000. The database may include baseline information associated with specific patient interface and/or a plurality of patients. In some examples, a server and/or the cloud computing platform of the remote external device 4286 may receive patient's data (e.g., age range, gender, weights, environment, facial profile, type of patient interface, type of RPT device, etc.) and categorize the data. The received information may include images captured of the patient and patient interface with a proper fit and images of the patient and patient interface when the patients indicates there are issues with the fit.

The remote external device 4286 may segment the received data to create models, and use the models to guide the user during initial setup of the patient interface 3000, when user experiences issues with the patient interface 3000 and/or when the user needs to readjust the patient interface 3000 (e.g., after changes in facial profile of the patient and/or changes in settings of the patient interface 3000). The models may be used as a baseline to detect improper mask fitting (e.g., based on photos and/or videos of the patient). The models may be predetermined by advanced analytics, artificial intelligence, and/or machine learning. The remote external device 4286 may include models determined based on information received from the user during prior use (e.g., history information) of the same or different patient interfaces 3000, other users (e.g., user associated with medical devices 6062 and 6064) and/or information input by the manufacturer and/or clinician. The advanced analytics, artificial intelligence, and/or machine learning may be performed on data from a large number of patients and the models may be updated with new data as new data (e.g., data including demographic, feedback, images, videos, and/or changes to compliance) become available. The analysis results may include tailored instructions, generated based on information received from other medical devices 6062 and 6064, manufacturer and/or clinician. The advance analytics may modify the baseline and/or solutions for detected issues as additional information is received from other medical devices 6062 and 6064, manufacturer and/or clinician.

In some examples, the different baseline may be determined and stored for each user and/or a patient interface. The database may include equivalent settings (e.g., tightness by straps) from different masks and/or headgears so that the user may be provided with correct settings when the user transitions from using one mask and/or headgear to another mask and/or headgear.

In some examples, the remote external device 4286 may perform the analysis and/or determine instructions for display to the patient. In this example, the remote external device 4286 may receive the captured images of the patient and the patient interface 3000 and perform the analysis to determine issues with the patient interface 3000. Based on the analysis, the determine information/instructions that should be displayed to the patient to correct the identified issues, and transmit the information/instructions to the RPT device 4000and/or the local external computing device 4288.

### 5.7.3 Method for establishing baseline and using baseline

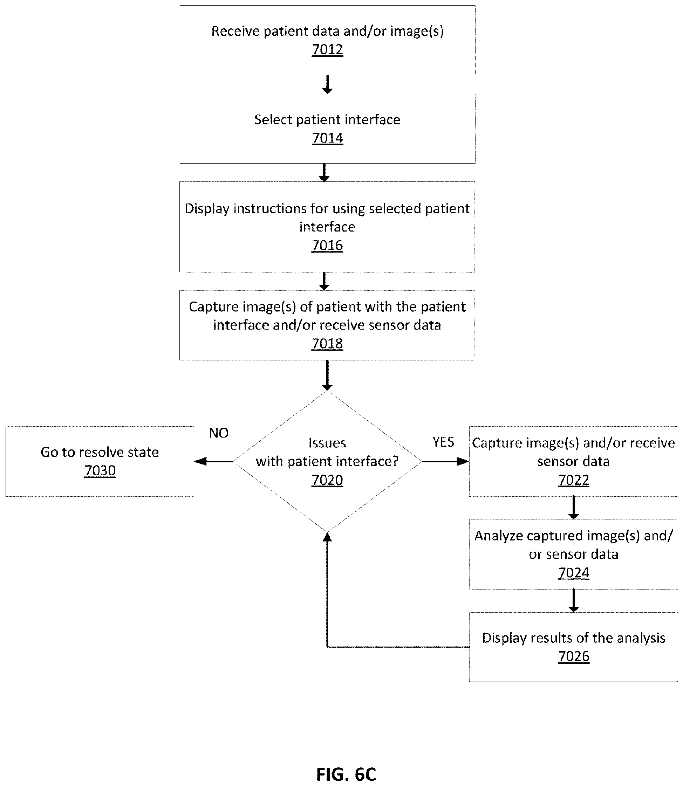

Fig. 6C illustrates an example of guided patient interface fitting based on a baseline associated with a patient interface 3000 during setup according to one form of the present technology. One or more operations shown in Fig. 6C may be performed by an application executed on the RPT devices 4000 and/or an application executed a local external devices (e.g., a personal computer, mobile phone, tablet and/or remote control) used by the patient. In some examples, one or more operations may be performed by an application executing on a remote external device 4286.

The application may provide a user interface to receive patient information and/or one or more images (Step 7012). The received patient information may include patient demographics, such as age, gender, weights, environment, facial profile information, sleep position, and/or type of RPT device. The one or more images may be captured by a camera included in the RPT device 4000 or the local external device. In step 7012, the one or more images may be captured by a camera to obtain a patient's facial profile. In some examples, the one or more images may be analysed to determine key points of facial structure as various data point.

In step 7014, a patient interface 3000 for use is selected. The selection of the patient interface 3000 may be made by an input received from the patient. In some examples, the user may enter model of the patient interface 3000, select the specific configuration of the patient interface 3000, and/or capture an image of the patient interface 3000. In some examples, an image of the patient interface 3000 may include a code provided on patient interface packaging or the patient interface 3000 to identify the patient interface 3000 and/or configuration of the patient interface 3000. The configuration of the patient interface 3000 may identify the type and/or size of stabilising structure 3300 and/or seal forming structure 3100 (e.g., nasal cushion and/or oro-nasal cushion) that is used by the patient.

In some examples, the patient interface 3000 for use may be selected automatically by the application based on the captured images of the patient. Mask fitting and selection systems and methods disclosed in US Patent No. 7,827,038 and/or US Patent No. 8,254,637, each of which is incorporated by reference in their entirety in this application, may be used to determine a mask for the patient.

The captured images may provide one or more different views of the patient's face. The application may generate a three-dimensional profile of the patient's face based on the captured images. In some examples, the application may provide a recommendation for a specific patient interface 3000 based on the three-dimensional profile. The three-dimensional profile may identify relative positioning and/or size of facial features (e.g., eyes, nose, mouth, ears, upper and lower lips, etc.). In some examples, the three-dimensional profile may identify relative positioning of one or more features shown in Figs. 2B-2F.

The recommendation for the patient interface 3000 may be made based on the features identified in the three-dimensional profile and/or based on the patient's demographic information. The recommendation for the patient interface 3000 may select the type and/or size of the stabilising structure 3300 and/or seal forming structure 3100 that will provide the most comfort and effective seal. The type of the stabilising structure 3300 and/or seal forming structure 3100 may identify the type of materials, texture, and/or colour in the stabilising structure 3300 and/or seal forming structure 3100. The comfort and effective seal may be determined based on a local or remote database of previously matched patient profiles and patient interfaces configurations.

In some examples, the application may generate an image or video with the recommended patient interface or the patient interface selected by the patient superimposed on the captured image. The application may be configured to provide real-time augmented reality display of the recommended or selected patient interface superimposed on the captured images of the patient's face. The application may provide the patient with options to store and/or share (e.g., on social media) the generated images.

The application may display instructions for using the patient interface selected for the patient (Step 7016). The instructions may include instructions setting up the RPT devices 4000 for use with the selected patient interface 3000, how to connect the patient interface 3000 to the RPT devices 4000, how to position the patient interface 3000 on the patient's face, and/or adjust settings of the patient interface 3000. The instructions may be generated by the application and/or a remote processing system based on the captured images (e.g., facial profile of the patient). For example, the instructions may instruct the patient on a setting for one or more straps of the headgear determined based on the facial profile of the patient.

In step 7018, images of the patient with the patient interface 3000 may be captured. The captured images may be used to establish a baseline for the patient using the selected patient interface 3000.

The images may be captured after the patient has positioned the patient interface 3000 on the face and a confirmation is received from the patient that the patient interface 3000 provides a comfortable fit and/or a confirmation is received from the patient or application indicating that the patient interface 3000 is operating properly (e.g., no leaks are detected during operation of the RPT device 4000).

In step 7020, a determination is made whether the patient is having issues with the patient interface. The determination may be made based on an input received from the patient via a user interface. In some examples, the determination may be made automatically based on data from sensors disposed in the RPT device 4000 and/or air circuit 4170 indicating whether there are any leaks in the patient interface 3000. In some examples, the application may display a request for the patient to indicate if they are having issues with the patient interface 3000 based on use of the patient interface (e.g., when the RPT device 4000 is not being used as prescribed for the patient).

If a signal is received indicating that the patient is experiencing issues with the patient interface (YES in Step 7020), then images may be captures of the patient and the patient interface (step 7022). The images may be captured from a plurality of different positions and orientations. In some examples, if a signal is received indicating that the patient is experiencing issues with the patient interface (YES in Step 7020), the patient may be asked to identify a source of the discomfort or issues by providing images, audible instructions and/or text input.

The captured images may be analysed (step 7024) to determine sources of discomfort or ineffective supply of breathable gas (e.g., leaks of breathable gas). The captured images may be compared to a baseline which may include the images captured in step 7018, or images stored in a database.

The analysis may include detecting features of the patient interface 3000 that may be the source of the discomfort or ineffective supply of breathable gas. For example, the images may be analysed to locate straps of the head-gear that may be causing the discomforts. Over-stressed or loose straps may cause discomfort and may be detected by changes in colour of the straps due to over-tightening or not being tightened. In some examples, spacing between markers disposed along the length of the straps that change in relative position of each other as the straps are tightened may be detected and used to identify over-tightening or straps that are not tightened enough. The markers may be visible to the patient or not-visible to the patient (e.g., without a camera). Markers that are not visible to the patient may be detected after performing image analysis to extract the markers from the captured images and/or captured by specialised cameras or camera settings (e.g., using an infrared camera).

In some examples, the camera may detect locations on the patient's face where the straps and/or seal forming structure are causing excessive pressure on the patients face based on difference in patient's skin colour or temperature distribution. The images from the camera may be analysed to determine heat zones on the patient caused by excessive pressure of the patient interface. In other examples, the heat distribution along the patient interface 3000 may be used to determine portions of the patient interface 3000 that are not in contact with the patient. Portions of the patient interface 3000 that are not in contact with the patient may appear cooler on an IR image as compared to portions of the patient interface 3000 that are in contact with the patient's face. In some examples, stretch marks on the patient interface (e.g., straps and/or mask cushion) may be analysed to determine the level of stretch within certain portions of the patient interface e.g. determining that certain portions are over stretched and/or are stretched more than other portions of the patient interface.

In some examples, the analysis may include comparing baseline images to the images captured of the patient with patient interface 3000 when they are experiencing issues. In this example, the images may be compared to determine changes in positioning of the patient interface 3000 relative to one or more features of the patient's face, changes in the relative positioning of one feature of the patient interface (e.g., a first strap) to another feature of the patient interface (e.g., a second strap). Before images are compared, the captured images may be processed (e.g., scaled, background removed) to improve the analysis. Captured images may be scaled such that the patient's features in the captured image correspond in size to the patient's features in the baseline images.

In some examples, the analysis may include analysing the received audible and/or text (with or without analysing the images) to determine the source of the discomfort or ineffective supply of breathable gas, and to provide a solution to the identified issues. In other examples, the analysis may include leak detection data obtained from sensors to provide adjustment feedback for better fit.

The results of the analysis may be displayed by the application (Step 7026). The results of the analysis may include advice to the user on how to correct the identified issue with the patient interface 3000. The results of the analysis may include identification of differences in positioning of the patient interface 3000 between the baseline images and the captured images. The application may display instructions for correcting the position of the patient interface 3000 such that the patient interface 3000 is provided in a position provided in the baseline images. For example, the displayed instructions may include text, images, and/or animations indicating how to adjust a length of a strap on one side of the face so that the strap exerts a pressure on the side of the seal forming structure that is approximately equal to pressure exerted on an opposite side of the seal forming structure by the strap on another side of the face.

The displayed analysis results may include displaying the captured images and one or more indicators showing locations where the patient interface can be adjusted to improve comfort and/or fit. The indicators may include text, symbols, shading, and/or outlines of patient interface edges to indicate the location of the source of issue. The indicator may be user selectable, to provide more information about the identified issues. The indicator may include a plurality of numbers indication locations where the patient interface can be adjusted, the numbers being ordered based on the degree of expected improvement in comfort and/or fit.

Once it has been determined that there are no issues with the patient interface (No in Step 7020), the program may go to a resolved state (Step 7030). The resolved state may be maintained until one or more of the following occurs:

- a user indicates that an issue is being experienced with the patient interface,
- a determination is made that a new patient interface is being used (e.g., based on detecting a new patient interface is coupled to the RPT device 4000),

- data from one or more of the sensors indicate that there is incorrect positioning of the patient interface 3000,
- a predetermined amount of time has passed after entering the resolved state,
- after the RPT device 4000 has been used for a predetermined amount of time after entering the resolved state, or
- after the RPT device 4000 has been used for a predetermined number of times after entering the resolved state.

After the program runs through steps 7022, 7024 and 7026, a determination may again be made in Step 7010 to determine of the patient is experiencing any new/further issues with the patient interface (step 7020). After adjusting the positioning of the patient interface, steps 7022, 7024 and 7026, may be repeated until the user is satisfied with the comfort and effectiveness of the patient interface 3000.

As discussed above, in some examples of the present technology, the determination of whether the patient is having issues with the patient interface may be made automatically based on data received from one or more sensors (e.g., a transducer). The data from the sensors may also be used to perform the analysis (step 7024) and/or make recommendations to the patient at step 7026). The sensors may be disposed in the RPT device 4000 and/or air circuit 4170 indicating whether there are any leaks in the patient interface 3000 and/or incorrect positioning of the patient interface 3000. Determining issues (steps 7020 and/or 7024) with the use of the patient interface 300 and/or displaying results (step 7026) of the analysis based on the sensor data may be performed together with the analysis of the images or as an alternative to the analysis made based on the images.

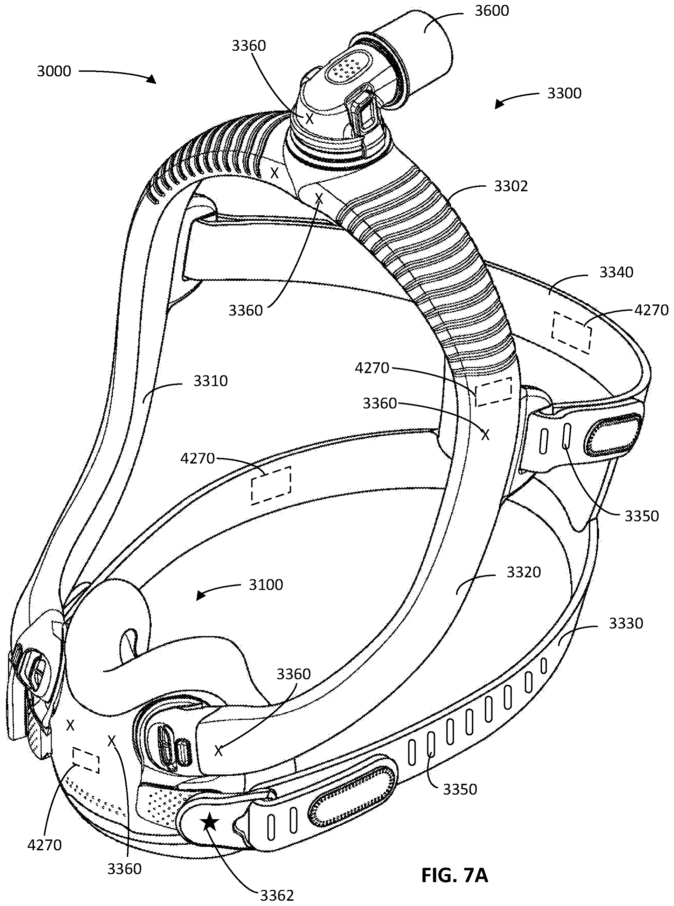

The output from the one or more sensors may be used as input data for determining issues with the mask and providing recommendations for correcting the issues. As shown in Fig. 7A, a sensor 4270 (e.g., a transducer) may be disposed on a surface of or inside a mask (e.g., a mouth cushion and/or a nasal cushion of the mask) and/or the stabilising structure 3300 (e.g., one or more of the straps 3330 and/or 3340 and/or tubes 3310 and 3320). The sensor 4270 may include one or more of a flow rate sensor, a pressure sensor, a temperature sensor, a piezo electric sensor, a motion sensor, a position sensor, and/or a strain gauge. In some examples, the sensor 4270 may detect a proper connection between different components of the patient interface. In one example, the strain gauge may be provided on a surface of or within the headgear and/or the cushion of the patient interface 3000 and be configured to sense tension in portions of the headgear and/or the cushion. The sensor 4270 may provide data to a processor wirelessly and/or over a wired connection.

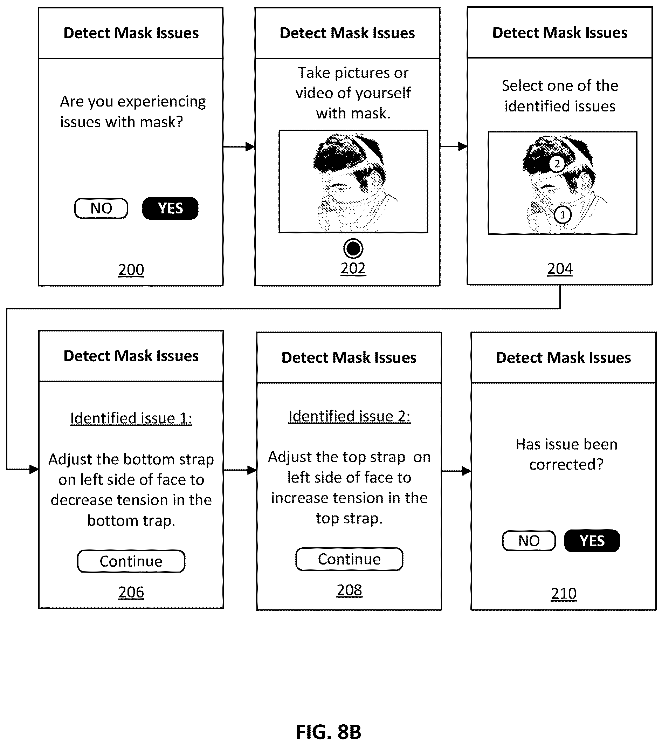

The output from the sensors may be used to detect changes in positioning and/or characteristics of the mask and/or headgear placement in real-time while the patient adjusts the setup in accordance with instructions (e.g., display screens 206 and 208 shown in Fig. 8B). The data from the sensors may be compared to pre-set values, stored in memory, which are determined by the manufacturer or clinician for example, or received from the sensors when the patient establishes a baseline with a mask/headgear providing a proper fit (e.g., step 7018 in Fig. 6C). During execution of steps 7020-7026, the sensor data may be received and compared to the pre-set values. The results of the analysis (step 7026) may be displayed based on the results of the comparison and updated in real-time as the patient adjusts the patient interface.

In some examples, the sensor data may be received periodically to determine if there are any issues detected with the use of the patient interface. For example, the sensor data may be received when the patient connects the patient interface to the RPT device 4000 and/or after a predetermined amount of time after start of a treatment cycle.

In some examples, one or more operations (e.g., operations in Fig. 6C) may be performed continuously over a predetermined period of time. For example, operations 7016 and 7018 in Fig. 6C may be performed continuously in real time over a predetermined period of time to aid the patient with fitting the patient interface. In this example, the application may capture a plurality of images, which may be captured in the form of a video, determine a proper fit (e.g., needed setting) of the selected patient interface for the patient based on the facial structure of the patient, and guide the patient in providing the proper fit until the patient interface is properly placed on the patient.

In the example for providing setting for headgear strap length, the patient's facial structure may be cross referenced with predetermined headgear strap lengths to calculate the length and tightening of the headgear for the patient. The data for this determination may be stored in the application or in a remote location. In some example, patient demographic information, images and/or information about the patient's facial profile may be transmitted to a remote processing system (e.g., the remote external device 4286) to perform the analysis and the application may receive the analysis results from the remote processing system.

The image of the patient, captured in operation 7018, may be analysed to determine if the current settings of the patient interface correspond to the determined length and tightening of the headgear for the patient. In operation 7016, instructions are displayed to instruct the patient in making the adjustments needed to achieve the determined length and tightening of the headgear for the patient. The instructions may include overlay of estimated tightening onto the patient's facial structure and provide visual and audible cues for when the patient has reached the estimated amount of headgear adjustment. The instructions may include a visual and/or audible indicator when the estimated amount of headgear adjustment is achieved.

**5.7.4 Patient interface with indicators and reference points**

Fig. 7A shows a patient interface 3000 including a plurality of measurable indicators 3350 and reference points according to one form of the present technology. The positioning and stabilising structure 3300 of the patient interface 3000 shown in Fig. 7A includes tubes 3310 and 3320 provided as part of the positioning and stabilising structure 3300. The tubes provide a path for breathable air between the connecting port 3600 and the seal-forming structure 3100. The positioning and stabilising structure 3300 includes a first strap 3330 and a second strap 3340. Ends of the first strap 3330 are removably coupled to the seal seal-forming structure 3100, and ends of the second strap 3340 are removably coupled to the tubes 3310 and 3320.

Measurable indicators 3350 and/or reference points 3360 may provide specific locations on the patient interface 3000 that can be compared to a baseline for determining if the patient is wearing the patient interface properly. Using measurable indicators 3350 and/or reference points 3360 may reduce the computation complexity involved with using image processing to detect more complex features of the patient interface 3000. The measurable indicators 3350 and/or reference points 3360 may not be visible to a person but may be detectable by a camera (e.g., an IR camera).

As shown in Fig. 7A, reference points 3360 are provided at different components and locations of the patient interface 3000. The tubes 3310 and 3320 include reference points 3360 provided on the outside and side surfaces. The reference points 3360 may be provided near ends of the tubes and in a mid-section of the tube. The oro-nasal cushion including the seal forming structure 3100, straps 3340 and 3330, and/or connecting port 3600 may also include one or more reference points 3360. One or more reference points 3360 may indicate a centre of a component (e.g., the oro-nasal cushion in the vertical and/or horizontal direction). The reference points are not limited to the locations shown in Fig. 7A and may be provided at other locations (e.g., on the straps and/or the connecting port 3600).

As shown in Fig. 7A, a reference marker 3362 may be provided on a connector (e.g., magnetic clip) used to couple the headgear straps to the oro-nasal cushion or nasal cushion. The reference marker 3362 may include a shape from which the positioning and/or orientation of the patient interface 3000 and/or a portion of the patient interface may be determined. The positioning and /or shape of the reference marker 3362 is not limited to the position and shape shown in Fig. 7A. One or more reference markers having a same and/or different shape may be provided in other locations on the mask and/or the headgear.

As shown in Fig. 7A, the first and second straps include indicators 3350 which may be used to determine the setting of the strap (e.g., how far the strap is stretched). If the spacing between the indicators 3350 on one side of the strap is larger than the spacing on the other side of the strap then one side of the strap may be over-tightened or not tightened enough. Counting the number of visible indicators 3350 may indicate a setting at which the strap should be positioned during use.

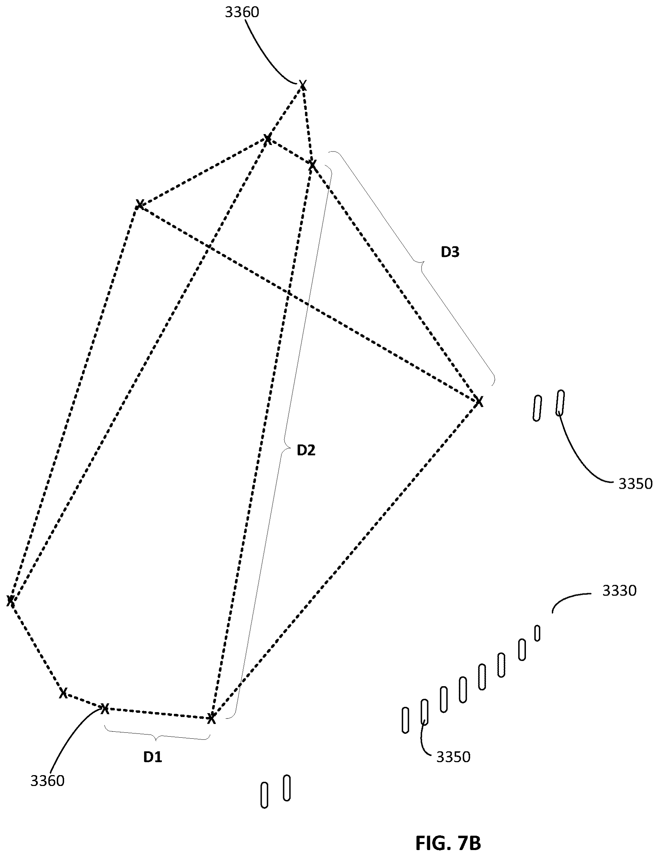

Fig. 7B shows measurable indicators 3350 and/or reference points 3360 extracted from images captured of the patient and the patient interface according to one form of the present technology. The extracted measurable indicators 3350 and/or reference points 3360 may be compared to measurable indicators 3350 and/or reference points 3360 of a baseline to determine differences. As discussed above, counting the number of extracted indicators 3350 and comparing the number to a number in the baseline may indicate if the strap is over-tightened or not tightened enough.

Differences in the distance between specific extracted reference points 3360 and corresponding reference points of the baseline may indicate if a proper connection between components of the patient interface 3000 is present or if patient interface 3000 need to be adjusted. For example if distance D1 between two reference points increases from the distance in the baseline, a determination may be made that a proper connection between the oro-nasal mask and tube 3320 connecting to the oro-nasal mask is not present. As another example, if distance D2 between two reference points increases from the distance in the baseline, a determination may be made that the length of tube 3320 is too long and needs to be reduced. If distance D3 between two reference points is different from the corresponding distance in the baseline, a determination may be made that the length of tube 3320 needs to be adjusted by extending or contracting an expandable portion 3302 of the tube 3320.

According to one form of the present technology, the extracted reference points 3360 may be used to build a three-dimensional profile of the patient interface (e.g., as shown in Fig. 7B) and measurements may be compared to a three-dimensional profile of the patient interface provided in the baseline. In this example, angles between lines connecting the reference points, distances of lines connecting the reference points, presence of parallel lines, and/or presence of perpendicular lines may be compared to the baseline to determine changes in the positioning and configuration of the patient interface 3000.

While not shown in Fig. 7B, patient features (e.g., one or more features shown in Figs. 2B-2F) may also be extracted and used as part of the analysis to determine changes in positioning of the patient interface 3000. For example, the analysis may include determine changes in distance between a person's eye and a reference point on a tube.

In some examples, issues with the positioning of the patient interface 3000 may be determined without using a baseline and/or features of the patient. For example, issues in positioning of the patient interface 3000 can be determined without a baseline by comparing measurements from extracted reference points on one side of the patient's head to corresponding measurements from extracted reference points on an opposite side of the patient's head. In this example, one side of the patient interface may be used a reference for comparison to an opposite side of the patient interface.

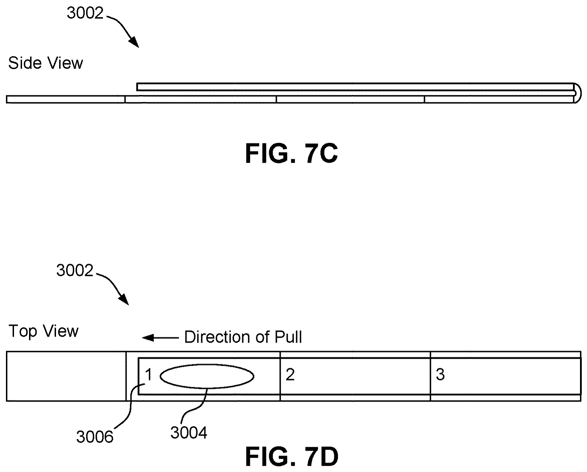

Figs. 7C and 7D show examples of a headgear strap 3002 that is part of a patient interface 3000 that may be adjusted based on examples of the present technology. Fig. 7C shows a side view of the strap 3002 and Fig. 7D shows a top view of the strap 3002. As shown in Figs. 7C and 7D, a portion of the strap 3002 may overlay another portion of the strap 3002 and removably connect to the overlayed portion using a connector 3004 (e.g., a hook tab). The strap 3002 may include a plurality of indicators 3006, which may include numerals, letters and/or symbols. As the strap 3002 is adjusted in the direction of pull, additional subdivisions of the strap 3002 and corresponding indicators may be made visible in the top view shown in Fig. 7D. The system may instruct the user as to how far the strap should be pulled using the indicators 3006 as references. For example, the system may instruct the user to adjust the strap 3002 such that indicator "3" is visible on the strap. In some examples, the system may analyse captured images of the strap 3002 to determine at what setting the strap 3002 is currently set and instruct the patient to further adjust the strap 3002 if the setting does not correspond to the needed setting of the strap 3002 determined for the patient.

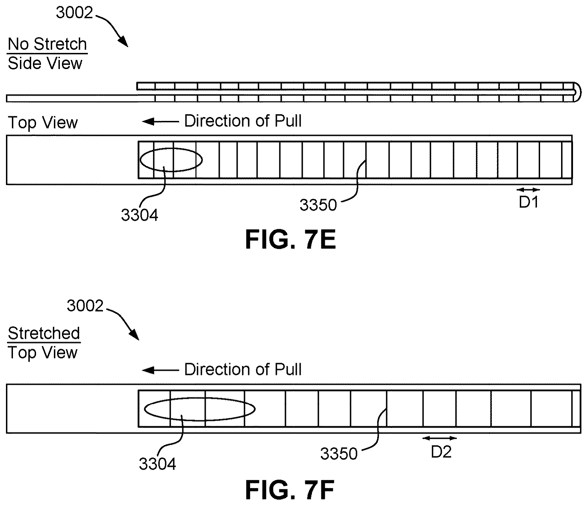

As discussed above, some examples of the present technology may provide indicators (see indicators 3350 in Fig. 7A) for determining the setting of the strap (e.g., how far the strap is stretched). Fig. 7E and 7F show examples of using spacing between indicators 3350 on a strap 3002 to determine a stretch force, according to examples of the present technology. Fig. 7E shows top and side views of the strap 3002 with no stretch. Fig. 7F shows a top view of the strap 3002 stretched. In some examples, the indicators 3350 provided in different portions of the patient interface 3000 may be provided at a same predetermined spacing in the non-stretched state.

As shown in Fig. 7E and 7F, the distance D1 between consecutive indicators 3350 in the strap 3002 without being stretched is smaller than the distance D2 between consecutive indicators 3350 in the strap 3002 that is stretched. The system may analyse captured images of the indicators 3350 and based on the distances between consecutive indicators 3350 compare forces applied by different straps of the patient interface 3000, determine if a force applied by a strap is within predetermined upper and/or lower limits, determine if the force applied a strap corresponds to a determined setting for the patient. In some examples, the system may monitor the change in the distance between consecutive indicators 3350 based on the captured images and provide audible and/or visual cues when the distance corresponds to the needed setting.

Because the distance between consecutive indicators 3350 may vary over the distance of the strap, the system may be configured to determine how much a strap is stretched based on a plurality of distances between consecutive indicators 3350. The system may determine an average of the plurality of distances for determining how much a strap is stretched.

### 5.7.4.1 Example display screens for guiding patient interface fitting

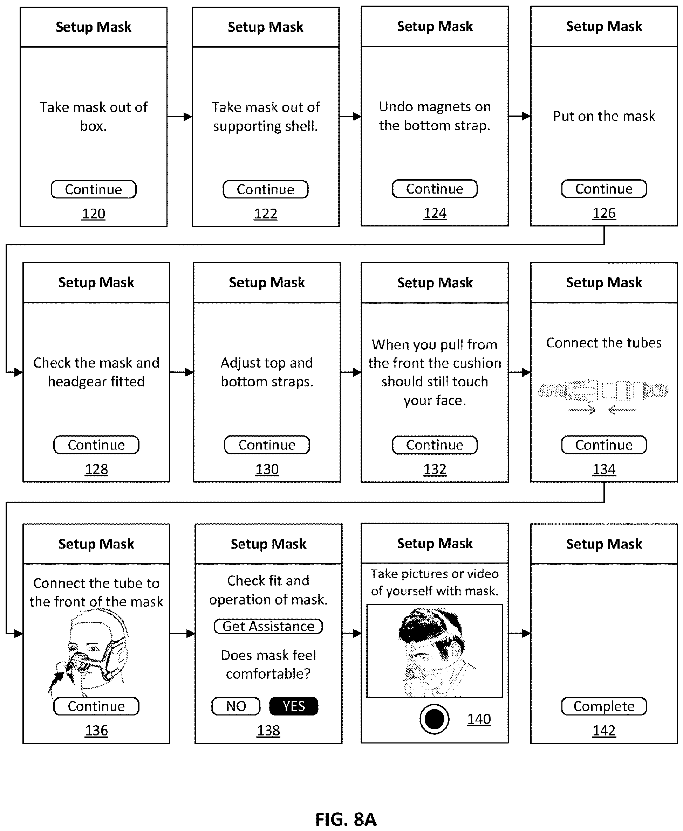

Fig. 8A includes a series of display screens that assist a user in setting up a mask (a patient interface) and capturing a baseline of the setup mask. The display screens may be displayed on a display of a local external device 4288 and/or the RPT device 4000. While some of the displays screens shown in Fig. 8A include instructions and show a mask with specific features, examples of the present technology are not so limited. One or more instructions and/or display screens may not be shown and/or other instructions and/or display screens may be added depending on the type of mask a patient is using. For example, not all patient interfaces may include magnets, full face masks, and/or lower straps shown in Fig. 8A.

In certain examples, the nature of the specific display screens may be generated based on the type of mask or patient interface that the patient is using. The type of patient interface may be determined from user specifying the mask, recognition of a bar code that is carried by the mask, use of image recognition, RFID, or other techniques for identifying the type of patient interface that is being setup. In certain examples, what information is related or what display screens are generated and shown may be based on the recognition. In certain examples, the order of the display screens and what instructional information is presented to the user may be based on the recognized information.

Display screen 120 indicates that the patient should take the mask out of the box in which it is packaged. In certain examples, an image, video, or animation may be displayed to the patient to demonstrate how to remove the mask from the box. In certain examples, the patient interface may come in its own packaging. Accordingly, for example, display screen 120 indicates that the patient should take the mask out of a supporting shell (e.g., packaging material that may be included around the mask). In certain examples, an image, video, or animation may be displayed to the patient to demonstrate how to remove the supporting shell or other packaging material.

Display screens 122 and 124 instruct the patient on how to put on the mask. Screen 124 may be utilized, for example, with a mask that includes magnetic headgear connects. Alternative screens could be used in connection with masks that use different headgear, for example, headgear with snap connects, Velcro, elastic bands, etc... In the included example, screen 124 instructs the patient to undo the magnets on the bottom straps of the mask to release the headgear and allow the mask to be properly positioned. In certain examples, an image, video, or animation may be displayed to the patient to demonstrate how to undo the magnets.

Display screen 126 instructs the patient to put on the mask. In certain examples, an image, video, or animation may be displayed to the patient to demonstrate how to put on the mask.

Display screen 128 instructs the patient to check that the mask and headgear are properly fitted. In certain examples, an image, video, or animation may be displayed to the patient to demonstrate how to properly fit the mask and/or headgear to the face of the patient.

Display screen 130 instructs the patient to adjust the top and bottom straps. In certain examples, an image, video, or animation may be displayed to the patient to demonstrate adjustment of the top and/or bottom straps of the mask. In certain examples, the top and bottoms straps should snugly fit to the patient face without being too tight. This display screen, and display screen 130, may facilitate correct adjustment of the straps by the patient to prevent over or under tightening of the straps.

Display screen 132 instructs the patient with additional information on adjusting the top and/or bottom straps of the mask. In particular, display screen 132 may indicate that a cushion portion of the mask should still be in contact with the face of the patient when the mask is pulled from the front.

Another step in the setup process for the patient interface includes display screen 134 that instructs the patient to connect one or more tubes or conduits to each other and a display screen 136 that shows a patient wearing a mask with a connection to an air conduit.

In certain examples, specific instructional notes may be included depending on the nature of the component (e.g., patient interface, air conduit, etc...) being set up. For example, if a connection between a patient interface and air conduit is provided and the type of connector that is being used between the two components includes dual clips on either side of a connector, then the displayed instructions on a display screen may include a note reminding the patient to ensure both sides of the connector are securely clipped. Such pinpoint instructions may prevent problems (e.g., leaks occurring in the connection).

Display screen 132 instructs the patient to check fit and operation of the mask. The instructions may include asking the patient to lay down and get up a certain number of times to ensure that the patient interface fits well with expected movement of the patient. Operation of the mask may be checked by controlling operation of the RPT device 4000 through a test cycle, in which pressurised breathable gas is provided to the patient via the patient interface 3000. In some examples, the components of the system may be structures to automatically determine proper operation. For example, the patient interface and/or air conduit may be structured to electronically determine that one has been correctly connected with the other. Data of such a determination may be transmitted to the computing device.

The patient may be provided with an option to indicate that the mask does not operate properly and/or feels uncomfortable. If the patient indicates that mask does not operate properly and/or feels uncomfortable, then one or more steps of the setup may be repeated. As shown in display screen 132, the patient may be provided with an option to get assistance in setting up the mask. The assistance may be provided via a video conference with a technician which can guide the patient with the setup based on video/images provided to the technician. In some examples, the assistance may be provided by accessing additional instructions (e.g., images, videos, or animations) to provide more detailed mask fitting instruction from a remote source (e.g., remote external device 4286). The assistance may be provided by an artificial intelligence guiding the setup of the mask.

The patient may be provided with an option to indicate that the mask operates properly and feels comfortable. If the patient indicates that mask operates properly and/or feels comfortable, then display screen 140 may be displayed to capture images and/or video of the patient and the mask. The application may guide the patient via the display screen 140 and/or audible instructions to capture images and/or video from different positions and/or orientations. The captured images and/or video may be stored in local memory of the device and/or transferred to a remote computing device (e.g., remote external device 4286).

Display screen 142 is displayed showing completion of the setup process for the patient interface. In certain examples, finishing a setup of a component and/or a given display screen may be logged to a data file or the like and associated with the baseline images. In certain examples, the data of a patient's progress in the setup of the equipment may be transmitted to a remote computing device. This may allow remote users (e.g., medical or customer support personnel) to verify that the setup for a given component has been completed and/or confirm, based on the captured images, that the patient interface has been setup properly. The medical or customer support personnel may provide a confirmation that the captured images can be used as a baseline when the patient experiences issues with the patient interface.

### 5.7.4.2 Example display screens for detecting issues with patient interface

Fig. 8B includes a series of display screens that assist a user in correcting positioning and setup of a mask (a patient interface). The display screens may be displayed on a display of a local external device 4288 and/or the RPT device 4000. While some of the displays screens shown in Fig. 8B include instructions and show a mask with specific features, examples of the present technology are not so limited. One or more instructions and/or display screens may not be shown and/or other instructions and/or display screens may be added depending on the type of mask a patient is using and issues identified with the specific patient interface.

Display screen 200 provides a patient with an option to indicate that they are experience issues with the mask. Display screen 120 may be displayed automatically based on the computing system determining that the user interface is not being used based on prescribed schedule and/or that RPT device is operating improperly and/or inconsistently.

Display screen 202 indicates that the patient should capture images or video of the patient with the mask. The application may guide the patient via the display screen 202 and/or audible instructions so that the images and/or video are captured from different positions and/or orientations. The captured images and/or video may provide a scan of the patient and the patient interface. The captured images and/or video may be stored in local memory of the device and/or transferred to a remote computing device (e.g., remote external device 4286).

The captured images may be analysed to determine potential issues with how the mask is set up and/or positioned on the patient's head. Display screen 204 indicates to the patient potential issues with the patient interface. The issues may be identified via text, images, and/or animations. In display screen 204 the issues are identified with number overlaid over one of the captured images, each number indicating a potential issue in an order of importance identified by the system.

Display screen 206 and display screen 208 identify and provide instructions for correcting the issues. Display screen 206 and display screen 208 may include images, videos, and/or animations to show correct setting and/or positioning of the mask. Display screen 206 may be displayed in response to the user selecting a first issue shown in display screen 204. Display screen 208 may be displayed in response to the user selecting a second issue shown in display screen 204. After displaying information for one or more of the identified issues, display screen 210 may be displayed to request the user to indicate if the issue has been corrected. If the issue has been corrected, then detecting mask issues may be completed. If the issue has not been corrected, then display screen 202 may be displayed to repeat the capture of images to detect additional issues with the patient interface. In some examples, the display screens 204 and/or 206 may display information for correcting the issue based on data received from one or more sensors disposed in the mask and/or headgear and update the displayed information in based on received sensor in real-time.

In some examples, after issues with the patient interface have been corrected (YES in display screen 210), display screen 140 in Fig. 8A may be displayed to capture additional images and/or video to be replace previously used baseline images or to add to the existing baseline images. In some examples, the determination that the issue has or has not been corrected may be made automatically based on data received from one or more sensors disposed in the mask and/or headgear.

### 5.7.4.3 Example display screens for guiding patient interface fitting using captured image of patient

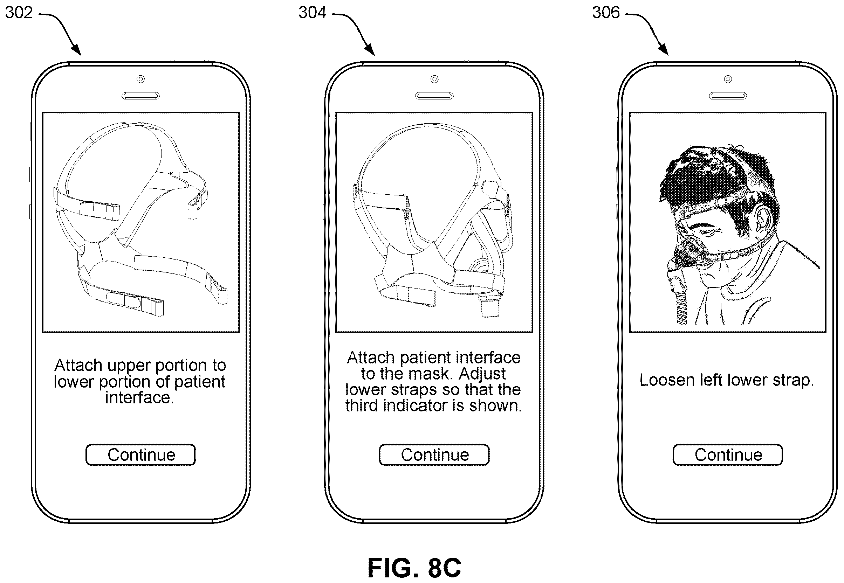

Fig. 8C illustrates series of display screens that can be used to assist a user in positioning a patient interface. One or more of the screen shown in Fig. 8C may be used in the series of screen shown in Figs. 8A and/or 8B. Display screen 302 illustrates a headgear selected for the user or by the user. The illustrated headgear may correspond to a type, size and configuration of headgear selected for the patient by the system. The display screen 302 may include instruction for attaching different portions of the headgear to each other.

Display screen 304 displays an image of a headgear attached to a mask and instructions for attaching the headgear to the mask. The illustrated mask may correspond to a type, size and configuration of mask selected for the patient by the system. The instructions may include instructions for headgear settings that should be made by the user before putting on the patient interface. The instructions may be generated based on images captured of the patient, use history and/or data stored in a database.

Display screen 306 displays an image of a patient interface provided on a mock patient. In some examples, the mock patient may be generated from previously captured images of the patient or correspond to an avatar generated based on images of the user. The patient interface selected for the user may be overlayed on the face of the patient using augmented reality. The patient interface may be overlayed on the face of the patient based on features of the patient extracted from images of the patient. In some examples, the image shown on the display screen 306 is a real-time image of the patient taken from a feed of a front-facing camera of a mobile device, for example. The location of certain portions of the patient interface may be determined by detecting the location of one or more reference markers 3362. For example, a reference marker 3362 may be used to locate the headgear strap. This may be used to determine the optimum fit for the patient. In some examples, the display screen 306 may include instructions for adjusting the headgear straps (e.g., lessening or tightening the straps) and/or for adjusting the positioning of the straps and/or the mask. The instructions may be generated based on images captured of the patient, use history and/or data stored in a database. The instructions may be provided by overlaying symbols such as arrows on the image to provide adjustment feedback to the user. In other examples, the instructions may be in the form of an audio cue.

One or more of the display screens shown in Figs. 8A-8C may include a three-dimensional model of the patient and/or the patient interface allowing a user to change, based on a user input, how the patient and/or the patient interface are displayed. For example, the user inputs may change orientation and/or positioning of a virtual camera used to capture the image of the patient and/or the patient interface for display to the patient.

### 5.7.4.4 Example computing device

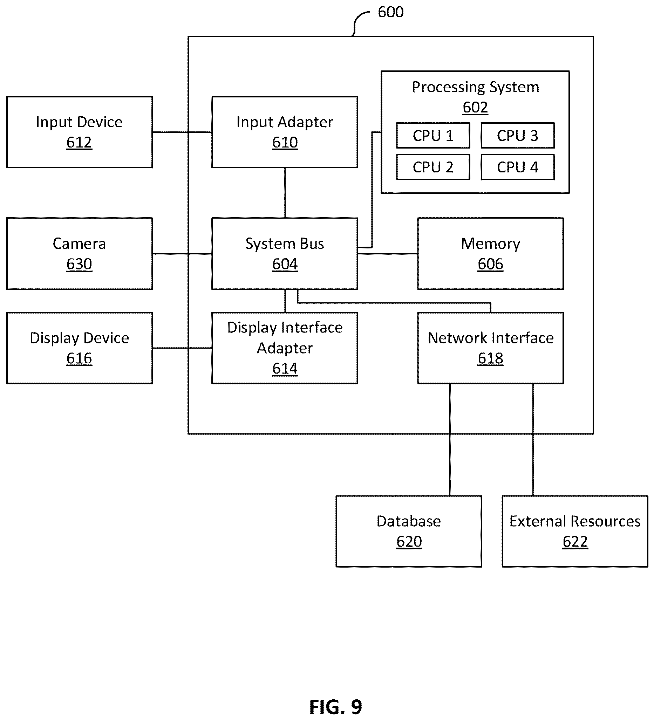

Fig. 9 shows a block diagram of an example computing device 600 (which may also be referred to, for example, as a "computing device," "computer system," or "computing system") herein. In certain examples, the computing device 600 may be provided in a local external device 4288. In certain examples, the computing device 600 may correspond to the central controller 4230.

In certain examples, the computing device 600 includes one or more of the following: a processing system 602, which includes one or more hardware processors (e.g., central processing units or CPUs); one or more memory devices 606; one or more network interface devices 618; one or more display interfaces 614; and one or more user input adapters 610. Elements of computing device 600 may communicate with one another via system bus 604. Additionally, in some examples, the computing device 600 is connected to or includes a display device 616, user input device 612, camera 630, database 620, and/or external resources 622 (which may be another instance of computing device 600). As will be explained below, these elements (e.g., the processing system 602, memory devices 606, network interface devices 618, display interfaces 614, user input adapters 610, camera 630, display device 616) are hardware devices (for example, electronic circuits or combinations of circuits) that are configured to perform various different functions for the computing device 600.

In some examples, each or any of the processors (e.g., CPUs 1, 2, 3, or 4) of the processing system 602 is or includes, for example, a single- or multi-core processor, a microprocessor (e.g., which may be referred to as a central processing unit or CPU), a digital signal processor (DSP), a microprocessor in association with a DSP core, an Application Specific Integrated Circuit (ASIC), a Field Programmable Gate Array (FPGA) circuit, and/or a system-on-a-chip (SOC) (e.g., an integrated circuit that includes a CPU and other hardware components such as memory, networking interfaces, and the like). In certain examples, each or any of the processors may use an instruction set architecture such as x86 or Advanced RISC Machine (ARM).

In some examples, each or any of the memory devices 606 is or includes a random access memory (RAM) (such as a Dynamic RAM (DRAM) or Static RAM (SRAM)), a flash memory (based on, e.g., NAND or NOR technology), a hard disk, a magneto-optical medium, an optical medium, cache memory, a register (e.g., that holds instructions), or other type of device that performs the volatile or non-volatile storage of data and/or instructions (e.g., software that is executed on or by processors of the processing system 602). Memory devices 606 are examples of non-transitory computer-readable storage media.

In some examples, each or any of the network interface devices 618 includes one or more circuits (such as a baseband processor and/or a wired or wireless transceiver), and implements layer one, layer two, and/or higher layers for one or more wired communications technologies (such as Ethernet (IEEE 802.3)) and/or wireless communications technologies (such as Bluetooth, WiFi (IEEE 802.11), GSM, CDMA2000, UMTS, LTE, LTE-Advanced (LTE-A), and/or other short-range, mid-range, and/or long-range wireless communications technologies). Transceivers may comprise circuitry for a transmitter and a receiver. The transmitter and receiver may share a common housing and may share some or all of the circuitry in the housing to perform transmission and reception. In some examples, the transmitter and receiver of a transceiver may not share any common circuitry and/or may be in the same or separate housings.

In some examples, each or any of the display interfaces 614 is or includes one or more circuits that receive data from the processors of the processing system 602, generate (e.g., via a discrete GPU, an integrated GPU, a CPU executing graphical processing, or the like) corresponding image data based on the received data, and/or output (e.g., a High-Definition Multimedia Interface (HDMI), a DisplayPort Interface, a Video Graphics Array (VGA) interface, a Digital Video Interface (DVI), or the like), the generated image data to the display device 616, which displays the image data. Alternatively, or additionally, in some examples, each or any of the display interfaces 614 is or includes, for example, a video card, video adapter, or graphics processing unit (GPU).

In some examples, each or any of the user input adapters 610 is or includes one or more circuits that receive and process user input data from one or more user input devices 612 that are included in, attached to, or otherwise in communication with the computing device 600, and that output data based on the received input data to the processors 602. Alternatively, or additionally, in some examples each or any of the user input adapters 610 is or includes, for example, a PS/2 interface, a USB interface, a touchscreen controller, or the like; and/or the user input adapters 610 facilitates input from user input devices 612, which may include, for example, a keyboard, mouse, trackpad, touchscreen, voice input, etc... In certain examples, user input adapter 610 may be configured to process data from other types of input sources that are not from a user. For example, user input adapter 610 (e.g., an input adapter) may process data from one or more sensors (e.g., flow, pressure, temperature, or other types of sensors).

In some examples, the display device 616 may be a Liquid Crystal Display (LCD) display, Light Emitting Diode (LED) display, or other type of display device. In examples where the display device 616 is a component of the computing device 600 (e.g., the computing device and the display device are included in a unified housing of, for example, a mobile or tablet device), the display device 616 may be a touchscreen display (e.g., using capacitive or resistive technology to sense a touch) or non-touchscreen display. In examples where the display device 616 is connected to the computing device 600 (e.g., is external to the computing device 600 and communicates with the computing device 600 via a wire and/or via wireless communication technology), the display device 616 is, for example, an external monitor, projector, television, display screen, etc...

In various examples, the computing device 600 includes one, or two, or three, four, or more of each or any of the above-mentioned elements (e.g., the processing system 602, CPUs 1, 2, 3, or 4, memory devices 606, network interface devices 618, display interfaces 6514, and user input adapters 610). In some examples, the computing device 600 includes one or more of: a processing system 602 that includes hardware processors (e.g., CPUs 1, 2, 3, and/or 4); a memory or storage system that includes the memory devices; and a network interface system that includes the network interface devices 618.

The computing device 600 may be arranged, in various examples, in many different ways. As just one example, the computing device 600 may be arranged such that the processors include: a multi (or single)-core processor; a first network interface device (which implements, for example, WiFi, Bluetooth, NFC, etc...); a second network interface device that implements one or more cellular communication technologies (e.g., 3G, 4G LTE, CDMA, etc...); memory or storage devices (e.g., RAM, flash memory, or a hard disk). The processor, the first network interface device, the second network interface device, and the memory devices may be integrated as part of the same SOC (e.g., one integrated circuit chip). As another example, the computing device 600 may be arranged such that: the processors include two, three, four, five, or more multi-core processors; the network interface devices 618 include a first network interface device that implements Ethernet and a second network interface device that implements WiFi and/or Bluetooth; and the memory devices 606 may include RAM and storage in the form of flash memory or hard disk.

Whenever it is described in this document that a software module or software process performs any action, the action is in actuality performed by underlying hardware elements according to the instructions that comprise the software module.

The hardware configurations shown in Fig. 9 and described above are provided as examples, and the subject matter described herein may be utilized in conjunction with a variety of different hardware architectures and elements. For example: in the Figures in this document, individual functional/action blocks are shown; in various examples, the functions of those blocks may be implemented using (a) individual hardware circuits, (b) using an application specific integrated circuit (ASIC) specifically configured to perform the described functions/actions, (c) using one or more digital signal processors (DSPs) specifically configured to perform the described functions/actions, (d) using the hardware configuration described above with reference to Figure 6, (e) via other hardware arrangements, architectures, and configurations, and/or via combinations of the technology described in (a) through (e).

In certain examples, the techniques herein provide for improved use of a patient interface. Such improvements may be based on comparison between a baseline and captured images with a patient and a patient interface. In certain examples, the techniques herein allow for increased patient comfort in using medical devices in their home without the need of a medical expert to set up and instruct on patient interface usage and/or correcting issues related the patient interface usage.

5.8 RESPIRATORY THERAPY MODES

Various respiratory therapy modes may be implemented by the disclosed respiratory therapy system.

**5.8.1 CPAP therapy**

In some implementations of respiratory pressure therapy, the central controller 4230 sets the treatment pressure $Pt$ according to the treatment pressure equation (1) as part of the therapy parameter determination algorithm 4329. In one such implementation, the amplitude $A$ is identically zero, so the treatment pressure $Pt$ (which represents a target value to be achieved by the interface pressure $Pm$ at the current instant of time) is identically equal to the base pressure $P_0$ throughout the respiratory cycle. Such implementations are generally grouped under the heading of CPAP therapy. In such implementations, there is no need for the therapy engine module 4320 to determine phase $\Phi$ or the waveform template $\Pi(\Phi)$.

In CPAP therapy, the base pressure $P_0$ may be a constant value that is hard-coded or manually entered to the RPT device 4000. Alternatively, the central controller 4230 may repeatedly compute the base pressure $P_0$ as a function of indices or measures of sleep disordered breathing returned by the respective algorithms in the therapy engine module 4320, such as one or more of flow limitation, apnea, hypopnea, patency, and snore. This alternative is sometimes referred to as APAP therapy.

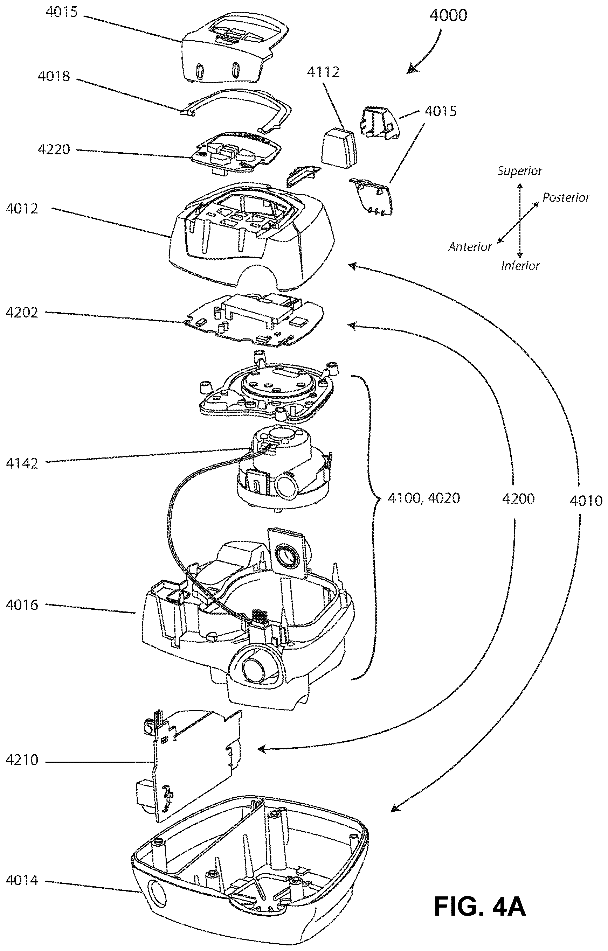

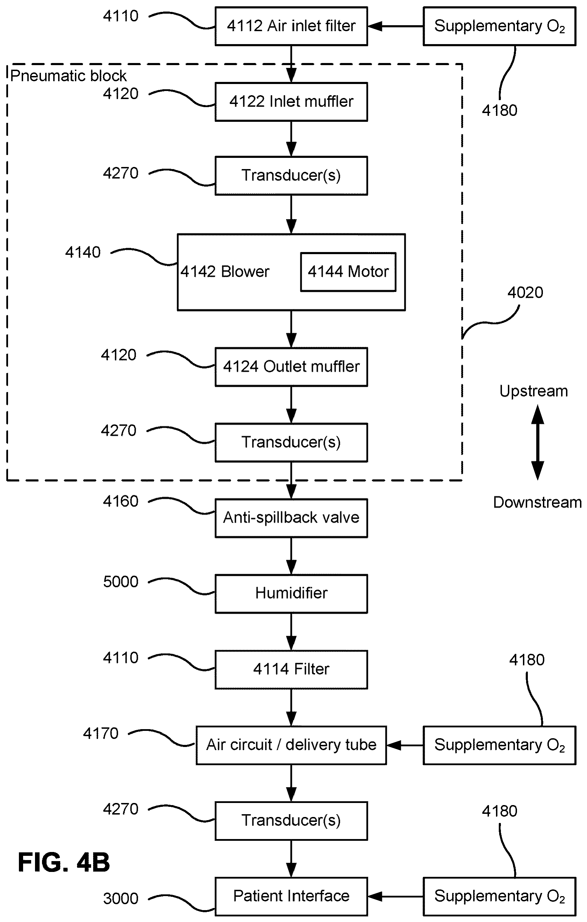

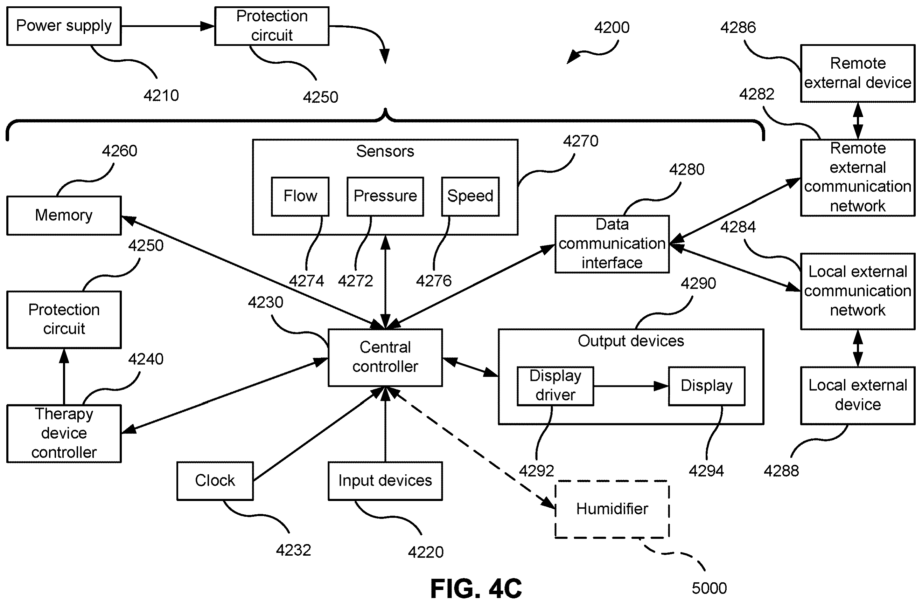

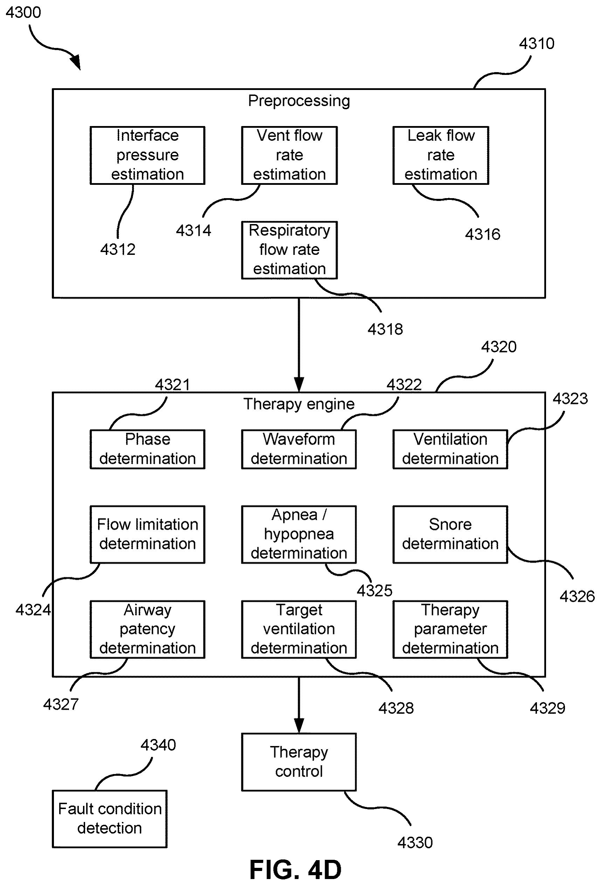

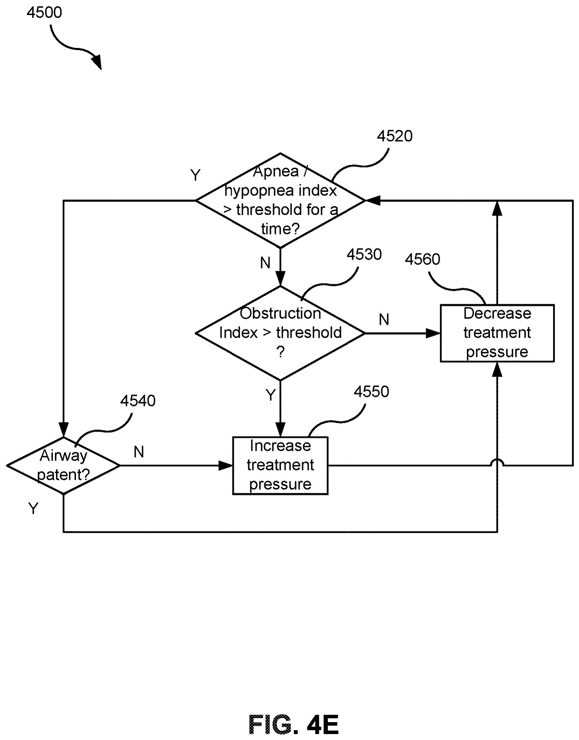

Fig. 4E is a flow chart illustrating a method 4500 carried out by the central controller 4230 to continuously compute the base pressure $P_0$ as part of an APAP therapy implementation of the therapy parameter determination algorithm 4329, when the pressure support A is identically zero.

The method 4500 starts at step 4520, at which the central controller 4230 compares the measure of the presence of apnea / hypopnea with a first threshold, and determines whether the measure of the presence of apnea / hypopnea has exceeded the first threshold for a predetermined period of time, indicating an apnea / hypopnea is occurring. If so, the method 4500 proceeds to step 4540; otherwise, the method 4500 proceeds to step 4530. At step 4540, the central controller 4230 compares the measure of airway patency with a second threshold. If the measure of airway patency exceeds the second threshold, indicating the airway is patent, the detected apnea / hypopnea is deemed central, and the method 4500 proceeds to step 4560; otherwise, the apnea / hypopnea is deemed obstructive, and the method 4500 proceeds to step 4550.

At step 4530, the central controller 4230 compares the measure of flow limitation with a third threshold. If the measure of flow limitation exceeds the third threshold, indicating inspiratory flow is limited, the method 4500 proceeds to step 4550; otherwise, the method 4500 proceeds to step 4560.

At step 4550, the central controller 4230 increases the base pressure $P_0$ by a predetermined pressure increment $\Delta P$, provided the resulting treatment pressure $Pt$ would not exceed a maximum treatment pressure $Pmax$. In one implementation, the predetermined pressure increment $\Delta P$ and maximum treatment pressure $Pmax$ are 1 $cmH_2O$ and 25 $cmH_2O$ respectively. In other implementations, the pressure increment $\Delta P$ can be as low as 0.1 $cmH_2O$ and as high as 3 $cmH_2O$, or as low as 0.5 $cmH_2O$ and as high as 2 $cmH_2O$. In other implementations, the maximum treatment pressure $Pmax$ can be as low as 15 $cmH_2O$ and as high as 35 $cmH_2O$, or as low as 20 $cmH_2O$ and as high as 30 $cmH_2O$. The method 4500 then returns to step 4520.

At step 4560, the central controller 4230 decreases the base pressure $P_0$ by a decrement, provided the decreased base pressure $P_0$ would not fall below a minimum treatment pressure $Pmin$. The method 4500 then returns to step 4520. In one implementation, the decrement is proportional to the value of $P_0$-$Pmin$, so that the decrease in $P_0$ to the minimum treatment pressure $Pmin$ in the absence of any detected events is exponential. In one implementation, the constant of proportionality is set such that the time constant $\tau$ of the exponential decrease of $P_0$ is 60 minutes, and the minimum treatment pressure $Pmin$ is 4 $cmH_2O$. In other implementations, the time constant $\tau$ could be as low as 1 minute and as high as 300 minutes, or as low as 5 minutes and as high as 180 minutes. In other implementations, the minimum treatment pressure $Pmin$ can be as low as 0 $cmH_2O$ and as high as 8 $cmH_2O$, or as low as 2 $cmH_2O$ and as high as 6 $cmH_2O$. Alternatively, the decrement in $P_0$ could be predetermined, so the decrease in $P_0$ to the minimum treatment pressure $Pmin$ in the absence of any detected events is linear.

### 5.8.2 Bi-level therapy

In other implementations of this form of the present technology, the value of amplitude A in equation (1) may be positive. Such implementations are known as bi-level therapy, because in determining the treatment pressure $Pt$ using equation (1) with positive amplitude A, the therapy parameter determination algorithm 4329 oscillates the treatment pressure $Pt$ between two values or levels in synchrony with the spontaneous respiratory effort of the patient 1000. That is, based on the typical waveform templates $\Pi(\Phi, t)$ described above, the therapy parameter determination algorithm 4329 increases the treatment pressure $Pt$ to $P_0 + A$ (known as the IPAP) at the start of, or during, or inspiration and decreases the treatment pressure $Pt$ to the base pressure $P_0$ (known as the EPAP) at the start of, or during, expiration.

In some forms of bi-level therapy, the IPAP is a treatment pressure that has the same purpose as the treatment pressure in CPAP therapy modes, and the EPAP is the IPAP minus the amplitude A, which has a "small" value (a few $cmH_2O$) sometimes referred to as the Expiratory Pressure Relief (EPR). Such forms are sometimes referred to as CPAP therapy with EPR, which is generally thought to be more comfortable than straight CPAP therapy. In CPAP therapy with EPR, either or both of the IPAP and the EPAP may be constant values that are hard-coded or manually entered to the RPT device 4000. Alternatively, the therapy parameter determination algorithm 4329 may repeatedly compute the IPAP and / or the EPAP during CPAP with EPR. In this alternative, the therapy parameter determination algorithm 4329 repeatedly computes the EPAP and / or the IPAP as a function of indices or measures of sleep disordered breathing returned by the respective algorithms in the therapy engine module 4320 in analogous fashion to the computation of the base pressure $P_0$ in APAP therapy described above.

In other forms of bi-level therapy, the amplitude A is large enough that the RPT device 4000 does some or all of the work of breathing of the patient 1000. In such forms, known as pressure support ventilation therapy, the amplitude A is referred to as the pressure support, or swing. In pressure support ventilation therapy, the IPAP is the base pressure $P_0$ plus the pressure support A, and the EPAP is the base pressure $P_0$.

In some forms of pressure support ventilation therapy, known as fixed pressure support ventilation therapy, the pressure support A is fixed at a predetermined value, e.g. 10 $cmH_2O$. The predetermined pressure support value is a setting of the RPT device 4000, and may be set for example by hard-coding during configuration of the RPT device 4000 or by manual entry through the input device 4220.

In other forms of pressure support ventilation therapy, broadly known as servo-ventilation, the therapy parameter determination algorithm 4329 takes as input some currently measured or estimated parameter of the respiratory cycle (e.g. the current measure *Vent* of ventilation) and a target value of that respiratory parameter (e.g. a target value *Vtgt* of ventilation) and repeatedly adjusts the parameters of equation (1) to bring the current measure of the respiratory parameter towards the target value. In a form of servo-ventilation known as adaptive servo-ventilation (ASV), which has been used to treat CSR, the respiratory parameter is ventilation, and the target ventilation value *Vtgt* is computed by the target ventilation determination algorithm 4328 from the typical recent ventilation *Vtyp,* as described above.

In some forms of servo-ventilation, the therapy parameter determination algorithm 4329 applies a control methodology to repeatedly compute the pressure support A so as to bring the current measure of the respiratory parameter towards the target value. One such control methodology is Proportional-Integral (PI) control. In one implementation of PI control, suitable for ASV modes in which a target ventilation *Vtgt* is set to slightly less than the typical recent ventilation *Vtyp,* the pressure support A is repeatedly computed as:

$$A = G \int \left( Vent - Vtgt \right) dt$$

$$(2)$$

where G is the gain of the PI control. Larger values of gain G can result in positive feedback in the therapy engine module 4320. Smaller values of gain G may permit some residual untreated CSR or central sleep apnea. In some implementations, the gain G is fixed at a predetermined value, such as -0.4 $cmH_2O/(L/min)/sec$. Alternatively, the gain G may be varied between therapy sessions, starting small and increasing from session to session until a value that substantially eliminates CSR is reached. Conventional means for retrospectively analysing the parameters of a therapy session to assess the severity of CSR during the therapy session may be employed in such implementations In yet other implementations, the gain G may vary depending on the difference between the current measure *Vent* of ventilation and the target ventilation *Vtgt.*

Other servo-ventilation control methodologies that may be applied by the therapy parameter determination algorithm 4329 include proportional (P), proportional-differential (PD), and proportional-integral-differential (PID).

The value of the pressure support A computed via equation **(Fehler! Verweisquelle konnte nicht gefunden werden.)** may be clipped to a range defined as [*Amin, Amax*]. In this implementation, the pressure support *A* sits by default at the minimum pressure support *Amin* until the measure of current ventilation *Vent* falls below the target ventilation *Vtgt,* at which point *A* starts increasing, only falling back to *Amin* when *Vent* exceeds *Vtgt* once again.

The pressure support limits *Amin* and *Amax* are settings of the RPT device 4000, set for example by hard-coding during configuration of the RPT device 4000 or by manual entry through the input device 4220.

In pressure support ventilation therapy modes, the EPAP is the base pressure $P_0$. As with the base pressure $P_0$ in CPAP therapy, the EPAP may be a constant value that is prescribed or determined during titration. Such a constant EPAP may be set for example by hard-coding during configuration of the RPT device 4000 or by manual entry through the input device 4220. This alternative is sometimes referred to as fixed-EPAP pressure support ventilation therapy. Titration of the EPAP for a given patient may be performed by a clinician during a titration session with the aid of PSG, with the aim of preventing obstructive apneas, thereby maintaining an open airway for the pressure support ventilation therapy, in similar fashion to titration of the base pressure $P_0$ in constant CPAP therapy.

Alternatively, the therapy parameter determination algorithm 4329 may repeatedly compute the base pressure $P_0$ during pressure support ventilation therapy. In such implementations, the therapy parameter determination algorithm 4329 repeatedly computes the EPAP as a function of indices or measures of sleep disordered breathing returned by the respective algorithms in the therapy engine module 4320, such as one or more of flow limitation, apnea, hypopnea, patency, and snore. Because the continuous computation of the EPAP resembles the manual adjustment of the EPAP by a clinician during titration of the EPAP, this process is also sometimes referred to as auto-titration of the EPAP, and the therapy mode is known as auto-titrating EPAP pressure support ventilation therapy, or auto-EPAP pressure support ventilation therapy.

### 5.8.3 High flow therapy

In other forms of respiratory therapy, the pressure of the flow of air is not controlled as it is for respiratory pressure therapy. Rather, the central controller 4230 controls the pressure generator 4140 to deliver a flow of air whose device flow rate *Qd* is controlled to a treatment or target flow rate *Qtgt* that is typically positive throughout the patient's breathing cycle. Such forms are generally grouped under the heading of flow therapy. In flow therapy, the treatment flow rate *Qtgt* may be a constant value that is hard-coded or manually entered to the RPT device 4000. If the treatment flow rate *Qtgt* is sufficient to exceed the patient's peak inspiratory flow rate, the therapy is generally referred to as high flow therapy (HFT). Alternatively, the treatment flow rate may be a profile $Qtgt(t)$ that varies over the respiratory cycle.

5.9 GLOSSARY

For the purposes of the present technology disclosure, in certain forms of the present technology, one or more of the following definitions may apply. In other forms of the present technology, alternative definitions may apply.

**5.9.1 General**

*Air:* In certain forms of the present technology, air may be taken to mean atmospheric air, and in other forms of the present technology air may be taken to mean some other combination of breathable gases, e.g. atmospheric air enriched with oxygen.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, $Qd$, is the flow rate of air leaving the RPT device. Total flow rate, $Qt$, is the flow rate of air and any supplementary gas reaching the patient interface via the air circuit. Vent flow rate, $Qv$, is the flow rate of air leaving a vent to allow washout of exhaled gases. Leak flow rate, $Ql$, is the flow rate of leak from a patient interface system or elsewhere. Respiratory flow rate, $Qr$, is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water ($H_2O$) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including cmH$_2$O, g-f/cm$^2$ and

hectopascal. 1 $cmH_2O$ is equal to 1 g-f/$cm^2$ and is approximately 0.98 hectopascal (1 hectopascal = 100 Pa = 100 N/$m^2$ = 1 millibar ~ 0.001 atm). In this specification, unless otherwise stated, pressure is given in units of $cmH_2O$.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

### 5.9.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 5.9.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).

- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 $cmH_2O$ pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.9.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Ttot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:

(i) *Flattened:* Having a rise followed by a relatively flat portion, followed by a fall.

(ii) *M-shaped:* Having two local peaks, one at the leading edge, and one at the trailing edge, and a relatively flat portion between the two peaks.

(iii) *Chair-shaped:* Having a single local peak, the peak being at the leading edge, followed by a relatively flat portion.

(iv) *Reverse-chair shaped:* Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:

(i) a 30% reduction in patient breathing for at least 10 seconds plus an associated 4% desaturation; or

(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. *A patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume *Ve* (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO):* includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

## 5.9.3 Ventilation

*Adaptive Servo-Ventilator (ASV):* A servo-ventilator that has a changeable, rather than fixed target ventilation. The changeable target ventilation may be learned from some characteristic of the patient, for example, a respiratory characteristic of the patient.

*Backup rate:* A parameter of a ventilator that establishes the minimum breathing rate (typically in number of breaths per minute) that the ventilator will deliver to the patient, if not triggered by spontaneous respiratory effort.

*Cycled:* The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP):* a base pressure, to which a pressure varying within the breath is added to produce the desired interface pressure which the ventilator will attempt to achieve at a given time.

*End expiratory pressure (EEP):* Desired interface pressure which the ventilator will attempt to achieve at the end of the expiratory portion of the breath. If the pressure waveform template $\Pi(\Phi)$ is zero-valued at the end of expiration, i.e. $\Pi(\Phi) = 0$ when $\Phi = 1$, the EEP is equal to the EPAP.

*Inspiratory positive airway pressure (IPAP)*: Maximum desired interface pressure which the ventilator will attempt to achieve during the inspiratory portion of the breath.

*Pressure support:* A number that is indicative of the increase in pressure during ventilator inspiration over that during ventilator expiration, and generally means the difference in pressure between the maximum value during inspiration and the base pressure (e.g., *PS = IPAP - EPAP).* In some contexts pressure support means the difference which the ventilator aims to achieve, rather than what it actually achieves.

*Servo-ventilator:* A ventilator that measures patient ventilation, has a target ventilation, and which adjusts the level of pressure support to bring the patient ventilation towards the target ventilation.

*Spontaneous/Timed (S/T):* A mode of a ventilator or other device that attempts to detect the initiation of a breath of a spontaneously breathing patient. If however, the device is unable to detect a breath within a predetermined period of time, the device will automatically initiate delivery of the breath.

*Swing:* Equivalent term to pressure support.

*Triggered:* When a ventilator delivers a breath of *air* to a spontaneously breathing patient, it is said to be triggered to do so at the initiation of the respiratory portion of the breathing cycle by the patient's efforts.

### 5.9.4 Anatomy

### 5.9.4.1 Anatomy of the face

*Ala:* the external outer wall or "wing" of each nostril (plural: alar)

*Alar angle:*

*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework*: The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

*Lip, lower (labrale inferius)*:

*Lip, upper (labrale superius):*

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold*: The skin fold or groove that runs from each side of the nose to the corners of the mouth, separating the cheeks from the upper lip.

*Naso-labial angle:* The angle between the columella and the upper lip, while intersecting subnasale.

*Otobasion inferior*: The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare*: The point at the lower margin of the alar base, where the alar base joins with the skin of the superior (upper) lip.

*Subnasal point:* Located on the soft tissue, the point at which the columella merges with the upper lip in the midsagittal plane.

*Supramenton:* The point of greatest concavity in the midline of the lower lip between labrale inferius and soft tissue pogonion

### 5.9.4.2 Anatomy of the skull

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 5.9.4.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

**5.9.5 Patient interface**

*Anti-asphyxia valve (AAV):* The component or sub-assembly of a mask system that, by opening to atmosphere in a failsafe manner, reduces the risk of excessive $CO_2$ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

**5.9.6 Shape of structures**

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

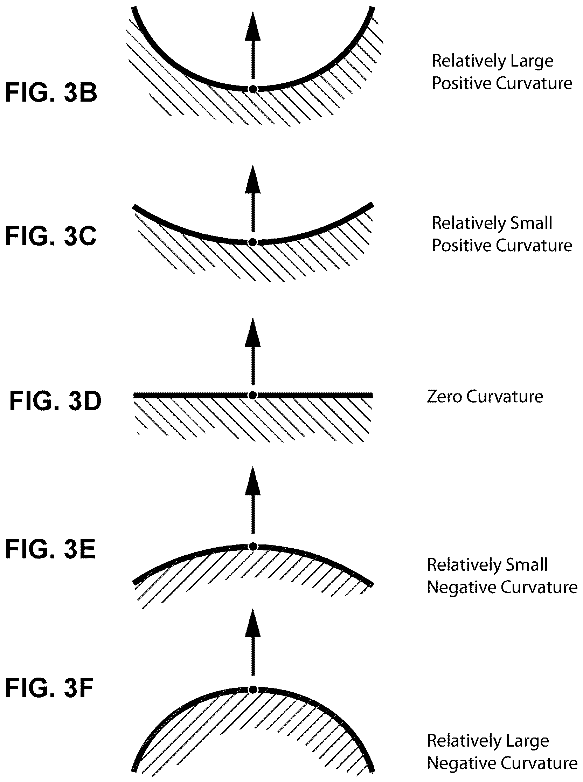

To facilitate describing the shape of the three-dimensional structures and the surfaces, we first consider a cross-section through a surface of the structure at a point, *p*. See Fig. 3B to Fig. 3F, which illustrate examples of cross-sections at point *p* on a surface, and the resulting plane curves. Figs. 3B to 3F also illustrate an outward normal vector at *p*. The outward normal vector at *p* points away from the surface. In some examples we describe the surface from the point of view of an imaginary small person standing upright on the surface.

### 5.9.6.1 Curvature in one dimension

The curvature of a plane curve at p may be described as having a sign (e.g. positive, negative) and a magnitude (e.g. 1/radius of a circle that just touches the curve at *p*).

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 5.9.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from *f*(0) to *f*(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.9.6.3 Space curves

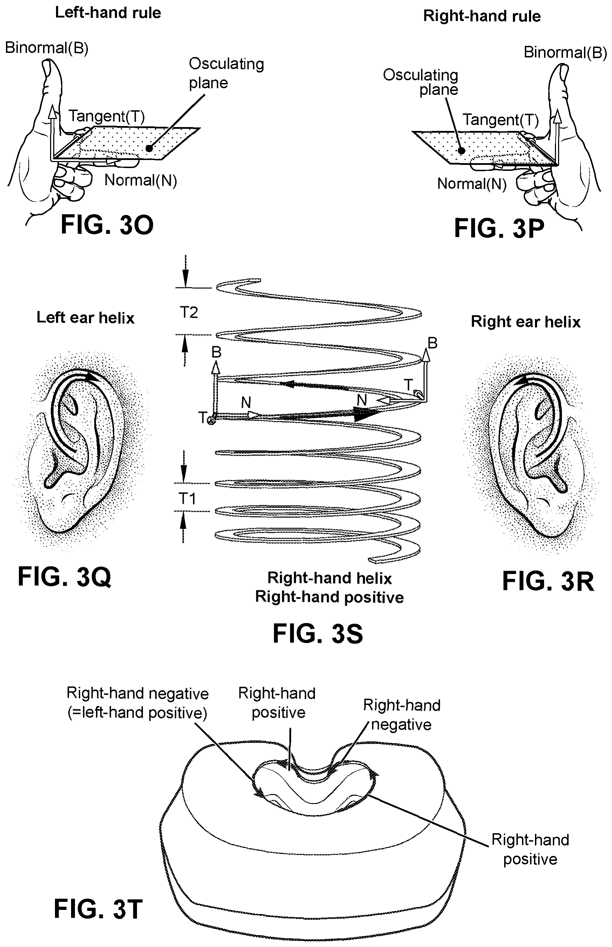

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

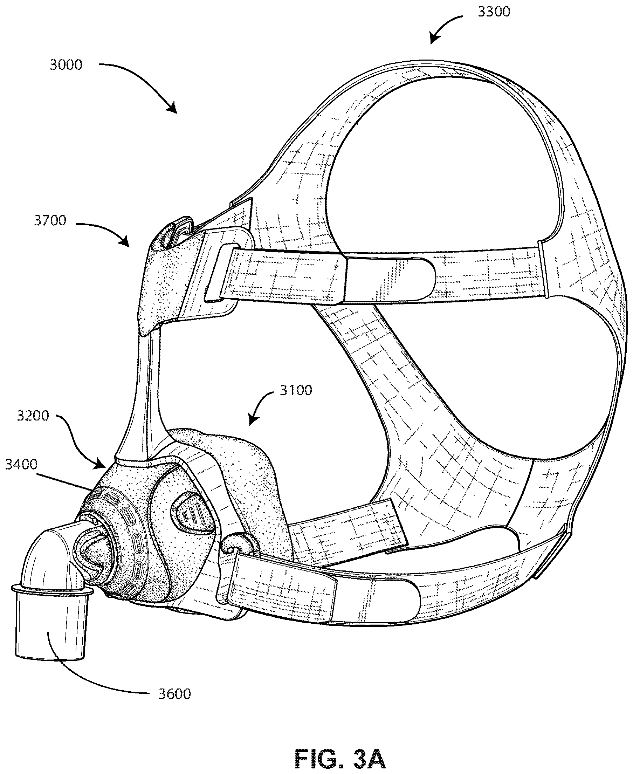

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 5.9.6.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 5.10 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are incorporated herein by reference in their entirety to disclose and describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

As used herein, the term "non-transitory computer-readable storage medium" includes a register, a cache memory, a ROM, a semiconductor memory device (such as a D-RAM, S-RAM, or other RAM), a magnetic medium such as a flash memory, a hard disk, a magneto-optical medium, an optical medium such as a CD-ROM, a DVD, or Blu-Ray Disc, or other type of device for non-transitory electronic data storage. The term "non-transitory computer-readable storage medium" does not include a transitory, propagating electromagnetic signal.

While certain examples are described in connection with CPAP systems, it will be appreciated that the techniques herein may be applicable to other types of home medical equipment.

It is therefore to be understood that numerous modifications may be made to the illustrative examples and that other arrangements may be devised without departing from the spirit and scope of the technology.

5.11 REFERENCE SIGNS LIST

| | |
|---|---|
| display screen | 120 |
| display screen | 122 |
| display screen | 124 |
| display screen | 126 |
| display screen | 128 |
| display screen | 130 |

(continued)

| | |
|---|---|
| display screen | 132 |
| display screen | 134 |
| display screen | 136 |
| display screen | 140 |
| display screen | 200 |
| display screen | 202 |
| display screen | 204 |
| display screen | 206 |
| display screen | 208 |
| display screen | 210 |
| display screen | 238 |
| display screen | 302 |
| display screen | 304 |
| display screen | 306 |
| computing device | 600 |
| processing system | 602 |
| system bus | 604 |
| memory devices | 606 |
| input adapter | 610 |
| input device | 612 |
| display interfaces | 614 |
| display device | 616 |
| network interface device | 618 |
| database | 620 |
| external resources | 622 |
| camera | 630 |
| IR emitter | 632 |
| patient | 1000 |
| patient | 1002 |
| patient | 1004 |
| bed partner | 1100 |
| patient interface | 3000 |
| strap | 3002 |
| connector | 3004 |
| indicator | 3006 |
| seal - forming structure | 3100 |
| plenum chamber | 3200 |
| chord | 3210 |
| superior point | 3220 |
| inferior point | 3230 |
| stabilising structure | 3300 |
| expandable portion | 3302 |
| tube | 3310 |
| tube | 3320 |
| first strap | 3330 |
| second strap | 3340 |
| indicator | 3350 |
| reference point | 3360 |
| reference marker | 3362 |
| vent | 3400 |
| connection port | 3600 |
| forehead support | 3700 |

(continued)

| | |
|---|---|
| unsealed patient interface | 3800 |
| RPT device | 4000 |
| external housing | 4010 |
| upper portion | 4012 |
| portion | 4014 |
| panels | 4015 |
| chassis | 4016 |
| handle | 4018 |
| pneumatic block | 4020 |
| air filter | 4110 |
| inlet air filter | 4112 |
| outlet air filter | 4114 |
| muffler | 4120 |
| inlet muffler | 4122 |
| outlet muffler | 4124 |
| pressure generator | 4140 |
| blower | 4142 |
| motor | 4144 |
| anti - spill back valve | 4160 |
| air circuit | 4170 |
| air circuit | 4171 |
| supplementary gas | 4180 |
| electrical components | 4200 |
| PCBA | 4202 |
| power supply | 4210 |
| input device | 4220 |
| central controller | 4230 |
| clock | 4232 |
| therapy device controller | 4240 |
| protection circuits | 4250 |
| memory | 4260 |
| transducer | 4270 |
| pressure sensor | 4272 |
| flow rate sensor | 4274 |
| motor speed transducer | 4276 |
| data communication interface | 4280 |
| remote external communication network | 4282 |
| local external communication network | 4284 |
| remote external device | 4286 |
| local external device | 4288 |
| output device | 4290 |
| display driver | 4292 |
| display | 4294 |
| algorithms | 4300 |
| pre - processing module | 4310 |
| interface pressure estimation algorithm | 4312 |
| vent flow rate estimation algorithm | 4314 |
| leak flow rate estimation algorithm | 4316 |
| respiratory flow rate estimation algorithm | 4318 |
| therapy engine module | 4320 |
| phase determination algorithm | 4321 |
| waveform determination algorithm | 4322 |

(continued)

| | |
|---|---|
| ventilation determination algorithm | 4323 |
| inspiratory flow limitation determination algorithm | 4324 |
| apnea / hypopnea determination algorithm | 4325 |
| snore determination algorithm | 4326 |
| snore determination | 4326 |
| airway patency determination algorithm | 4327 |
| target ventilation determination algorithm | 4328 |
| therapy parameter determination algorithm | 4329 |
| therapy parameter determination | 4329 |
| therapy control module | 4330 |
| algorithm | 4340 |
| method | 4500 |
| step | 4520 |
| step | 4530 |
| step | 4540 |
| step | 4550 |
| step | 4560 |
| humidifier | 5000 |
| humidifier inlet | 5002 |
| humidifier outlet | 5004 |
| humidifier base | 5006 |
| reservoir | 5110 |
| conductive portion | 5120 |
| humidifier reservoir dock | 5130 |
| locking lever | 5135 |
| water level indicator | 5150 |
| humidifier transducer | 5210 |
| humidifier transducers sensors | 5210 |
| air pressure sensor | 5212 |
| flow rate transducers | 5214 |
| temperature transducers | 5216 |
| humidity sensor | 5218 |
| heating element | 5240 |
| humidifier controller | 5250 |
| central humidifier controller | 5251 |
| heating element controller | 5252 |
| air circuit controller | 5254 |
| communication link | 6020 |
| server | 6030 |
| cloud computing platform | 6040 |
| medical devices | 6062 |
| medical devices | 6064 |
| display interfaces | 6514 |
| step | 6910 |
| step | 6912 |
| step | 6914 |
| step | 7012 |
| step | 7014 |
| step | 7016 |
| step | 7018 |
| step | 7020 |
| step | 7022 |

(continued)

| step | 7024 |
| step | 7026 |
| step | 7030 |
| nasal prongs | 3810a |
| nasal prongs | 3810b |
| air supply lumens | 3820a |
| air supply lumens | 3820b |

The following aspects are preferred embodiments of the invention.

1. A respiratory pressure therapy system for providing continuous positive air pressure (CPAP) to a patient via a patient interface configured to engage with at least one airway of the patient, the system comprising:

a flow generator configured to generate supply of breathable gas for delivery to the patient via the patient interface, wherein the breathable gas is output from the flow generator at a pressure level that is above atmospheric pressure;

at least one sensor that is configured to measure a physical quantity while the breathable gas is supplied to the patient;

a display; and

a computing device including memory and at least one hardware processor, the computing device configured to:

receive, from the at least one sensor, sensor data that is based on measured physical property of the supply of breathable gas;

control, based on the received sensor data, the flow generator to adjust a property of the supply of breathable gas that is delivered to the patient;

receive an input indicating assistance is needed with using the patient interface;

receive one or more images including the patient with the patient interface;

analyse the received one or more images; and

based on the analysis, display, on the display, instructions for positioning the patient interface.

2. The respiratory pressure therapy system of aspect 1, wherein the memory includes one or more reference images including the patient with the patient interface, and the displayed instructions are generated by the computing device based on comparison between the one or more reference images and the received one or more images.

3. The respiratory pressure therapy system of aspect 2, wherein computing device is coupled to a camera configured to capture the one or more reference images.

4. The respiratory pressure therapy system of any one of aspects 2 to 3, wherein the computing device is configured to compare the one or more reference images and the received one or more images to determine improper patient interface fitting position.

5. The respiratory pressure therapy system of any one of aspects 1 to 4, wherein the computing device is configured to superimpose a correct position of the patient interface on the received one or more images, and the displayed instructions include the one or more superimposed images.

6. The respiratory pressure therapy system of any one of aspects 1 to 5, further comprising a remote computing system and the remote computing system is configured to determine the instructions for positioning the patient interface, and transmit the instructions to the computing device.

7. The respiratory pressure therapy system of aspect 6, wherein the remote computing system is configured to:

receive, from the computing device, the one or more images;

train a machine learning model for instructing correct positioning of a patient interface; and

the instructions for positioning the patient interface are determined based on the trained machine learning model.

8. The respiratory pressure therapy system of any one of aspects 1 to 7, wherein the computing device is further configured to receive an input indicating a type of the patient interface, and display, on the display, instructions for using the type of patient interface indicated by the input.

9. The respiratory pressure therapy system of any one of aspects 1 to 5 or 8, wherein the computing device is configured to transmit the received one or more images to a remote processing system configured to perform machine learning using the one or more images.

10. The respiratory pressure therapy system of any one of aspects 1 to 9, wherein the instructions include text, images, and/or graphics.

11. A non-transitory computer readable storage medium storing instructions for use with a computing device that is configured to control a continuous positive air pressure (CPAP) device configured to generate the supply of breathable gas that is delivered to a patient via a patient interface configured to engage with at least one airway of the patient, wherein the breathable gas is output from a flow generator at a pressure level that is above atmospheric pressure, the CPAP device associated with at least one sensor that is configured to measure a physical quantity while the breathable gas is supplied to the patient, the computing device including at least one hardware processor, the stored instructions comprising instructions that are configured to cause the computing device to:

receive, from the at least one sensor, sensor data that is based on measured physical property of the supply of breathable gas;

control, based on the received sensor data, the flow generator to adjust a property of the supply of breathable gas that is delivered to the patient;

receive, an input indicating assistance is needed with using the patient interface;

receive one or more images including the patient with the patient interface;

analyse the received one or more images; and

based on the analysis, display, on the display, instructions for positioning the patient interface.

12. A device comprising:

a display;

a camera;

a memory; and

a processing system including at least one hardware processor coupled to the display, the camera, and the memory, the processing system configured to:

receive an indication identifying a type of a patient interface, configured to engage with at least one airway of a patient and supply breathable gas received from a continuous positive air pressure (CPAP) device to the patient;

receive, from the camera, one or more images including a patient with the patient interface;

analyse the received one or more images to determine fit of the patient interface on the patient; and

based on the analysis, display, on the display, feedback for improving the fit of the patient interface on the patient.

13. The device of aspect 12, wherein the received indication identifying the type of the patient interface is a user input.

14. The device of aspect 12, wherein the received indication identifying the type of the patient interface is determined, by the processing system, based on an image including the patient interface.

15. The device of any one of aspects 12 to 14, wherein the analysis includes comparing the received one or more images to one or more reference images.

16. The device of aspect 15, wherein the one or more reference images include the patient with the patient interface.

17. The device of any one of aspects 15 and 16, wherein the one or more reference images include a plurality of reference points, and the analysis includes, detecting reference points in the received one or more images and comparing the detected reference points to the plurality of reference points in the one or more defence images.

18. The device of any one of aspects 12 to 17, wherein the analysis includes extracting, from the received one or more images, one or more indicators included in patient interface.

19. The device of any one of aspects 12 to 18, wherein the received one or more images are captured from different positions and orientations of the camera.

20. The device of any one of aspects 12 to 19, wherein a plurality of images are captured at different times and the analysis includes comparing the plurality of images to determine changes in positioning of the patient interface between images captured at the different times.

21. The device of any one of aspects 12 to 20, wherein displaying the feedback includes displaying at least one of the received images, and the processing system is configured to include, in the displayed received images, one or more visual indicators indicating locations on the patient interface where the fit of the patient interface can be improved.

22. The device of any one of aspects 12 to 21, wherein the processing system is configured to display instructions for using the identified type of patient interface.

23. The device of any one of aspects 12 to 22, wherein the analysis includes comparing the received one or more images to models generated based on information received from other patients.

24. The device of any one of aspects 12 to 23, wherein the analysis includes extracting features from the received one or more images, and comparing positions and/or orientations of the features to a three-dimensional model of the patient interface.

25. The device of any one of aspects 12 to 24, wherein the analysis includes comparing the received one or more images to a machine trained model that is updated based on data received from other patients.

26. The device of any one of aspects 12 to 25, wherein the device is a mobile phone, tablet or remote control.

27. The device of any one of aspects 12 to 26, wherein the processing system is further configured to receive sensor data from one or more sensors disposed on a surface of or in the patient interface.

28. The device of aspect 27, wherein the processing system is further configured to perform the analysis and display the feedback based on data received from the sensors.

29. The device of any one of aspects 27 or 28, wherein the sensor data is compared to pre-set sensor values stored in the memory.

30. A non-transitory computer readable storage medium storing instructions for use with a computing device, the stored instructions comprising instructions that are configured to cause the computing device to:
receive an indication identifying a type of a patient interface, configured to engage with at least one airway of a patient and supply breathable gas received from a continuous positive air pressure (CPAP) device to the patient;

receive, from a camera, one or more images including a patient with the patient interface;

analyse the received one or more images to determine fit of the patient interface on the patient; and

based on the analysis, display, on the display, feedback for improving the fit of the patient interface on the patient.

31. A respiratory pressure therapy system for providing continuous positive air pressure (CPAP) to a patient via a patient interface including one or more patient interface sensors and configured to engage with at least one airway of the patient, the system comprising:

a flow generator configured to generate supply of breathable gas for delivery to the patient via the patient interface, wherein the breathable gas is output from the flow generator at a pressure level that is above atmospheric pressure;

a display; and

a computing device including memory and at least one hardware processor, the computing device configured to:

control the flow generator to adjust a property of the supply of breathable gas that is delivered to the patient;

receive sensor data from the one or more patient interface sensors;

compare the received sensor data to pre-set values in the memory; and

based on the comparison, display, on the display, instructions for positioning the patient interface.

32. The respiratory pressure therapy system of aspect 31, wherein the memory includes one or more images including a patient with the patient interface, and the displayed instructions include displaying the one or more images based on the comparison.

33. The respiratory pressure therapy system of any one of aspects 31 or 32, wherein the computing device is coupled to a camera configured to capture one or more images of the patient with the patient interface.

34. The respiratory pressure therapy system of aspect 33, wherein the computing device is configured to compare one or more reference images stored in memory and the captured one or more images to determine improper patient interface fitting position.

35. The respiratory pressure therapy system of aspect 34, wherein the computing device is configured to superimpose a correct position of the patient interface on the captured one or more images, and the displayed instructions include the one or more superimposed images.

36. The respiratory pressure therapy system of any one of aspects 31 to 35, further comprising a remote computing system and the remote computing system is configured to determine the instructions for positioning the patient interface, and transmit the instructions to the computing device.

37. The respiratory pressure therapy system of any one of aspects 31 to 36, wherein the computing device is further configured to receive an input indicating a type of the patient interface, and display, on the display, instructions for using the type of patient interface indicated by the input.

38. The respiratory pressure therapy system of any one of aspects 31 to 37, wherein at least one of the one or more patient interface sensors is disposed on a surface of the patient interface.

39. The respiratory pressure therapy system of any one of aspects 31 to 38, wherein at least one of the one or more patient interface sensors is a pressure sensor configured to measure pressure inside of a mask of the patient interface.

40. The respiratory pressure therapy system of any one of aspects 31 to 39, wherein at least one of the one or more patient interface sensors is a sensor configured to measure a physical property of the supply of breathable gas in an oral or nasal cushion of the patient interface.

41. The respiratory pressure therapy system of any one of aspects 31 to 40, wherein at least one of the one or more patient interface sensors is disposed on a surface or inside of a strap of the patient interface.

42. A device comprising:

a display;

a camera;

a memory; and

a processing system including at least one hardware processor coupled to the display, the camera, and the memory, the processing system configured to:

capture one or more images including a patient using the camera;

determine characteristics of facial features from the captured images;

transmit the determined characteristics of the facial features to a remote processing system for analysis;

receive, from the remote processing system, data for one or more settings of a patient interface configured to engage with at least one airway of the patient and supply breathable gas received from a continuous positive air pressure (CPAP) device to the patient; and

display, on the display, instructions for adjusting the one or more settings of the patient interface based on the received data for the one or more settings of the patient interface.

43. The device of aspect 42, wherein the processing system is further configured to:

receive a user input identifying a type of the patient interface; and

transmit the type of the patient interface to the remote processing system for analysis.

44. The device of aspect 42, wherein the processing system is further configured to:

receive, from the camera, one or more second images including the patient with the patient interface;

analyse the received one or more second images to determine fit of the patient interface on the patient; and

based on the analysis and the received data for the one or more settings of the patient interface, display, on the display, feedback for improving the fit of the patient interface on the patient.

45. The device of aspect 44, wherein the analysis includes comparing the received one or more second images to one or more reference images.

46. The device of aspect 45, wherein the one or more reference images include the patient wearing the patient interface.

47. The device of any one of aspects 45 and 46, wherein the one or more reference images include a plurality of reference points, and the analysis includes, detecting reference points in the received one or more second images and comparing the detected reference points to the plurality of reference points in the one or more defence images.

48. The device of any one of aspects 44 to 47, wherein the analysis includes extracting, from the received one or more second images, one or more indicators included on the patient interface.

49. The device of aspect 48, wherein the one or more indicators include a plurality of indicators provided on one or more straps of the patient interface.

50. The device of any one of aspects 48 and 49, wherein the one or more indicators include at least one indicator provided on a mask of the patient interface.

51. The device of any one of aspects 48 and 50, wherein the one or more indicators include an indicator provided on a connector configured to connect at least one of the straps to a mask of the patient interface.

52. The device of any one of aspects 48 and 51, wherein the processing system is further configured to: determine a force applied by one or more straps based on the characteristics of the one or more indicators in the one or more second images.

53. The device of any one of aspects 48 and 52, wherein the processing system is further configured to compare forces applied by different straps of the patient interface based on characteristics of the one or more indicators included in the different straps.

54. The device of any one of aspects 48 and 53, wherein the processing system is further configured to indicate that one or more straps are over tightened based on characteristics of the one or more indicators in the one or more straps.

55. The device of aspect 42, wherein the analysis performed by the remote processing system includes comparing the received one or more images to models generated based on information received from a plurality of other patients.

56. The device of any one of aspects 42 and 55, wherein the device is the respiratory pressure therapy device for providing continuous positive air pressure (CPAP) to the patient, the respiratory pressure therapy device comprising:

a flow generator configured to generate supply of breathable gas for delivery to the patient via the patient interface, wherein the breathable gas is output from the flow generator at a pressure level that is above atmospheric pressure; and

at least one sensor that is configured to measure a physical quantity while the breathable gas is supplied to the patient.

57. A non-transitory computer readable storage medium storing instructions for use with a computing device, the stored instructions comprising instructions that are configured to cause the computing device to:

receive one or more images including a patient using captured by a camera;

determine characteristics of facial features from the captured images;

transmit the determined characteristics of the facial features to a remote processing system for analysis;

receive, from the remote processing system, data for one or more settings of a patient interface configured to engage with at least one airway of the patient and supply breathable gas received from a continuous positive air pressure (CPAP) device to the patient; and

output instructions for adjusting the one or more settings of the patient interface based on the received data for the one or more settings of the patient interface.

, instructions for positioning the patient interface.

## Claims

1. A device comprising:

a display;
a camera;

a memory; and

a processing system including at least one hardware processor coupled to the display, the camera, and the memory, the processing system configured to:

capture one or more images including a patient using the camera;

determine characteristics of facial features from the captured images;

transmit the determined characteristics of the facial features to a remote processing system for analysis;

based on analysis performed by the remote processing system, receive, from the remote processing system, data for one or more settings of a patient interface configured to engage with at least one airway of the patient and supply breathable gas received from a continuous positive air pressure (CPAP) device to the patient; and

display, on the display, instructions for adjusting the one or more settings of the patient interface based on the received data for the one or more settings of the patient interface.

2. The device of claim 1, wherein the processing system is further configured to:

receive a user input identifying a type of the patient interface; and

transmit the type of the patient interface to the remote processing system for analysis.

3. The device of claim 1, wherein the processing system is further configured to:

receive, from the camera, one or more second images including the patient with the patient interface;

analyse the received one or more second images to determine fit of the patient interface on the patient; and

based on the analysis and the received data for the one or more settings of the patient interface, display, on the display, feedback for improving the fit of the patient interface on the patient.

4. The device of claim 3, wherein the analysis includes comparing the received one or more second images to one or more reference images, wherein the one or more reference images preferably include the patient wearing the patient interface.

5. The device of claim 4, wherein the one or more reference images include a plurality of reference points, and the analysis includes, detecting reference points in the received one or more second images and comparing the detected reference points to the plurality of reference points in the one or more reference images.

6. The device of any one of claims 3 to 5, wherein the analysis includes extracting, from the received one or more second images, one or more indicators included on the patient interface.

7. The device of claim 6, wherein the one or more indicators include a plurality of indicators provided on one or more straps of the patient interface.

8. The device of any one of claims 6 and 7, wherein the one or more indicators include at least one indicator provided on a mask of the patient interface.

9. The device of any one of claims 6 and 8, wherein the one or more indicators include an indicator provided on a connector configured to connect at least one of the straps to a mask of the patient interface.

10. The device of any one of claims 6 and 9, wherein the processing system is further configured to: determine a force applied by one or more straps based on the characteristics of the one or more indicators in the one or more second images.

11. The device of any one of claims 6 and 10, wherein the processing system is further configured to compare forces applied by different straps of the patient interface based on characteristics of the one or more indicators included in the different straps.

12. The device of any one of claims 6 to 11, wherein the processing system is further configured to indicate that one or more straps are over tightened based on characteristics of the one or more indicators in the one or more straps.

13. The device of claim 1, wherein the analysis performed by the remote processing system includes comparing the received one or more images to models generated based on information received from a plurality of other patients.

**14.** The device of any one of claims 1 to 13, wherein the device is the respiratory pressure therapy device for providing continuous positive air pressure (CPAP) to the patient, the respiratory pressure therapy device comprising:

a flow generator configured to generate supply of breathable gas for delivery to the patient via the patient interface, wherein the breathable gas is output from the flow generator at a pressure level that is above atmospheric pressure; and
at least one sensor that is configured to measure a physical quantity while the breathable gas is supplied to the patient.

**15.** A non-transitory computer readable storage medium storing instructions for use with a computing device, the stored instructions comprising instructions that are configured to cause the computing device to:

receive one or more images including a patient using captured by a camera;
determine characteristics of facial features from the captured images;
transmit the determined characteristics of the facial features to a remote processing system for analysis;
based on analysis performed by the remote processing system, receive, from the remote processing system, data for one or more settings of a patient interface configured to engage with at least one airway of the patient and supply breathable gas received from a continuous positive air pressure (CPAP) device to the patient; and
output instructions for adjusting the one or more settings of the patient interface based on the received data for the one or more settings of the patient interface.

**FIG. 1A**

FIG. 1B

FIG. 1C

**FIG. 2A**

Nasal cavity

Nasal bone

Lateral nasal cartilage

Greater alar cartilage

Nostril

Lip superior

Lip inferior

Hard palate

Soft palate

Oropharynx

Tongue

Epiglottis

Vocal folds

Larynx

Esophagus

Trachea

**FIG. 2B**

**FIG. 2C**

EP 4 700 787 A2

Superior

Anterior ← → Posterior

Inferior

Glabella

Otobasion superior

Sellion

Ridge

Pronasale

Alar crest
point

Otobasion inferior

Subnasale

Lip superior

Lip Inferior

Supramenton

**FIG. 2D**

Coronal plane

Superior

Posterior

Anterior

Inferior

Frankfort horizontal

Nasolabial angle

**FIG. 2E**

FIG. 2F

FIG. 2G   FIG. 2H   FIG. 2I

FIG. 2K

FIG. 2J

Parietal bone
Temporal bone
Occipital bone
Trapezius m.

Frontal bone
Sphenoid bone
Nasal bone
Zygomatic bone
Maxilla
Masseter m.
Mandible
Mental protuberance
Digastricus m.
Sternocleidomastoid m.

Concha

Frontal bone

Supraorbital foramen

Nasal bones

Septal cartilage

Lateral cartilage

Sesamoid cartilage

Greater alar cartilage

Medial crus of greater alar cartilage

Anterior nasal spine

Infraorbital foramen

Lesser nasal cartilage

**Alar fibrofatty** tissue

Septal cartilage

# FIG. 2L

**FIG. 3A**

**FIG. 3B**                    Relatively Large
                               Positive Curvature

**FIG. 3C**                    Relatively Small
                               Positive Curvature

**FIG. 3D**                    Zero Curvature

**FIG. 3E**                    Relatively Small
                               Negative Curvature

**FIG. 3F**

                               Relatively Large
                               Negative Curvature

**FIG. 3H**

Legend:
+ = Positive Curvature
− = Negative Curvature
= Outward Normal

Labels: Exterior Surface, A Path on Surface, Edge of Surface, Straight Line Distance, B, A, Dome Region, Saddle Region

**FIG. 3G**

Legend:
+ = Positive Curvature
− = Negative Curvature
= Outward Normal

Labels: Edge of Surface, Saddle Region, Dome Region, Exterior Surface, Saddle Region

FIG. 3J FIG. 3J

Curve

**FIG. 3I**

Surface

**FIG. 3K**

Surface

**FIG. 3J**

FIG. 3M

FIG. 3M

**FIG. 3L**

FIG. 3N

FIG. 3N

Interior surface

Interior
surface

**FIG. 3M**

**FIG. 3N**

Interior surface

**Left-hand rule**

Binormal(B)

Osculating plane

Tangent(T)

Normal(N)

# FIG. 3O

**Right-hand rule**

Binormal(B)

Osculating plane

Tangent(T)

Normal(N)

# FIG. 3P

**Left ear helix**

T2

B

N

T

B

T

N

T1

# FIG. 3Q

**Right-hand helix**
**Right-hand positive**

# FIG. 3S

**Right ear helix**

# FIG. 3R

Right-hand negative (=left-hand positive)

Right-hand positive

Right-hand negative

Right-hand positive

# FIG. 3T

**FIG. 3U**

**FIG. 3V**

**FIG. 3W**

**FIG. 3X**

**FIG. 3Y**

**FIG. 4A**

4015
4018
4112
4000
4220
4015
4012
Superior
Posterior
Anterior
Inferior
4202
4142
4100, 4020
4200
4010
4016
4210
4014

4110 — 4112 Air inlet filter ← Supplementary O₂

Pneumatic block

4120 — 4122 Inlet muffler

4270 — Transducer(s)

4140 — | 4142 Blower | 4144 Motor |

4020

4120 — 4124 Outlet muffler

Upstream

4270 — Transducer(s)

Downstream

4160 — Anti-spillback valve

5000 — Humidifier

4110 — 4114 Filter

4180

4170 — Air circuit / delivery tube ← Supplementary O₂

4180

4270 — Transducer(s)

**FIG. 4B**

4180

3000 — Patient Interface ← Supplementary O₂

**FIG. 4C**

4300

4310

**Preprocessing**

| Interface pressure estimation | Vent flow rate estimation | | Leak flow rate estimation |

4312      4314

Respiratory flow rate estimation

4316

4318

4320

4321      4322      4323

**Therapy engine**

| Phase determination | Waveform determination | Ventilation determination |

| Flow limitation determination | Apnea / hypopnea determination | Snore determination |

4324      4325      4326

| Airway patency determination | Target ventilation determination | Therapy parameter determination |

4327      4328      4329

4340

Fault condition detection

Therapy control

4330

# FIG. 4D

4500

4520

Y — Apnea / hypopnea index > threshold for a time?

N

4530    4560

Obstruction Index > threshold ? — N → Decrease treatment pressure

Y

4540

Airway patent? — N → 

4550

Increase treatment pressure

Y

**FIG. 4E**

FIG. 5A

FIG. 5B

FIG. 5C

FIG. 6A

**FIG. 6B**

EP 4 700 787 A2

Receive patient data and/or image(s)
7012

Select patient interface
7014

Display instructions for using selected patient
interface
7016

Capture image(s) of patient with the patient
interface and/or receive sensor data
7018

Issues
with patient interface?
7020

NO → Go to resolve state
7030

YES → Capture image(s) and/or receive
sensor data
7022

Analyze captured image(s) and/
or sensor data
7024

Display results of the analysis
7026

**FIG. 6C**

FIG. 7A

3360

D3

D2

3350

3330

3350

D1

3360

**FIG. 7B**

3002

Side View

# FIG. 7C

3002

Top View

← Direction of Pull

1    2    3

3006    3004

# FIG. 7D

3002

No Stretch
Side View

Top View  ← Direction of Pull

3304

3350

D1

**FIG. 7E**

3002

Stretched
Top View

← Direction of Pull

3304

3350

D2

**FIG. 7F**

| Setup Mask | Setup Mask | Setup Mask | Setup Mask |
|---|---|---|---|
| Take mask out of box. | Take mask out of supporting shell. | Undo magnets on the bottom strap. | Put on the mask |
| ( Continue ) | ( Continue ) | ( Continue ) | ( Continue ) |
| 120 | 122 | 124 | 126 |

| Setup Mask | Setup Mask | Setup Mask | Setup Mask |
|---|---|---|---|
| Check the mask and headgear fitted | Adjust top and bottom straps. | When you pull from the front the cushion should still touch your face. | Connect the tubes |
| ( Continue ) | ( Continue ) | ( Continue ) | ( Continue ) |
| 128 | 130 | 132 | 134 |

| Setup Mask | Setup Mask | Setup Mask | Setup Mask |
|---|---|---|---|
| Connect the tube to the front of the mask | Check fit and operation of mask. ( Get Assistance ) Does mask feel comfortable? | Take pictures or video of yourself with mask. | |
| ( Continue ) | ( NO ) **YES** | ● 140 | ( Complete ) |
| 136 | 138 | | 142 |

**FIG. 8A**

| Detect Mask Issues | Detect Mask Issues | Detect Mask Issues |
|---|---|---|
| Are you experiencing issues with mask?<br><br><br>( NO )  **YES**<br><br>**200** | Take pictures or video of yourself with mask.<br><br>◉<br>**202** | Select one of the identified issues<br><br>**204** |

| Detect Mask Issues | Detect Mask Issues | Detect Mask Issues |
|---|---|---|
| Identified issue 1:<br><br>Adjust the bottom strap on left side of face to decrease tension in the bottom trap.<br><br>( Continue )<br>**206** | Identified issue 2:<br><br>Adjust the top strap on left side of face to increase tension in the top strap.<br><br>( Continue )<br>**208** | Has issue been corrected?<br><br><br>( NO )  **YES**<br><br>**210** |

## FIG. 8B

302

304

306

Attach upper portion to lower portion of patient interface.

Continue

Attach patient interface to the mask. Adjust lower straps so that the third indicator is shown.

Continue

Loosen left lower strap.

Continue

**FIG. 8C**

**FIG. 9**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- SG 10201911248T
- US 4944310 A, Sullivan
- US 6532959 B, Berthon-Jones
- WO 1998004310 A
- WO 2006074513 A
- WO 2010135785 A
- US 4782832 A, Trimble
- WO 2004073778 A
- US 20090044808 A
- WO 2005063328 A
- WO 2006130903 A
- WO 2009052560 A
- US 20100000534 A
- WO 1998034665 A
- WO 2000078381 A
- US 6581594 B
- US 20090050156 A
- US 20090044808
- US 7866944 B
- US 8638014 B
- US 8636479 B
- WO 2013020167 A
- US 8733349 B
- WO 2012171072 A
- US 7827038 B
- US 8254637 B